(19) Europäisches Patentamt / European Patent Office / Office européen des brevets

(11) **EP 3 437 595 A1**

(12) **EUROPEAN PATENT APPLICATION**
published in accordance with Art. 153(4) EPC

(43) Date of publication:
**06.02.2019 Bulletin 2019/06**

(21) Application number: **17773682.4**

(22) Date of filing: **07.02.2017**

(51) Int Cl.:
***A61F 11/14*** (2006.01)          ***G10K 11/16*** (2006.01)

(86) International application number:
**PCT/JP2017/004352**

(87) International publication number:
**WO 2017/169133 (05.10.2017 Gazette 2017/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.03.2016 JP 2016066245**

(71) Applicant: FUJIFILM Corporation
**Tokyo 106-8620 (JP)**

(72) Inventors:
• **NAYA Masayuki**
  **Ashigarakami-gun,**
  **Kanagawa 258-8577 (JP)**
• **YAMAZOE Shogo**
  **Ashigarakami-gun,**
  **Kanagawa 258-8577 (JP)**
• **HAKUTA Shinya**
  **Ashigarakami-gun,**
  **Kanagawa 258-8577 (JP)**

(74) Representative: **Klunker IP**
**Patentanwälte PartG mbB**
**Destouchesstraße 68**
**80796 München (DE)**

(54) **EARMUFF**

(57)     Provided is an earmuff that is compact and lightweight, has high sound insulating performance, is capable of insulating sound of a specific frequency range, and has excellent air permeability.

The earmuff includes a headband, and two ear cups each having a housing attached to an end part of the headband and an ear pad locked to the housing, the housing has a housing opening part, a sound insulation structure is disposed in the housing opening part and insulates sound of a specific frequency range, and the housing opening part having the sound insulation structure disposed therein has a ventilation hole.

FIG. 1

**Description**

**BACKGROUND OF THE INVENTION**

1. Field of the Invention

The present invention relates to an earmuff.

2. Description of the Related Art

In the related art, in order to protect workers under noisy environments, earmuffs are widely used. Earmuffs each have a pair of ear cups attached to a headband, and are worn on the head by putting the ear cups on both ears like headphones.

Since general sound shielding materials shield sound better as the mass is heavier, in order to enhance sound insulating performance in the earmuffs, it is necessary to enlarge the ear cups, to dispose a sound insulating material within a housing of each ear cup, or to make the housing heavy. For that reason, in a case where the sound insulating performance of the earmuffs is enhanced, there are problems that the mass of the earmuffs becomes heavy, and the comfort of wearing gets worse.

For example, JP2011-15338 discloses including a headband, ear cups attached to the headband, a baffle plate fixed to each ear cup, and an ear pad locked to the baffle plate, and having an air-permeable member between the baffle plate and the ear pad, so that the volume of a front air space can be increased to make the sound shielding performance high.

**SUMMARY OF THE INVENTION**

However, in the related-art earmuffs that insulate sound on the basis of the volume, the mass, and the like of the ear cups, in order to achieve higher sound insulating performance, it is also necessary to enlarge the ear cups, to dispose the sound insulating material within the housing of each ear cup, or to make the housing heavy. Additionally, in that case, since air permeability gets worse, there is a problem that the comfort of wearing gets worse.

Although it is considered that a ventilation hole is provided in the ear cup to secure the air permeability, in a case where the ventilation hole is provided, there is a problem that the sound insulating performance degrades.

Additionally, in a case where sound is insulated on the basis of the volume, the mass, and the like of the ear cups, sound in an entire range is cut off. Therefore, there is a problem that required sound, such as conversation, is also insulated.

As a device capable of insulating sound of a specific frequency range, a so-called active noise reduction device that picks up noise, and outputs sound having a phase opposite to the noise from a loudspeaker, to cancel out the noise is known. However, since a power source is required, in a case where this device is used for the earmuffs, there are problems that the earmuffs become heavy and cannot be not used for a long time. Additionally, since the power source is required, there is a problem that the place of use is limited.

The object of the invention is to provide an earmuff that solves the above related-art problems, is compact and lightweight, has high sound insulating performance, is capable of insulating sound of a specific frequency range, and has excellent air permeability.

In addition, in the invention, although the "sound insulation" includes the meanings of both "sound shielding" and "sound absorption" as acoustic characteristics, the "sound insulation" means particularly the "sound shielding". However, the "sound shielding" means including "shield sound", that is, "does not transmit sound", and thus "reflect" sound (reflection of sound) and "absorb" sound (absorption of sound) (refer to http://www.onzai.or.jp/question/soundproof.html and http://www.onzai.or.jp/pdf/new/gijutsu201312_3.pdf. of web pages of Sanseido Daijirin (third edition) and Acoustical Materials Association of Japan).

In the following, basically, the "sound insulation" refers to "sound shielding" and "shielding" including both "reflection" and "absorption" without distinguishing between them, and refers to "reflection" and "absorption" in a case where "reflection" and "absorption" are distinguished from each other.

The present inventors have conducted intensive studies in order to achieve the above object, and as a result, have found that the above problems can be solved by including a headband, and two ear cups each having a housing attached to an end part of the headband and an ear pad locked to the housing, the housing has a housing opening part, a sound insulation structure is disposed in the housing opening part and insulates sound of a specific frequency range, and the housing opening part having the sound insulation structure disposed therein has ventilation hole, and have completed the invention.

That is, the present inventor have found that the above object can be achieved by the following configurations.

[1] An earmuff comprising a supporting member; and two ear cups each having a housing attached to the supporting member and an ear pad locked to the housing, in which the housing has a housing opening part, a sound insulation structure is disposed in the housing opening part and insulates sound of a specific frequency range, and in which the housing opening part having the sound insulation structure disposed therein has a ventilation hole.

[2] The earmuff according to [1] in which the sound insulation structure has one or more sound insulation cells, in which the one or more sound insulation cells each include a frame having a frame hole part penetrating thereof, a film fixed to the frame, an opening part including one or more through-holes pierced in the film, and in which both end parts of the frame hole parts of the frame are not blocked, and the sound insulation structure is disposed to close the housing opening part.

[3] The earmuff according to [2], further comprising a weight disposed on the film.

[4] The earmuff according to [1] in which the sound insulation structure has two or more sound insulation cells that are two-dimensionally arranged, wherein at least one of the sound insulation cells is a first sound insulation cell including a first frame having a first frame hole part penetrating thereof, and a film fixed to the first frame, in which at least other one of the sound insulation cells is a second sound insulation cell including a second frame having a second frame hole part penetrating thereof, and in which the sound insulation structure is disposed so as to close the housing opening part.

[5] The earmuff according to [1], wherein the sound insulation structure includes a plate-shaped member having a plurality of through-holes penetrating in a thickness direction thereof, in which an average opening diameter of the through-holes is 0.1 $\mu$m or more and less than 100 $\mu$m, in which an average opening ratio rho of the through-holes is within a range more than 0 and less than 1 and is within a range having rho_center - (0.052 x (phi/30)$^{-2}$) as a lower limit and having rho _center+ (0.795 x (phi/30)$^{-2}$) as an upper limit, with rho_center = (2 + 0.25 x t) x phi$^{-1.6}$ as a center in a case where the average opening diameter of the through-holes is phi ($\mu$m) and a thickness of the plate-shaped member is t ($\mu$m), and in which the sound insulation structure is disposed so as to close the housing opening part.

[6] The earmuff according to [1] in which the sound insulation structure includes a plate-shaped member having a plurality of through-holes penetrating in a thickness direction, and a frame having a frame hole part, and the plate-shaped member performs film vibration by fixing the plate-shaped member to a circumferential edge of the frame hole part of the frame, in which an average opening diameter of the through-holes is 0.1 $\mu$m or more and 250 $\mu$m or less, and in which a first natural vibration frequency of the film vibration of the plate-shaped member is present between 10 Hz to 100000 Hz.

[7] The earmuff according to [1] in which the sound insulation structure has one or more sound insulation cells, in which the one or more sound insulation cells each include a frame having a frame hole part penetrating thereof, and a film that covers the frame hole part and is fixed to the frame, and in which the sound insulation structure is disposed in the housing opening part in a state where a film surface of the film is inclined with respect to an opening cross-section of the housing opening part and the housing opening part is provided with a region serving as a ventilation hole through which gas passes.

[8] The earmuff according to [7] in which the film has a plurality of through-holes penetrating in a thickness direction thereof, and in which an average opening diameter of the through-holes is 0.1 $\mu$m or more and 250 $\mu$m or less.

[9] The earmuff according to any one of [1] to [8] further comprising a sound insulating material disposed within the housing.

[10] The earmuff according to any one of [1] to [9] in which two or more sound insulation structures in which frequency ranges of sound to be insulated are different from each other are disposed in the housing opening part.

[11] The earmuff according to any one of [1] to [10] in which the sound insulation structure is attachably and detachably disposed in the housing opening part.

[12] The earmuff according to any one of [1] to [11] further comprising a case having a case opening part penetrating thereof, in which the case opening part includes a cassette member in which the sound insulation structure is disposed, and in which the cassette member is attachably and detachably disposed in the housing.

According to the invention it is possible to provide an earmuff that is compact and lightweight, has high sound insulating performance, is capable of insulating sound of a specific frequency range, and has excellent air permeability.

## BRIEF DESCRIPTION OF THE DRAWINGS

Fig. 1 is a cross-sectional view schematically illustrating an example of an earmuff of the invention.
Fig. 2A is a cross-sectional view of an ear cup of Fig. 1.
Fig. 2B is a side view of Fig. 2A as seen from direction b.

Fig. 2C is a side view of Fig. 2A as seen from direction c.

Fig. 3 is a cross-sectional view schematically illustrating another example of the earmuff of the invention.

Fig. 4 is a cross-sectional view schematically illustrating still another example of the earmuff of the invention.

Fig. 5A is a cross-sectional view schematically illustrating a still further example of the earmuff of the invention.

Fig. 5B is a cross-sectional view schematically lustrating a still further example of the earmuff of the invention.

Fig. 6 is a front view schematically illustrating an example of a sound insulation structure to be used for the earmuff of the invention.

Fig. 7 is a cross-sectional view taken along line II-II of Fig. 6.

Fig. 8 is a front view schematically illustrating another example of the sound insulation structure to be used for the earmuff of the invention.

Fig. 9 is a front view schematically illustrating still another example of the sound insulation structure to be used for the earmuff of the invention.

Fig. 10A is a front view schematically illustrating a still further example of the sound insulation structure to be used for the earmuff of the invention.

Fig. 10B is a cross-sectional view taken along line B-B illustrated in Fig. 10A.

Fig. 11 is a front view schematically illustrating a still further example of the sound insulation structure to be used for the earmuff of the invention.

Fig. 12 is a cross-sectional view taken along line II-II of Fig. 11.

Fig. 13 is a front view schematically illustrating a still further example of the sound insulation structure to be used for the earmuff of the invention.

Fig. 14 is a cross-sectional view taken along line B-B of Fig. 13.

Fig. 15A is a schematic cross-sectional view for illustrating an example of a preferred method of manufacturing the sound insulation structure of Fig. 13.

Fig. 15B is a schematic cross-sectional view for illustrating the example of the preferred method of manufacturing the sound insulation structure of Fig. 13.

Fig. 15C is a schematic cross-sectional view for illustrating the example of the preferred method of manufacturing the sound insulation structure of Fig. 13.

Fig. 15D is a schematic cross-sectional view for illustrating the example of the preferred method of manufacturing the sound insulation structure of Fig. 13.

Fig. 15E is a schematic cross-sectional view for illustrating the example of the preferred method of manufacturing the sound insulation structure of Fig. 13.

Fig. 16 is a graph showing a relationship between average opening diameter, an average opening ratio, and absorbance.

Fig. 17 is a graph showing a relationship between the average opening diameter, the average opening ratio, and the absorbance.

Fig. 18 is a graph showing a relationship between the average opening diameter and the average opening ratio at which the absorbance has a local maximum.

Fig. 19 is a graph showing a relationship between the average opening diameter and the local maximum absorbance.

Fig. 20 is a graph showing a relationship between the average opening diameter and the absorbance.

Fig. 21 is a graph showing a relationship between the average opening ratio and acoustic characteristics.

Fig. 22 is a graph showing a relationship between the average opening ratio and the acoustic characteristics.

Fig. 23 is a graph showing a relationship between average opening diameter and an optimal average opening ratio.

Fig. 24 is a graph showing a relationship between average opening diameter and the optimal average opening ratio.

Fig. 25 is a graph showing a relationship between the average opening ratio and maximum absorbance.

Fig. 26 is a graph showing a relationship between the average opening ratio and the maximum absorbance.

Fig. 27 is a front view schematically illustrating a still further example of the sound insulation structure to be used for the earmuff of the invention.

Fig. 28 is a cross-sectional view taken along line II-II of Fig. 27.

Fig. 29 is a cross-sectional view schematically illustrating a still further example of the earmuff of the invention.

Fig. 30A is a cross-sectional view of an ear cup of Fig. 29.

Fig. 30B is a side view of Fig. 30A as seen from direction b.

Fig. 30C is a side view of Fig. 30A as seen from direction c.

Fig. 31 is a front view schematically illustrating a still further example of the sound insulation structure to be used for the earmuff of the invention.

Fig. 32 is a cross-sectional view taken along line II-II of Fig. 31.

Fig. 33A is a graph showing sound absorption characteristic expressed by the absorbance with respect to frequency.

Fig. 33B is a graph showing sound shielding characteristic expressed by transmission loss with respect to the frequency.

Fig. 34 is a perspective view illustrating a measurement system that measures the sound insulating performance of the sound insulation structure that is arranged to be inserted into a tubular opening part.

Fig. 35 is an illustrative view illustrating the inclination angle of a film surface of the sound insulation structure with respect to the opening cross-section of the opening part.

Fig. 36 is a graph showing the dependability of sound shielding performance on the inclination angle of a film surface.

Fig. 37 is an illustrative view illustrating a relationship between the inclination angle of the film surface of the sound insulation structure and the traveling direction of sound waves.

Fig. 38A is a graph showing the dependability of the sound shielding characteristic on the inclination angle of the film surface.

Fig. 38B is a graph showing the dependability of the sound absorption characteristic on the inclination angle of the film surface.

Fig. 38C is a graph showing the dependability of the sound shielding characteristic on the inclination angle of the film surface.

Fig. 38D is a graph showing the dependability of the sound absorption characteristic on the inclination angle of the film surface.

Fig. 38E is a graph showing the dependability of the sound shielding characteristic on the inclination angle of the film surface.

Fig. 38F is a graph showing the dependability of the sound absorption characteristic on the inclination angle of the film surface.

Fig. 39 is a graph showing the dependability of the sound shielding characteristic (transmission loss) on sound wave incidence angle.

Fig. 40A is a graph showing a relationship between the frequency and the absorbance.

Fig. 40B is a graph showing a relationship between the frequency and the transmission loss.

Fig. 41A is a graph showing a relationship between the frequency and the absorbance.

Fig. 41B is a graph showing a relationship between the frequency and the transmission loss.

## DESCRIPTION OF THE PREFERRED EMBODIMENTS

Hereinafter, an earmuff related to the invention will be described in detail with reference to preferred embodiments illustrated in the attached drawings.

Although the description of constituent elements to be described below is made on the basis of the representative embodiments of the invention, the invention is not limited to such embodiments.

In addition, numerical ranges expressed using "to" in the present specification mean ranges including numerical values described before and after "to" as lower limits and upper limits.

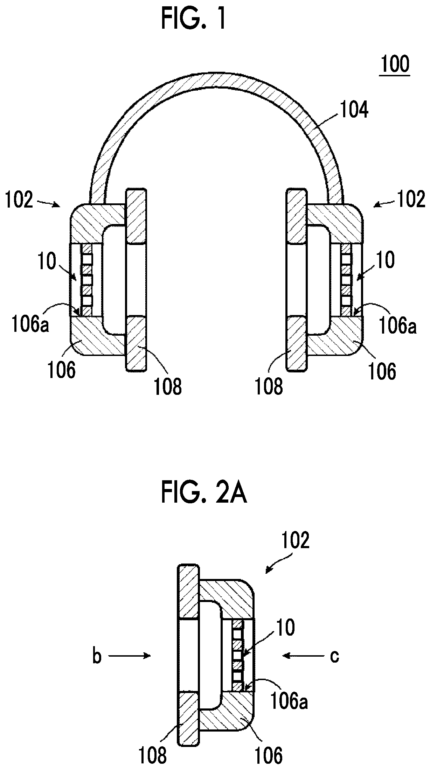

The earmuff of the invention includes a supporting member, and two ear cups each having a housing attached to the supporting member and an ear pad locked to the housing, the housing has a housing opening part, a sound insulation structure is disposed in the housing opening part and insulates sound of a specific frequency range, and the housing opening part having the sound insulation structure disposed therein has a ventilation hole. The configuration of one embodiment of the earmuff of the invention will be described with reference to Figs. 1 and 2A to 2C.

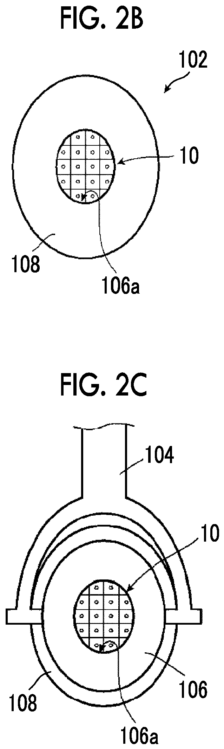

Fig. 1 is a cross-sectional view schematically illustrating an example of the earmuff of the invention, Fig. 2A is a cross-sectional view of an ear cup of the earmuff illustrated in Fig. 1, Fig. 2B is a side view of Fig. 2A as seen from direction b, and Fig. 2C is a side view of Fig. 2A as seen from direction c.

An earmuff 100 illustrated in Figs. 1 and 2A to 2C has a headband 104 and two ear cups 102 attached to both end parts of the headband 104.

The headband 104 is a supporting member in the invention, and is the same as a well-known headband used for related-art earmuffs, related-art headphones, and the like. In the illustrated example, the headband 104 has a shape in which a long plate-shaped member is bent, and includes support parts, which rotatably support the ear cups, at both end parts thereof. Additionally, the headband 104 has a spring property in a bending direction, and presses the two ear cups 102 against both a user's ears to elastically hold the ears.

In addition, the supporting member is not limited to the above headband. For example, the ear cups may be fixed to positions corresponding to the positions of the ears in a case where the user wears a helmet with the helmet as the supporting member.

The ear cups 102 cover the user's ears to shield noise or the like from the outside. The ear cups 102 each have a housing 106, an ear pad 108, and a sound insulation structure 10.

The housing 106 is the same as a housing of an ear cup used in the related-art earmuffs, the related-art headphones, and the like except having a housing opening part 106a for disposing the sound insulation structure 10 therein.

Specifically, the housing 106 is a substantially bowl-like member and is rotatably supported by an end part of the headband 104. Additionally, the ear pad 108 is locked to a substantially bowl-like opening side.

Additionally, the housing 106 has the housing opening part 106a formed to penetrate therethrough. The sound insulation structure 10 to be described below is disposed in the housing opening part 106a.

The position and size of the housing opening part 106a are not limited, and may be appropriately set in accordance with desired sound insulating performance, desired air permeability, or the like.

Additionally, the shape of the opening cross-section of the housing opening part 106a is not also limited, and can be various shapes, such as a circular shape, an elliptical shape, a square shape, an oblong shape, and a polygonal shape.

Materials for forming the housing 106 are not limited, and various resin materials and metallic materials that are used as materials of the housing of the ear cup of the related-art earmuffs, the related-art headphones, and the like are variously available.

The ear pad 108 is the same as an ear pad used in the related-art earmuffs, the related-art headphones, and the like. As an example, the ear pad 108 is a member that covers a substantially annular cushioning member having a predetermined thickness with a skin material, and is locked to the opening side of the substantially bowl-like housing 106. A pressing force in a case where the earmuff is worn can be decentralized by the ear pad 108, and the user can comfortably continue to wear the earmuff.

Materials for forming the ear pad 108 are not limited, and various resin materials that are used as materials of the ear pad of the related-art earmuffs, the related-art headphones, and the like are variously available.

For example, as the cushioning member of the ear pad 108, foams, such as polyurethane and polyvinyl chloride, can be used. Additionally, as skin materials, resin thin films, woven fabric (for example, cloth), natural leather, and the like can be used.

The sound insulation structure 10 insulates the sound of the specific frequency range that is disposed in the housing opening part 106a of the housing 106.

As illustrated in a Fig., the sound insulation structure 10 is disposed within the housing opening part 106a so as to close the housing opening part 106a.

Here, the sound insulation structure 10 has a plurality of holes that penetrate therethrough in a thickness direction thereof as will be described above. For that reason, although the sound insulation structure 10 covers the housing opening part 106a, the holes formed in the sound insulation structure 10 become ventilation holes.

Hence, the earmuff 100 of the invention can insulate the sound of the specific frequency range with high sound insulating performance and make the air permeability high, using the sound insulation structure 10, without increasing the weight and the size of the ear cups 102. The configuration of the sound insulation structure 10 will be described in detail below.

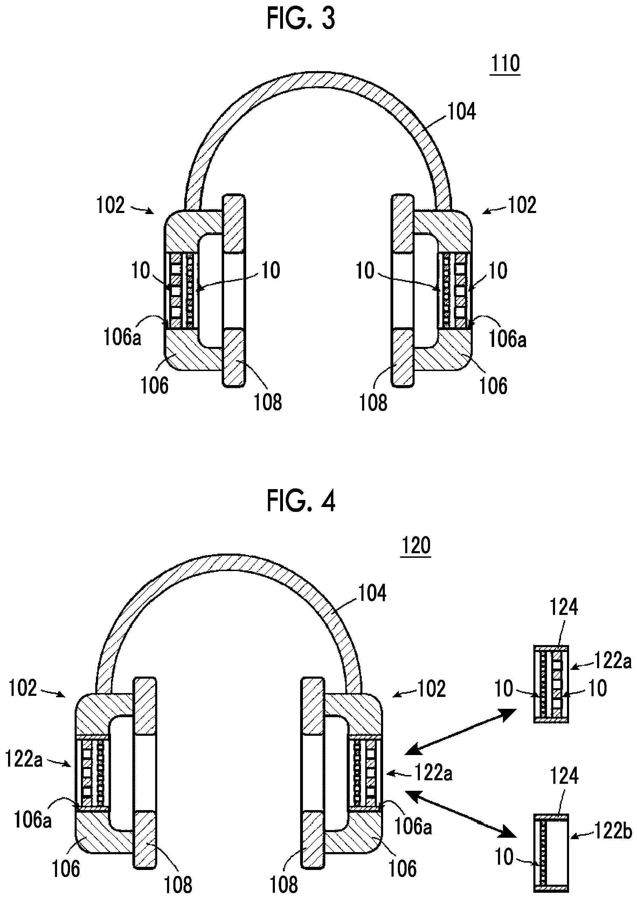

Here, in the example illustrated in Fig. 1, a configuration in which one sound insulation structure 10 is disposed in the housing opening part 106a of each of the two ear cups 102 is adopted. However, the invention is not limited to this. For example, as in an earmuff 110 illustrated in Fig. 3, a configuration in which two or more sound insulation structures 10 are disposed in one housing opening part 106a may be adopted. In a case where the two or more sound insulation structures 10 are disposed, as illustrated in Fig. 3, the sound insulation structures may be arranged in a direction perpendicular to an opening cross-section of the housing opening part 106a.

Additionally, in a case where the two or more sound insulation structures 10 are disposed in the one housing opening part 106a, it is preferable to use the sound insulation structures 10 that insulate sound of mutually different frequency ranges. By using the two or more sound insulation structures 10 that insulate the sound of the mutually different frequency ranges, sound of two or more types of frequency ranges or a wider frequency range can be insulated.

Additionally, a configuration in which the sound insulation structures 10 are attachably and detachably disposed in the housing opening part 106a may be adopted.

In a case where disposing the sound insulation structures 10 are attachably and detachably disposed in the housing opening part 106a, as in an earmuff 120 illustrated in Fig. 4, a configuration in which cassette members 122 (122a, 122b) each including the sound insulation structures 10 are inserted into the housing opening part 106a and the cassette members 122 are detachably disposed may be adopted.

In addition, in Fig. 4, different types of cassette members are respectively illustrated using reference signs of 122a and 122b. However, in a case where the types of cassette members do not need to be distinguished from each other, these cassette members are collectively referred to as the cassette members 122.

The cassette members 122 each include a case 124 having a case opening part 124a penetrating therethrough, and one or more sound insulation structures 10 disposed in the case opening part 124a.

The case 124 has the case opening part 124a of which the outer shape of a cross-sectional shape has the same size and shape as those of the opening cross-section of the housing opening part 106a of the housing 106 and which penetrates in the same direction as a penetration direction of the housing opening part 106a.

The sound insulation structures 10 are disposed in the case opening part 124a so as to close the case opening part 124a.

In this way, by making the sound insulation structures 10 attachable to and detachable from the housing opening part 106a as the cassette members 122, replacement of the sound insulation structures 10 can be easily performed.

For example, as in the illustrated example, by appropriately replacing the cassette member 122a in which two sound insulation structures 10 are disposed in the case opening part 124a and the cassette member 122b in which one sound insulation structure 10 is disposed in the case opening part 124a, in accordance with a frequency range of the sound to be insulated, sound of a desired frequency range can be easily insulated.

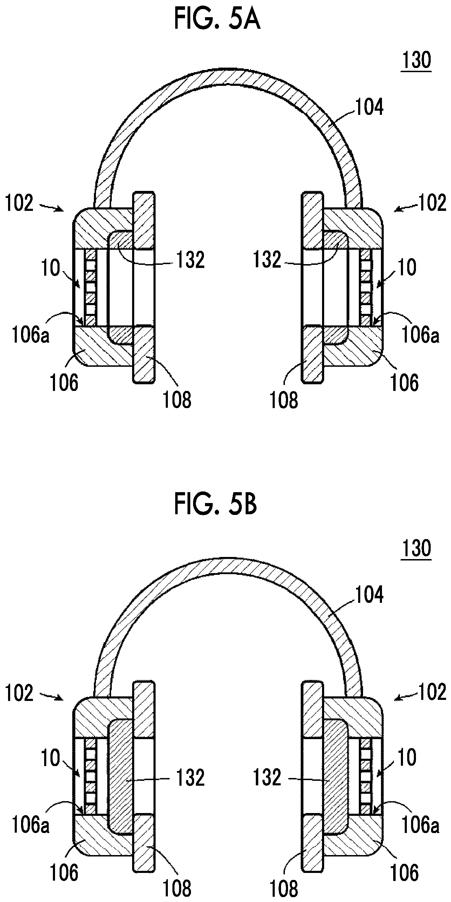

Additionally, as in an earmuff 130 illustrated in Fig. 5A, a sound insulating material 132 may be disposed within the housing 106 of the ear cup 102.

The sound insulating material 132 is not limited, and various sound insulating materials that are used in the related-art earmuffs, the related-art headphones, and the like are variously available.

In addition, as illustrated in Fig. 5A, the sound insulating material 132 may be formed in an annular shape having an opening with the same size as the housing opening part 106a, at a position corresponding to a housing opening part 106a, or may have a shape that has no opening as illustrated in Fig. 5B.

Next, a specific configuration of the sound insulation structure 10 will be described.

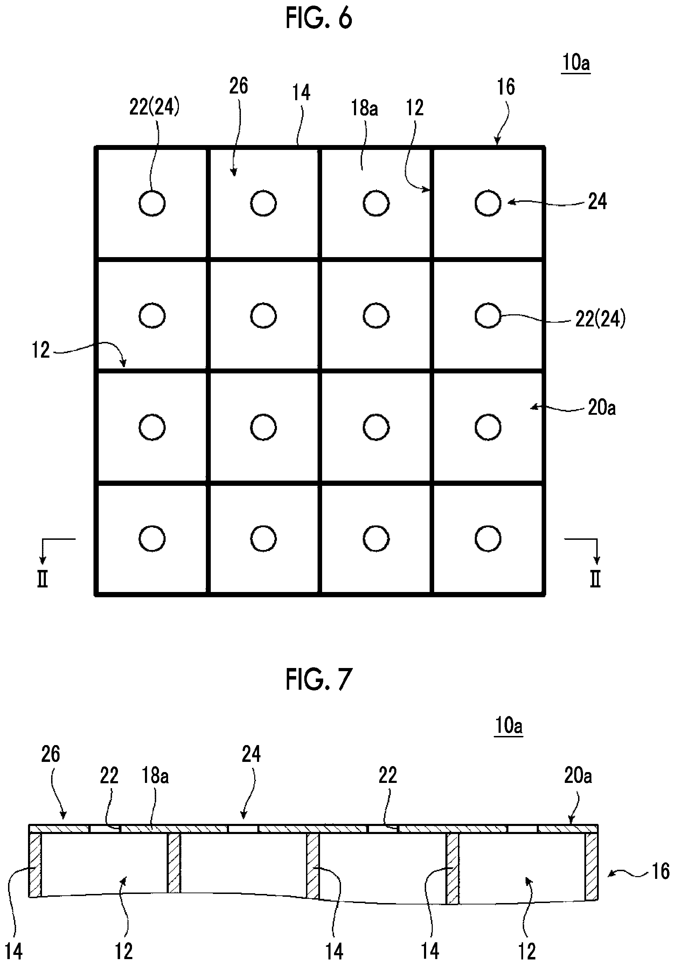

Fig. 6 is a front view schematically illustrating an example of the sound insulation structure, and Fig. 7 is a cross-sectional view taken along line II-II of Fig. 6.

A sound insulation structure 10a illustrated in Figs. 6 and 7 has a frame body 16 that forms a plurality of (sixteen in the illustrated example) frames 14 which have frame hole parts 12, respectively, and are two-dimensionally disposed; a sheet-like film body 20a that is fixed to the respective frames 14 so as to cover the frame hole parts 12 of the respective frames 14 and forms a plurality of (sixteen in the illustrated example) films 18a; and a plurality of (sixteen in the illustrated example) opening parts 24 including one or more (one in the illustrated example) through-holes 22 pierced so as to penetrate the films 18a within the respective frames 14.

In the sound insulation structure 10a, one frame 14, a film 18a fixed to the frame 14, and an opening part 24 provided in the films 18a constitute one sound insulation cell 26. For this reason, the sound insulation structure 10a is constituted of a plurality of (sixteen in the illustrated example) the sound insulation cells 26.

Although the sound insulation structure 10a of the illustrated example is constituted of the plurality of sound insulation cells 26, the sound insulation structure 10a is limited to this, and may be constituted of one sound insulation cell 26 including one frame 14, one film 18a, and one opening part 24.

A frame 14 is formed so as to be annularly surrounded with a thick plate-shaped member, has a frame hole part 12 therein, is for fixing a film 18a so as to cover the frame hole part 12 on at least one side, and becomes a film vibration node of the film 18a fixed to the frame 14. Hence, compared to the film 18a, the frame 14 needs to have higher stiffness, specifically, have higher mass per unit area and stiffness.

Although it is preferable that the shape of the frame 14 is a continuous closed shape capable of fixing the film 18a such that the entire outer periphery of the film 18a can be suppressed, the invention is not limited to this. The frame 14 may be a partially cut discontinuous shape as long as the frame 14 becomes a film vibration node of the film 18a fixed to the frame. That is, since the role of the frame 14 is to fix the film 18a to control the film vibration, an effect is exhibited even in a case where a small cut is made in the frame 14 or even in a case where a region that is not bonded extremely slightly is present.

Additionally, although the geometrical form of the frame hole part 12 formed by the frame 14 is a planar shape, and a square in the example illustrated in Fig. 6, the geometrical form is not particularly limited in the invention. For example, the geometrical form of the frame hole part may be another quadrangular shape, such as a rectangle, a rhombus, or a parallelogram; a triangle, such as an equilateral triangle, an isosceles triangle, or a right-angled triangle; a polygon including a regular polygon, such as a regular pentagon or a regular hexagon; a circle; or an ellipse, or may be an indefinite shape.

Additionally, the size of the frame 14 is a size in plan view and can be defined as the size of the frame hole part 12. However, in the case of the regular polygon like the square illustrated in Fig. 6, or the circle, the size of the frame 14 can be defined as a distance between opposed sides passing through the center of the regular polygon or a circle-equivalent diameter, and in the case of the polygon, the ellipse, or the indefinite shape, the size of the frame 14, the size of the frame 14 can be defined as a circle-equivalent diameter. In the invention, the circle-equivalent diameter and a circle-equivalent radius are respectively a diameter and a radius in a case where the geometrical form is converted into a circle having equivalent area.

In addition, in the sound insulation structure 10 of the invention, in all the frames 14, the sizes of the frames 14 may be constant. However, frames of different sizes (also including a case where shapes are different from each other) may be included. In this case, the average size of the frames 14 may be used as the size of each frame 14.

Such a size of the frame 14 is not particularly limited, and may be set in accordance with the frequency range or the like of the sound to be insulated.

In addition, although details will be described below, it is preferable to make the size of each frame 14 small in order to obtain the natural frequency mode of the structure including the frames 14 and the films 18a on a high-frequency side.

Additionally, although the average size of the frames 14 will be described in detail below, it is preferable that the average size is equal to or less than a wavelength size corresponding to a shielding peak frequency to be described below in order to prevent leakage of sound resulting from diffraction in the shielding peak of the sound insulation cell 26 formed by the opening part 24 including the hole provided in each film 18a.

For example, the size of the frame 14 is preferably 0. 5 mm to 200 mm, more preferably 1 mm to 100 mm, and most preferably 2 mm to 30 mm. In addition, in a case where different sizes are included in the respective frames 14, it is preferable that the size of each frame 14 is expressed by the average size.

Additionally, the width and the thickness of the frame 14 can also be fixed so as to reliably suppress the film 18a. Although not particularly limited as long as the film 18a can be reliably supported, the width and the thickness can be set in accordance with, for example, the size of the frame 14.

For example, the width of the frame 14 is preferably 0.5 mm to 20 mm, more preferably 0.7 mm to 10 mm, and most preferably 1 mm to 5 mm.

In a case where the ratio of the width of the frame 14 to the size of the frame 14 becomes excessively large, there is a concern that the area ratio of the frame 14 occupying a portion with respect to the total becomes large and the device becomes heavy. On the other hand, in a case where the above ratio becomes excessively small, it becomes difficult to strongly fix the film in the portion of the frame 14 with an adhesive or the like.

In addition, in the invention, it is preferable that the plurality of, that is, two or more frames 14 are constituted as the frame body 16 disposed so as to be two-dimensionally connected to each other.

Here, the number of frames 14 of the sound insulation structure 10a, that is, in the illustrated example, the number of frames 14 that constitute the frame body 16 is also not particularly limited, and may be set in accordance with the frequency range of the sound to be insulated. Alternatively, since the above-described size of the frame 14 is set in accordance with the frequency range of the sound to be insulated, the number of frames 14 may be set in accordance with the size of the frame 14.

This is because the size of the earmuff is substantially limited and because it is necessary to shield, that is, reflect and/or absorb noise with the frame body 16 in which the plurality of sound insulation cells 26 are combined with each other in order to set the size of one sound insulation cell 26 to a size suitable for the frequency of the noise.

In addition, since one sound insulation cell 26 has one frame 14 as a constitutional unit, the number of frames 14 of the sound insulation structure 10a can also be referred to as the number of sound insulation cells 26.

Materials of the frame 14, that is, materials of the frame body 16 are not particularly limited and can be selected in accordance with sound insulating environments, as long as the film 18a can be supported, a strength suitable for being disposed in the housing opening part 106a of the ear cups 102 is provided, and there is resistance against the sound insulating environments. The materials of the frame 14 may include, for example, metallic materials, such as aluminum, titanium, magnesium, tungsten, iron, steel, chromium, chromium molybdenum, nichrome molybdenum, and alloys thereof; resin materials, such as acrylic resin, polymethylmethacrylate, polycarbonate, polyamideimide, polyarylate, polyetherimide, polyacetal, polyetheretherketone, polyphenylene sulfide, polysulfone, polyethylene telephthlate, polybutylene telephthlate, polyimide, and triacetylcellulose; carbon fiber reinforced plastic (CFRP); carbon fiber; glass fiber reinforced plastic (GFRP), and the like. Additionally, a plurality of types of these materials of the frame 14 may be used in combination.

The film 18a is fixed so as to cover the frame hole part 12 inside the frame 14 and be suppressed by the frame 14, and performs the film vibration in correspondence with sound waves from the outside, thereby absorbing or reflecting and insulating the energy of the sound waves. For that reason, it is preferable that the film 18a is impermeable to air.

Meanwhile, since it is necessary for the film 18a to perform the film vibration with the frame 14 as a node, the film 18a needs to be fixed so as to be reliably suppressed by the frame 14, become a belly of the film vibration, and absorb or reflect and insulate the energy of the sound waves. For this reason, it is preferable that the film 18a is made of flexible elastic materials.

For this reason, the shape of the film 18a is the shape of the frame hole part 12 of the frame 14. Additionally, it can be said that the size of the film 18a is the size of the frame 14, more specifically, the size of the frame hole part 12 of the frame 14.

Here, the film 18a fixed to the frame 14 of the sound insulation cell 26 has a first natural vibration frequency at which the transmission loss is minimum, for example, 0 dB, as a resonant frequency that is the frequency of a lowest-order natural frequency mode. Since the first natural vibration frequency is determined depending on a structure including the frame 14 and the film 18a, the present inventors have found out that the first natural vibration frequency has substantially the same value irrespective of the presence or absence of the through-hole 22 (opening part 24) pierced in the film 18a.

Here, the first natural vibration frequency of the film 18a fixed so as to be suppressed by the frame 14 in the structure including the frame 14 and the film 18a is the frequency of a natural frequency mode in which sound waves are greatly transmitted at the frequency thereof in a location where the sound waves vibrate with the film vibration most due to a resonance phenomenon.

In addition, according to the knowledge of the present inventors, in the sound insulation structure 10a, the through-hole 22 that constitutes the opening part 24 including the through-hole 22 is pierced as a through-hole in the film 18a. Therefore, the peak of shielding of the sound waves at which the transmission loss has a peak (local maximum) appears at a shielding peak frequency on a lower frequency side than the first natural vibration frequency. Additionally, particularly, from the peak of shielding caused by the penetrating through-hole 22, an increase in absorption of sound resulting from the presence of the penetrating through-hole 22 is observed on the lower frequency side.

Hence, since the shielding (transmission loss) has a peak (local maximum) at the shielding peak frequency, the sound insulation structure 10a can selectively insulate sound of a certain frequency range centered on the shielding peak frequency.

In the sound insulation structure 10a as illustrated in Fig. 6, firstly, the shielding of sound can be increased and the peak of shielding can be controlled. However, in addition to these, there is a feature that the absorption of sound (the energy of the sound waves) appears on the lower frequency side due to the effect of the penetrating through-hole 22.

For this reason, in the structure including the frame 14 and the film 18a, in order to set the shielding peak frequency depending on the opening part 24 including one or more through-holes 22 to an arbitrary frequency within an audible range, it is important to obtain the natural frequency mode as close to the high-frequency side possible, and this is particularly important for practical use. Therefore, it is preferable to thicken the film 18a, it is preferable to set the Young's modulus of a material of the film 18a to a large value, and it is preferable to make the size of the frame 14, that is, the size of the film 18a small, as described above. Hence, in the invention, these preferable conditions become important.

Thus, since the sound insulation structure 10a follows rigid rules, and cause shielding of sound waves at a frequency less than the first natural vibration frequency of the film 18a fixed to the frame 14, the first natural vibration frequency of the film 18a is preferably 10 Hz to 100000 Hz equivalent to a human being's sensing range for sound waves, more preferably 20 Hz to 20000 Hz that is a human being's audible range for sound waves, further more preferably 40 Hz to 16000 Hz, and most preferably 100 Hz to 12000 Hz.

Additionally, although the thickness of the film 18a is not particularly limited as long as the film vibration can be performed in order to absorb or reflect and insulate the energy of sound waves, it is preferable to thicken the film in order to obtain the natural frequency mode on the high-frequency side. For example, in the invention, the thickness of the film 18a can be set in accordance with the size of the frame 14, that is, the size of the film.

For example, the thickness of the film 18 is preferably 0.005 mm (5 $\mu$m) to 5 mm, more preferably 0.007 mm (7 $\mu$m) to 2 mm, and most preferably 0.01 mm (10 $\mu$m) to 1 mm.

Here, in the sound insulation structure 10a, the first natural vibration frequency of the film 18a in the structure including the frame 14 and the film 18a can be determined depending on the geometric form (for example, the shape and the dimension (size) of the frame 14) of the frame 14 of each of the plurality of sound insulation cells 26, and the stiffness (for example, the thickness and the flexibility of the film) of the film of each of the plurality of sound insulation cells.

In addition, as parameters characterizing a first natural frequency mode of the film 18a, in the case of the film 18a of the same kind of material, a ratio between a thickness (t) of the film 18a and square of a size (a) of the frame 14 (for example, a ratio with respect to the size of one side [$a^2/t$]) in the case of a square) can be used. In a case where this ratio [$a^2/t$] is equal (for example, in a case where (t, a) is (50 $\mu$m, 7.5 mm) and in a case where (t, a) is (200 $\mu$m, 15 mm)), the above first natural frequency mode has the same frequency, that is, the same first natural vibration frequency. That is, by setting the ratio [$a^2/t$] to a certain value, the scale rule is established and a suitable size can be selected.

Additionally, although the Young's modulus of the film 18a is not particularly limited as long as the film 18a has elasticity capable of performing the film vibration in order to absorb or reflect and insulate the energy of sound waves, it is preferable to increase the Young's modulus in order to obtain the natural frequency mode on the high-frequency side. For example, in the invention, the Young's modulus of the film 18a can be set in accordance with the size of the frame 14, that is, the size of the film.

For example, the Young's modulus of the film 18a is preferably 1000 Pa to 3000 GPa, more preferably 10000 Pa to 2000 GPa, and most preferably 1 MPa to 1000 GPa.

Additionally, the density of the film 18a is also not particularly limited as long as the film vibration can be performed in order to absorb or reflect and insulate the energy of sound waves. For example, the density is preferably 10 kg/m$^3$ to 30000 kg/m$^3$, more preferably 100 kg/m$^3$ to 20000 kg/m$^3$, and most preferably 500 kg/m$^3$ to 10000 kg/m$^3$.

Materials of the film 18a are not particularly limited as long as a strength suitable at the time of being applied to the above-described sound insulated object is given in a case where the materials are used as film-like materials or foil-like materials, there is resistance against the sound insulating environments of the sound insulated object, and the film 18a can perform the film vibration in order to absorb or reflect and insulate the energy of sound waves, and can be selected in accordance with the sound insulated object, the sound insulating environments thereof, or the like. For example, materials for the film 18a may include materials, structures, or the like capable of forming a thin structure, such as resin materials capable of being made into a film, such as polyethylene terephthalate (PET), polyimide, polymethylmethacrylate, polycarbonate, acrylic (PMMA), polyamideimide, polyarylate, polyetherimide, polyacetal, polyetheretherketone, polyphenylene sulfide, polysulfone, polyethylene telephthlate, polybutylene telephthlate, polyimide, triacetylcellulose, polyvinylidene chloride, low-density polyethylene, high-density polyethylene, aromatic polyamide, silicone resin, ethylene ethyl acrylate, vinyl acetate copolymers, polyethylene, chlorinated polyethylene, polyvinyl chloride, polymethylpentene, and polybutene; metallic materials capable of being made into a foil, such as aluminum, cromium, titanium, stainless steel, nickel, tin, niobium, tantalum, molybdenum, zirconium, gold, silver, platinum, palladium, iron, copper, and permalloy; materials that become other fibrous films, such paper and cellulose; porous materials, such as non-woven fabrics, films including nano-size fiber, and thinly processed urethane and synthrate; and carbon materials processed into a thin film structure.

The film 18a may be individually fixed to each of the plurality of frames 14 of the frame body 16 of the sound insulation structure 10a to constitute the sheet-like film body 20a as a whole, and conversely, the film 18a that covers each frame 14 may be formed by one sheet-like film body 20a fixed so as to cover all the frames 14. Otherwise, as an intermediate between these, a film 18a that fixes a sheet-like film body to some frames 14 to cover the respective frames 14 so as to cover some of the plurality of frames 14 may be formed, and a sheet-like film body 20a that covers all (all the frames 14) the plurality of frames 14 using some of these sheet-like film bodies may be configured.

Additionally, the film 18a is fixed to the frame 14 so as to cover an opening at least on one side of the frame hole part 12 of the frame 14. That is, the film 18a may be fixed to the frame 14 so as to cover an opening on one side or the other side or openings on both sides of the frame hole part 12 of the frame 14.

Here, all the films 18a may be provided on the same sides of the frame hole parts 12 of the plurality of frames 14 of the sound insulation structure 10a; some films 18a may be provided on one sides of some frame hole parts 12 of the plurality of frames 14 and the remaining films 18a may be provided on the other sides of the remaining some frame hole parts 12 of the plurality of frames 14; and films provided on one sides, on the other sides, on both sides of the frame hole parts 12 of the frames 14 may be present in a mixed manner.

A method of fixing the film 18a to the frame 14 is not particularly limited, and may be any method as long as the film 18a can be fixed to the frame 14 so as to become the film vibration node, and may include, for example, a method using an adhesive, a method using a physical fixture, or the like.

In the method using an adhesive, an adhesive is coated on a surface that surrounds the frame hole part 12 of the frame 14, the film 18a is placed thereon, and the film 18a is fixed to the frame 14 with the adhesive. The adhesive may include, for example, an epoxy adhesive (Araldite or the like), a cyanoacrylate adhesive (Aron Alpha or the like), an acrylic adhesive, or the like.

The method using a physical fixture may include a method of sandwiching the film 18a disposed so as to cover the frame hole part 12 of the frame 14 between the frame 14 and a fixing member, such as a rod, and fixing the fixing member to the frame 14, using a fixture, such as a screw, a machine screw, or the like.

Additionally, a configuration in which the frame 14 and the film 18a are made of the same material and are integrally formed may be adopted.

The configuration in which the frame 14 and the film 18a are integrated with each other can be manufactured by simple processes, such as compression molding, injection molding, imprinting, shaving processing, a processing method using a three-dimensional shape forming (3D) printer.

The film 18a, that is, the sound insulation cell 26 has the opening part 24 including one or more through-holes 22.

As illustrated in Figs. 6 and 7, the sound insulation structure 10 has a peak of the transmission loss at which the shielding has a peak (local maximum) on the lower frequency side than the first natural vibration frequency of the film 18a by having the opening part 24 including one or more through-holes 22 pierced in the film 18a. A frequency at which this shielding (transmission loss) has a peak (local maximum) is referred to as the shielding peak frequency.

This shielding peak frequency appears due to the through-hole 22 of the opening part 24 on the lower frequency side than the first natural vibration frequency that mainly depends on the film 18a of the sound insulation cell 26 of the sound insulation structure 10a. The shielding peak frequency is determined in accordance with the size of the opening part 24 with respect to the size of the frame 14 (or the film 18a), specifically, the opening ratio of the opening part 24 that is the percentage of the total area of the through-hole 22 to the area of the frame hole part 12 of the frame 14 (or the film 18a that covers the frame hole part 12).

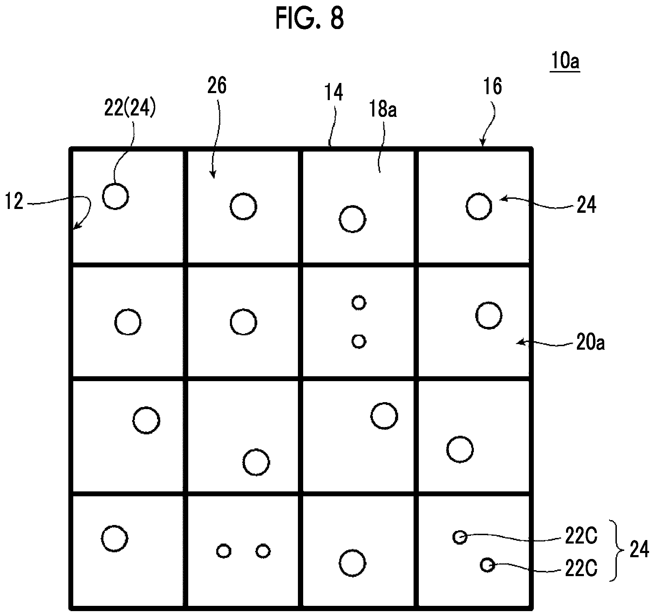

Here, as illustrated in Fig. 6, one or more through-holes 22 may be pierced within the film 18a that covers the frame hole part 12 of the sound insulation cell 26. Additionally, as illustrated in Fig. 6, the piercing position of the through-hole 22 may be in the middle of the sound insulation cell 26 or the film 18a (hereinafter, represented by the sound insulation cell 26). However, the invention is not limited to this, and as illustrated in Fig. 8, the piercing position may not be in the middle of the sound insulation cell 26 or may be pierced at any position.

That is, simply by changing the piercing position of the through-hole 22, the sound shielding characteristic of the sound insulation structure 10a does not change.

Additionally, as is illustrated in Fig. 6, the number of through-holes 22 that constitute the opening part 24 within the sound insulation cell 26 may be one with respect to one sound insulation cell 26. However, the invention is not limited to this, and two or more (that is, a plurality of) through-holes may be provided as illustrated in Fig. 8.

Here, as illustrated in Fig. 6, it is preferable that the opening part 24 of each sound insulation cell 26 is constituted of one through-hole 22 from a viewpoint of the air permeability. The reason is that, in the case of a certain opening ratio, the easiness of flow of air as wind is larger in a case where one hole is large and the viscosity at a boundary does not work greatly.

Meanwhile, in a case where a plurality of through-holes 22 are present within one sound insulation cell 26, the sound shielding characteristic of the sound insulation structure 10a show a corresponding sound shielding peak in a sound shielding characteristic (that is, a corresponding sound shielding peak frequency) corresponding to the total area (that is, the area of the opening part 24) of the plurality of through-holes 22. Hence, as illustrated in Fig. 8, it is preferable that the area of the opening part 24 that is the total area of the plurality of through-holes 22C within one sound insulation cell 26 (or the film 18a) is equal to the area of the opening part 24 that is the area of only one through-hole 22 within another sound insulation cell 26 (or the film 18a). However, the invention is not limited to this.

In addition, in a case where the opening ratio of the opening part 24 within the sound insulation cell 26 (the area ratio of the opening part 24 to the area of the film 18a that covers the frame hole part 12 (the percentage of the total area of all the through-holes 22)) is the same, the same sound insulation structure 10a is obtained by the single through-hole 22 and the plurality of through-holes 22. Therefore, sound insulation structures of various frequency ranges can be manufactured even in a case where the sizes of the through-holes are fixed to the size of a certain through-hole 22.

In the sound insulation structure 10a, the opening ratio (area ratio) of the opening part 24 within the sound insulation cell 26 is not particularly limited and may be set in accordance with a sound shielding frequency range of the noise to be selectively shielded. However, the opening ratio is preferably 0.000001% to 70%, more preferably 0.000005% to 50%, and most preferably 0.00001% to 30%. By setting the opening ratio of the opening part 24 to the above ranges, the sound shielding peak frequency that is located at the center of the sound shielding frequency range of the noise to be selectively shielded, and the transmission loss of the sound shielding peak can be determined.

It is preferable that the sound insulation structure 10a has a plurality of through-hole 22 of the same size within one sound insulation cell 26 from a viewpoint of manufacturability. That is, it is preferable that the opening part 24 of each sound insulation cell 26 is constituted of the plurality of through-holes 22 of the same size.

Moreover, in the sound insulation structure 10a, it is preferable that the through-holes 22 that constitutes the opening parts 24 of all the sound insulation cells 26 are holes of the same size.

In the invention, it is preferable that the through-hole 22 is pierced by processing methods that absorb energy, for example, laser processing, or it is preferable that the through-hole 22 is pierced by machining methods by physical contact, for example, punching or needle processing.

For this reason, in a case where the plurality of through-holes 22 within one sound insulation cell 26 or one or a plurality of through-holes 22 within all the sound insulation cells 26 are made to have the same size, and in a case where holes are made by laser processing, punching, or needle processing, the holes can be continuously made without changing the settings or the processing strength of a processing apparatus.

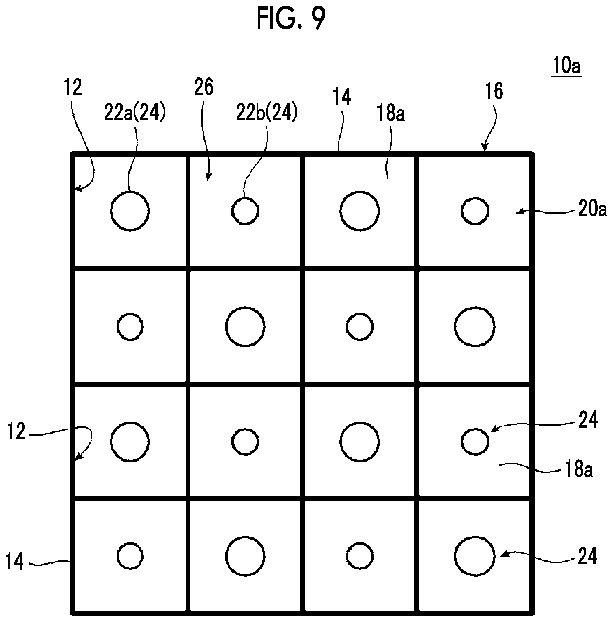

Additionally, as illustrated in Fig. 9, in the sound insulation structure 10a, the sizes of the through-holes 22 within the sound insulation cells 26 (or the films 18a) may be different from each other in the respective sound insulation cells 26 (or films 18a). In this way, in a case where through-holes 22 having different sizes are present in the respective sound insulation cells 26 (or films 18a), a corresponding sound shielding peak is shown in a sound shielding characteristic corresponding to an average area obtained by averaging the areas of the through-holes 22, that is, at a corresponding sound shielding peak frequency.

Additionally, it is preferable that 70% or more of the opening parts 24 of the respective sound insulation cells 26 of the sound insulation structure 10 of the invention is constituted of holes of the same size.

The size of the through-holes 22 that constitute the opening part 24 may be any size as long as the through-holes can be appropriately pierced by the above-described processing methods, and is not particularly limited.

However, the size of the through-hole 22 is preferably 2 $\mu$m or more, more preferably 5 $\mu$m or more, and most preferably 10 $\mu$m or more on a lower limit side thereof from a viewpoint of the processing accuracy of laser processing, such as the accuracy of a diaphragm of a laser, the processing accuracy of punching processing, needle processing or the like, manufacturability, such as the easiness of processing.

In addition, since the upper limit value of the size of the through-hole 22 needs to be less than the size of the frame 14, normally, the size of the frame 14 is on the order of mm. In a case where the size of the through-hole 22 is set to the order of $\mu$m, the upper limit value of the size of the through-hole 22 does not exceed the size of the frame 14. However, in a case where the upper limit value exceeds the size of the frame 14, the upper limit value of the size of the through-hole 22 may be set to a value equal to or less than the size of the frame 14.

In addition, in the sound insulation of the sound insulation structure 10a, it becomes important that both the through-hole 22 that allows sound to be transmitted as sound waves instead of vibrating and the film 18a through which sound passes as the film vibration are present.

Therefore, in the through-hole 22 that allows sound to be transmitted therethrough, even in a state where sound is covered with a member through which the sound can pass as sound waves traveling through the air instead of the film vibration, it is possible to obtain a sound shielding peak similarly to a case where the through-hole is opened. Such a member is generally a member having air permeability. A representative member having such air permeability includes a net of a net door. Although an Amidology 30-mesh product made by NBC Meshtec Co is mentioned as an example, the present inventors have confirmed that a spectrum obtained does not change even in a case where the penetrating through-hole 22 is blocked by this.

The net may be lattice-like or may be triangular lattice-like, and particularly, is not dependent on or not limited by its shape. The size of the entire net may be more than or may be less than the size of the frame body of the invention. Additionally, the size of the net may be such a size that the net covers the through-hole 22 of the film 18a one by one. Additionally, the net may be a net of a size such that the mesh thereof is intended for so-called insect repelling, or may be a net that prevents entry of finer sand. A material of the net may be a net made of synthetic resin, or may be a wire for preventing crimes or for shielding electric waves.

Additionally, the above-described member having air permeability is not limited to the net of the net door, and includes a non-woven fabric material, an urethane material, Synthrate (made by 3M), Thinsulate (made by Toyobo Co., Ltd.), Dot Air (made by Toray Industries, Inc.), or the like in addition to the net. In the invention, by being covered with such materials having air permeability, it is possible to prevent insects or sand from invading from entering through the hole, to prevent the inside from being seen through the penetrating through-hole 22, and the like.

Additionally, in the sound insulation of the sound insulation structure 10a, it becomes important that both the through-hole 22 that allows sound to be transmitted as sound waves instead of vibrating and the film 18a that allows sound to pass therethrough as the film vibration are present. Therefore, both the end parts of the frame hole part of the frame 14 are disposed at the housing opening part 106a so as not to be blocked together.

The sound insulation structure 10a is basically configured as described above.

Since the sound insulation structure 10a is configured as described above, there are also features that it is possible to achieve low frequency shielding that is difficult in related-art sound insulation structures, and it is also possible to design a structure that strongly shields noise in conformity with noise of various frequencies ranging from a low frequency to a frequency exceeding 1000 Hz. Additionally, since the sound insulation structure 10a adopts a sound insulating principle that is not dependent on the mass (mass law) of a structure, it is possible to realize an extremely lightweight and thin sound insulation structure as compared to the related-art sound insulation structures. Therefore, sufficient sound insulating performance is obtained without increasing the size and the weight of the ear muffs.

Additionally, since the sound insulation structure 10a has the through-hole 22 in the film 18a, it is possible to realize a structure that has air permeability, that is, shields sound while allowing wind or heat to pass therethrough.

Additionally, as materials for forming the frame 14 (frame body 16) of the sound insulation structure 10a and the film 18a (film body 20a), non-magnetic materials, such as resin, can be used. Therefore, the entire earmuff 100 can be formed of the non-magnetic materials by forming the headband 104, the housing 106, and the ear pad 108, and the like of the non-magnetic materials, such as resin, and is also suitably available in magnetic resonance imaging (MRI) examination or the like.

The sound insulation structure 10a is manufactured as follows.

First, the frame body 16 having the plurality of (for example, 225) frames 14, and the sheet-like film body 20a that covers the frame hole parts 12 of all the frame 14 of the frame body 16 are prepared.

Next, the sheet-like film body 20a is fixed to all the frames 14 of the frame body 16 with an adhesive, the films 18a that cover the frame hole parts 12 of all the frames 14, respectively, are formed to constitute the plurality of sound insulation cells each having a structure including a frame 14 and a film 18a.

Next, one or more through-holes 22 are pierced in the film 18a of each of the plurality of sound insulation cells by processing methods that absorb energy, such as laser processing, or machining methods based on physical contact, such as punching or needle processing, to form the opening part 24 in each sound insulation cell 26.

In this way, the sound insulation structure 10a can be manufactured.

Next, another example of the sound insulation structure will be described.

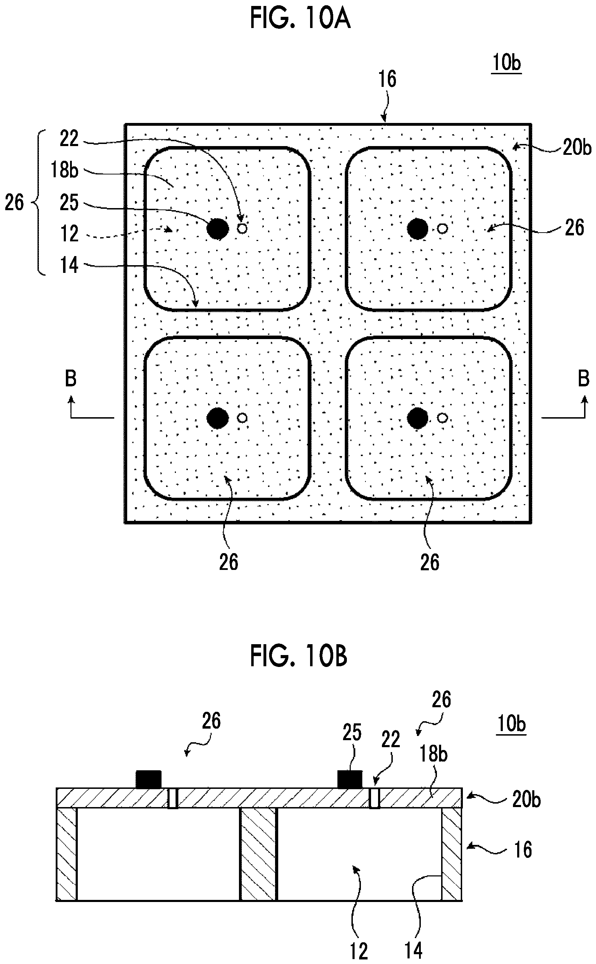

Fig. 10A is a front view schematically illustrating another example of the sound insulation structure, and Fig. 10B is a cross-sectional view taken along line B-B of Fig. 10A.

In addition, since a sound insulation structure 10b illustrated in Figs. 10A and 10B has the same configuration as the sound insulation structure 10a illustrated in Figs. 6 and 7 except having a weight 25 on a film 18b, the same parts will be denoted by the same reference signs, and the following description will mainly be performed regarding different parts.

The sound insulation structure 10b illustrated in Figs. 10A and 10B has a frame body 16 that forms a plurality of (four in the illustrated example) frames 14 which have frame hole parts 12, respectively, through which sound is transmitted and are two-dimensionally disposed; a sheet-like film body 20b that is fixed to the respective frames 14 so as to cover the frame hole parts 12 of the respective frames 14 and forms a plurality of (four in the illustrated example) films 18b; a plurality of (four in the illustrated example) opening parts 24 including one or more (one in the illustrated example) through-holes 22 pierced so as to penetrate the films 18b within the respective frames 14; and one or more (four in the illustrated example) weights 25 disposed on the films 18b within the respective frames 14.

In addition, in Fig. 10A, in order to illustrate the configuration of the sound insulation structure 10b, the structure of the frame 14 is illustrated through the film 18b, and the film 18b is illustrated with halftone dots.

In the sound insulation structure 10b, one frame 14, a film 18b fixed to the frame 14, and an opening part 24 provided in the films 18b, and a weight 25 disposed on the film 18b constitute one sound insulation cell 26. For this reason, the sound insulation structure 10b of the invention is constituted of a plurality of (four in the illustrated example) the sound insulation cells 26.

The film 18b is the same as the film 18a except disposing the weight 25.

In addition, according to the knowledge of the present inventors, in the sound insulation structure 10b using the film 18b provided with that the through-hole 22 and the weight 25, the through-hole 22 that constitutes the opening part 24 is pierced as a through-hole in the film 18b. Therefore, the peak of shielding of the sound waves at which the transmission loss has a peak (local maximum) appears at a first shielding peak frequency on a lower frequency side than the first natural vibration frequency. Moreover, since the weight 25 is disposed on the film 18b, the shielding peak of the sound waves at which the transmission loss has a peak (local maximum) appears at a second shielding peak frequency on a higher frequency side than the first natural vibration frequency.

Hence, since the shielding (transmission loss) has a peak (local maximum) at the first shielding peak frequency and the second shielding peak frequency, the sound insulation structure 10b can selectively insulate sound of a certain frequency range centered on the first shielding peak frequency and sound of a certain frequency range centered on the second shielding peak frequency.

For this reason, in the structure including the frame 14 and the film 18b, in order to set the first shielding peak frequency depending on the opening part 24 including one or more through-holes 22 to an arbitrary frequency within an audible range and set the second shielding peak frequency depending on the weight 25 to an arbitrary frequency within the audible range, it is important to obtain the natural frequency mode within the audible range, and this is particularly important for practical use. For that reason, similar to the film 18a of the above-described sound insulation structure 10a, the thickness of the film 18b, the Young's modulus and the density of a material of the film 18b, and the size of the frame 14, and the like may be appropriately set.

Additionally, the arrangement position of the film 18b in the frame 14, and a method of fixing the film 18b to the frame 14 are the same as those of the sound insulation structure 10a.

The film 18b has the opening part 24 including one or more through-holes 22. By having the opening part 24, similar to the sound insulation structure 10a, the sound insulation structure 10b has a peak of the transmission loss at which the shielding has a peak (local maximum) on the lower frequency side than the first natural vibration frequency of the film 18b. A frequency at which this shielding (transmission loss) has a peak (local maximum) is referred to as the first shielding peak frequency.

In addition, similar to the sound insulation structure 10a, the number of through-holes 22 that constitute the opening part 24 within each sound insulation cell 26 may be two or more. Additionally, the sizes of the through-holes 22 may be the same as each other or may be different from each other. Additionally, the areas of the opening parts 24 of the respective sound insulation cells 26 may be the same as each other or may be different from each other.

The sound insulation cell 26 has one or more weights 25 disposed on the film 18b.

Here, as mentioned above, the sound insulation structure 10b has a peak of the transmission loss at which the shielding has a peak (local maximum) on the higher frequency side than the first natural vibration frequency of the film 18b by having the weight 25 disposed on the film 18b. A frequency at which this shielding (transmission loss) has a peak (local maximum) is referred to as the second shielding peak frequency.

This second shielding peak frequency appears due to the weight 25 on the higher frequency side than the first natural vibration frequency that mainly depends on the film 18b of the sound insulation cell 26 of the sound insulation structure 10b. The second shielding peak frequency is determined in accordance with the weight of the weight 25, more specifically, the weight of the weight 25 and the stiffness of the film 18b.

Here, one or more weights 25 may be disposed on the film 18b that covers the frame hole part 12 of the sound insulation cell 26. Additionally, the arrangement position of the weight 25 may be in the middle of the sound insulation cell 26 (film 18b). However, the invention is not limited to this, and the arrangement position may not be in the middle of the sound insulation cell 26 or may be disposed at any position.

Additionally, in the example illustrated in Fig. 10B, a configuration in which the weight 25 is disposed on a front side (a surface opposite to the frame 14) of the film 18b is adopted. However, the invention is not limited to this. A configuration in which the weight 25 is disposed on a back side of the film 18b, that is, within the frame hole part 12 of the frame 14. Alternatively, weights 25 may be disposed on both surfaces of the film 18b.

Additionally, the number of weights 25 within the sound insulation cell 26 may be one with respect to one sound insulation cell 26. However, the invention is not limited to this, and two or more (that is, a plurality of) weights may be provided.

In the invention, the weight of the weight 25 within the sound insulation cell 26 is not particularly limited and may be set in accordance with a sound shielding frequency range of the noise to be selectively shielded. However, the weight is preferably 0.01 g to 10 g and more preferably 0.1 g to 1 g. By setting the weight of the weight 25 to the above ranges, the second sound shielding peak frequency that is located at the center of the sound shielding frequency range of the noise to be selectively shielded, and the transmission loss of the sound shielding peak can be determined.

Additionally, the shape of the weight 25 is not particularly limited, and can be various shapes, such as a plate-shape, a columnar shape, and a tubular shape.

Here, from a viewpoint of not hindering the vibration of the film 18b, the percentage of the area of the weight 25 with respect to the area of the film 18b in plan view is preferably 50% or less and more preferably 10% or less.

Additionally, materials of the weight 25 are not particularly limited, and can be selected in accordance with the above-described sound insulated object, its sound insulating environments, and the like.

Specifically, the materials of the weight 25 may include, for example, metallic materials, such as aluminum, titanium, magnesium, tungsten, iron, steel, chromium, chromium molybdenum, nichrome molybdenum, and alloys thereof; resin materials, such as acrylic resin, polymethylmethacrylate, polycarbonate, polyamideimide, polyarylate, polyetherimide, polyacetal, polyetheretherketone, polyphenylene sulfide, polysulfone, polyethylene telephthlate, polybutylene telephthlate, polyimide, and triacetylcellulose; magnetic materials, such as ferrite magnets and neodymium magnets; carbon fiber reinforced plastic (CFRP); carbon fiber; glass fiber reinforced plastic (GFRP), and the like.

Here, as described above, the percentage of the area of the weight 25 with respect to the area of the film 18b is preferably smaller, and desirably has a sufficient weight in a predetermined range. Hence, as the materials of the weight 25, it is preferable to use high-density materials. From this point, as the material of the weight 25, metals, such as iron and steel, are more preferable.

In the invention, methods of fixing the weight 25 to the film 18b are not particularly limited, and can be, for example, a method using an adhesive, a method using double-stick tape, and the like. The adhesive may include, for example, an epoxy adhesive (Araldite or the like), a cyanoacrylate adhesive (Aron Alpha or the like), an acrylic adhesive, or the like.

Additionally, in the sound insulation structure 10 of the invention, the weights of the weights 25 of the sound insulation cells 26 may be different from each other in the respective sound insulation cells 26. In this way, in a case where weights 25 having different weights are present in the respective sound insulation cells 26, a corresponding sound shielding peak is shown in a sound shielding characteristic corresponding to an average value obtained by averaging the weights of the weights 25, that is, at a corresponding second sound shielding peak frequency.

Additionally, it is preferable that 70% or more of the weights 25 of the respective sound insulation cells 26 of the sound insulation structure 10 of the invention is constituted of weights of the same weight.

Since the sound insulation structure 10b is configured as described above, there are also features that it is possible to achieve low frequency shielding that is difficult in related-art sound insulation structures, and it is also possible to design a structure that strongly shields noise in conformity with noise of various frequencies ranging from a low frequency to a frequency exceeding 1000 Hz. Additionally, since a configuration having two shielding peaks can be adopted, it is also possible to use the sound insulation structure for an application in which the sound from a plurality of noise sources is shielded.

Additionally, since the sound insulation structure of the invention adopts a sound insulating principle that is not dependent on the mass (mass law) of a structure, it is possible to realize an extremely lightweight and thin sound insulation structure as compared to the related-art sound insulation structures. Therefore, sufficient sound insulating performance is obtained without increasing the size and the weight of the ear muffs.

Additionally, since the sound insulation structure 10b has the through-hole 22 in the film 18b, it is possible to realize a structure that shields sound while having air permeability, that is, a structure that shields sound while allowing wind or heat to pass therethrough.

In a method of manufacturing the sound insulation structure 10b, it is possible to manufacture the sound insulation structure 10b having the sound insulation cell having the frame 14, the film 18b, the opening part 24, and the weight 25 by fixing the weight 25 to the film 18b of each of the plurality of sound insulation cells by using an adhesive or double-stick tape before or after the through-hole 22 is formed, in the same manufacturing method as the sound insulation structure 10a.

Here, in the example illustrated in Fig. 10A, the through-hole 22 and the weight 25 are independently provided on the film 18b, respectively. However, the invention is not limited to this. The through-hole 22 may be formed so as to penetrate the film 18b and the weight 25.

In other words, a configuration in which the weight 25 is cylindrical, a central axis of a hollow part of this cylinder and a central axis of a through-hole 22 are made to coincide with each other, and the weight 25 and the through-hole 22 are disposed so as to overlap each other may be adopted.

Additionally, although the number of weights 25 and the number of through-holes 22 may be the same as each other or may be different from each other.

For example, a configuration having one through-hole 22 pierced at the center of the film 18b and four weights 25 disposed around the through-hole 22 may be adopted, or a configuration having one weight 25 disposed at the center of the film 18b and four through-holes 22 pierced around this weight 25.

Moreover, still another example of the sound insulation structure will be described.

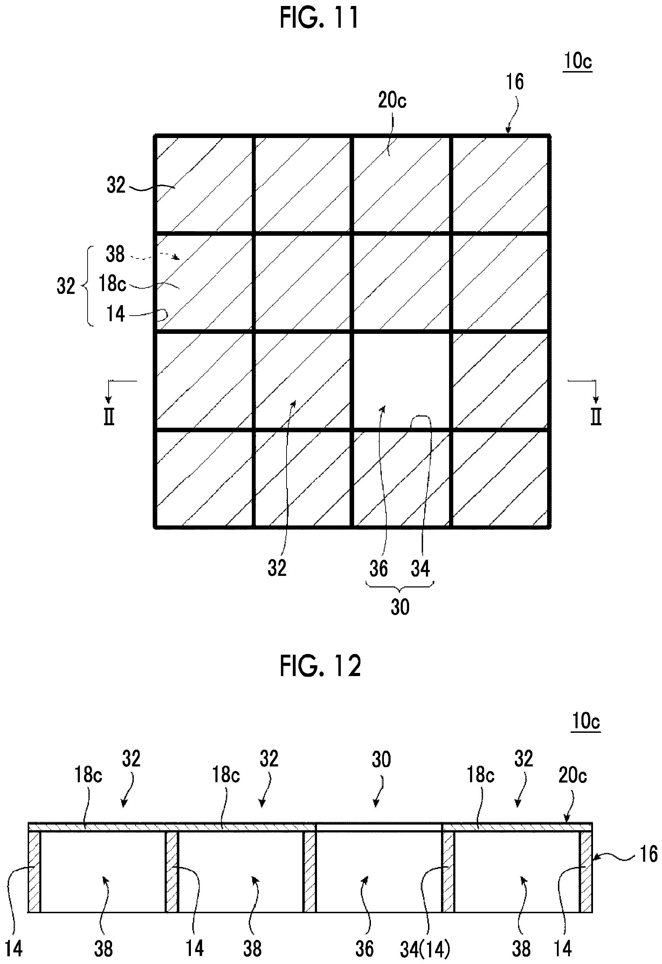

Fig. 11 is a front view schematically illustrating still another example of the sound insulation structure, and Fig. 12 is a cross-sectional view taken along line II-II of the sound insulation structure illustrated of Fig. 11.

In addition, in Fig. 11, in order to clarify the configuration, a film 18c is illustrated by hatching.

Additionally, since a sound insulation structure 10c illustrated in Figs. 11 and 12 has the same configuration as the sound insulation structure 10a illustrated in Figs. 6 and 7 except having a sound insulation cell that does not have the through-hole 22 on the film 18c and a sound insulation cell that does not have a film, the same parts will be denoted by the same reference signs, and the following description will mainly be performed regarding different parts.

As illustrated in Figs. 11 and 12, the sound insulation structure 10c has a plurality of (sixteen arranged in 4x4 in the illustrated example) sound insulation cells that are two-dimensionally arranged. One of the sound insulation cells is a second sound insulation cell 30 including a second frame 34 having a second frame hole part 36, and the remaining fifteen sound insulation cells are first sound insulation cells 32 each including a frame (first frame) 14 having a first frame hole part 38, and a film 18c that is disposed so as to cover one opening surface of the frame 14 and is fixed to a frame 14.

Additionally, the second sound insulation cell 30 and the first sound insulation cells 32 that are two-dimensionally arranged are arranged such that an opening surface of the second frame hole part 36, and a surface of the film 18c are directed to the same direction.

Additionally, the shape of the second sound insulation cell 30 (the opening part of the second frame hole part 36) and the first sound insulation cells 32 (the opening parts of the first frame hole parts 38) as seen from a direction perpendicular to a surface where the sound insulation cells are two-dimensionally arranged (hereinafter also referred to as "plan view") is substantially square.

Each first sound insulation cell 32 has a configuration in which the first frame hole part 38 of the frame 14 is covered with the film 18c. In the first sound insulation cell 32, by virtue of such a configuration, the film 18c vibrates in correspondence with the sound waves from the outside to absorb or reflect and insulate the energy of the sound waves. For that reason, it is preferable that the film 18c impermeable to air.

Here, the film 18c fixed to the frame 14 of the first sound insulation cell 32 has the first natural vibration frequency at which the transmission loss is minimum, for example, 0 dB, as the resonant frequency that is the frequency of the lowest-order natural frequency mode. The first natural vibration frequency is determined depending on the geometric shape of the frame 14, the stiffness of the film 18c, or the like.

Additionally, the second sound insulation cell 30 has a configuration including the second frame 34 having the second frame hole part 36. For that reason, the sound waves from the outside are transmitted through the second sound insulation cell.

Here, in a case where sound waves on the lower frequency side than the first natural vibration frequency among the sound waves transmitted through the first sound insulation cells 32 are transmitted through the first sound insulation cells 32, a phase delay of approximately 90° occurs, and in a case where sound waves of a higher frequency than the first natural vibration frequency are transmitted through the first sound insulation cells 32, a phase advance of approximately 90° occurs.

Meanwhile, the sound waves transmitted through the second sound insulation cell 30 is phase-advanced depending on the structure (the opening diameter and the path length) of the second frame hole part 36 of the second sound insulation cell 30.

Here, the path length is the shortest propagation path length of the sound waves that pass through the second sound insulation cell 30, and in Fig. 12, the thickness of the second frame 34 is the path length.

For that reason, a phase difference occurs between the sound waves transmitted through the second sound insulation cell 30, and the sound waves on the lower frequency side than the first natural vibration frequency among the sound waves transmitted through the first sound insulation cells 32, and these acoustic waves are offset from each other. Accordingly, it is possible to insulate sound on the lower frequency side than the first natural vibration frequency.

In this case, due to the second frame hole part 36 of the second sound insulation cell 30, sound of a certain frequency range centered on this frequency can be selectively insulated as a shielding peak of a predetermined frequency on a lower frequency side than the first natural vibration frequency.

Here, in the following description, the peak wavelength of shielding at which the transmission loss has a local maximum on a lower frequency side than the first natural vibration frequency determined due to the second frame hole part 36 of the second sound insulation cell 30 is referred to as a "third shielding peak frequency".

As described above, the sound insulation structure 10c has the second sound insulation cell 30 including the second frame 34 having the second frame hole part 36, and the first sound insulation cells 32 each having the frame 14 having the first frame hole part 38, and the film 18c fixed to the frame 14. Accordingly, a phase difference occurs between the sound waves transmitted through the second sound insulation cell 30, and the sound waves on the lower frequency side than the first natural vibration frequency of the first sound insulation cells 32 among the sound waves transmitted through the first sound insulation cells 32, and these acoustic waves are offset from each other. Thus, it is possible to perform sound insulation at a certain frequency range centered on the third shielding peak frequency on the lower frequency side than the first natural vibration frequency.

Additionally, since the second sound insulation cell 30 does not have a film and the second frame hole part 34 of the second frame 36 is not closed, it is possible to allow wind and heat to pass therethrough. Hence, it is possible to realize a structure that shields sound while having air permeability, that is, a structure that shields sound while allowing wind or heat to pass therethrough.

Additionally, since the sound shielding characteristic is hardly dependent on the position of the second sound insulation cell 30 that does not have a film, there is an advantage that stability is extremely high in manufacturing.

Additionally, only by providing the second sound insulation cell 30 that does not have a film, any desired frequency component can be extremely strongly shielded, and sound can be insulated on a lower frequency side than the first natural vibration frequency of the film 18c of each first sound insulation cell 32. Thus, the sound shielding characteristic in a low frequency region can be further improved.

In addition, in the second sound insulation cell 30, it is preferable that the phase advance that is caused with respect the sound waves to be transmitted is 20° or more and more preferably 55° or more.

In a case where the phase advance resulting from the second sound insulation cell 30 is 20° under the condition that the amplitudes of the sound waves transmitted through the second sound insulation cell 30 and the first sound insulation cells 32 are equal to each other, the transmission loss (sound shielding characteristic) of 5 dB or more can be obtained, and in a case where the phase advance is 55°, the transmission loss of 10 dB or more can be obtained.

Here, in the illustrated example, the second frame 34 and the frames 14 are all integrally formed and are constituted of one frame body 16. That is, this frame body is the same as the frame body 16 of the sound insulation structure 10a.

Additionally, the films 18c of the first sound insulation cells 32 are all integrally formed and are constituted of one film body 20c.

That is, the sound insulation structure 10c has the frame body 16 having sixteen frame hole parts that are two-dimensionally arranged, and the film body 20c fixed to the frame body 16 so as to cover the opening surfaces of the respective through-holes of the frame body 16, on one surface side of the frame body 16. The film body 20c is configured to have an opening part of substantially the same size as the size of the opening of a frame hole part, in a region corresponding to one frame hole part of the sixteen frame hole parts. Accordingly, a region corresponding to the respective frame hole parts of the frame body 16 constitute the one second sound insulation cell 30 and the fifteen first sound insulation cells 32.

Additionally, in the illustrated example, the sound insulation structure 10c had a configuration having a total of sixteen sound insulation cells of the one second sound insulation cell 30 and the fifteen first sound insulation cells 32. However, a configuration having one or more second sound insulation cells 30 and one or more first sound insulation cells 32 may be adopted.

For example, a configuration having 16 sound insulation cells of two second sound insulation cells 30 and fourteen first sound insulation cells 32 may be adopted. Alternatively, for example, a configuration having twenty five sound insulation cells of one second sound insulation cell 30 and twenty four first sound insulation cells 32 may be adopted.

Additionally, the percentage (opening ratio) of the total area of the second frame hole part 36 of the second sound insulation cell 30 to the area of the sound insulation structure 10c in plan view (that is, the total area of the front surfaces of the second sound insulation cell 30 and the first sound insulation cells 32) is preferably 0.1% to 50% and more preferably, 1% to 10%.

Additionally, although the arrangement position of the second sound insulation cell 30 within the sound insulation structure 10c is not particularly limited, it is preferable that the first sound insulation cells 32 are mutually uniformly disposed.

Additionally, in Fig. 11, the size of the second sound insulation cell 30 (that is, the size of the second frame hole part 36) in plan view, and the size of each first sound insulation cells 32 (that is, the size of each first frame hole part 38) are made to have the same size. However, the invention is not limited to this, and the size of the second sound insulation cell 30 may be different from the size of the first sound insulation cell 32.

Additionally, in a case where two or more second sound insulation cells 30 are provided, the sizes of the two or more second sound insulation cells 30 may be different from each other.

Similarly, in a case where two or more first sound insulation cells 32 are provided, the sizes of the two or more first sound insulation cells 32 may be different from each other.

From a viewpoint of manufacturing efficiency, it is preferable that the sizes of all the frame hole parts, that is, the sizes of the sound insulation cells are the same.

Additionally, in a case where has two or more first sound insulation cells 32 are provided, a configuration two or more types of first sound insulation cells 32 in which the first natural vibration frequencies of the films 18c are different from each other may be adopted.

Similarly, in a case where two or more second sound insulation cells 30 are provided, a configuration having two or more types of second sound insulation cells 30 in which the opening diameters and the path lengths of the second frame hole parts 36 are different from each other may be adopted.

Here, as mentioned above, the third shielding peak frequency is determined in accordance with the structure, specifically, the opening diameter and the path length of the second frame hole part 36 formed in each second frame 34.

Hence, the thickness of the second frame 34 may be set to a thickness so as to provide an arbitrary third shielding peak frequency in accordance with the size of the frame or the frequency of the sound to be insulated.

Additionally, the thickness of the frame 14 and the thickness of the second frame 34 may be different from each other. By adjusting the thickness of the second frame 34 to adjusting the path length of the second frame hole part 36 of the second frame 34, the amount of the phase advance resulting from the second sound insulation cell 30 can be adjusted, and the third shielding peak frequency can be determined to a desired frequency.

Additionally, the shape of the second frame hole part 36 of the second frame 34 is not limited to a straight pipe shape, and a configuration in which the third shielding peak frequency is determined to a desired frequency by appropriately setting the shape of the second frame hole part 36.

For example, by forming the second frame hole part 36 of the second frame 34 constituting the second sound insulation cell 30 in a shape that is repeatedly bent in an S shape in the direction of a central axis, it is possible to increase the path length of the sound waves to be transmitted to adjust the amount of the phase advance resulting from the second sound insulation cell 30 to determine the third shielding peak frequency to a desired frequency.

Additionally, a configuration in which the path length of the sound waves is increased may be adopted as a configuration in which the second frame 34 constituting the second sound insulation cell 30 is provided with one or more adjusting plates extending in a direction orthogonal to the direction of the central axis of the second frame hole part 36.

Additionally, the sound insulation structure 10c may have a configuration having two or more types of second sound insulation cells 30 in which the opening diameters in such second frame hole parts 36 or the path lengths of the sound waves are different from each other.

The film 18c is the same as the film 18a of the sound insulation structure 10a except not having a through-hole.

Here, as mentioned above, in the sound insulation structure 10c, a phase difference occurs between the sound waves transmitted through the second sound insulation cells 30 having no film, and the sound waves on the lower frequency side than the first natural vibration frequency among the sound waves transmitted through each first sound insulation cell 32, and these acoustic waves are offset from each other. Accordingly, it is possible to insulate sound on the lower frequency side than the first natural vibration frequency.

For this reason, in the sound insulation structure 10c, in order to set the shielding peak frequency to an arbitrary frequency within an audible range, it is important to obtain the natural frequency mode of the film 18c of the first sound insulation cell 32 as close to the high-frequency side possible, and this is particularly important for practical use. For that reason, similar to the film 18a of the above-described sound insulation structure 10a, the thickness of the film 18c, the Young's modulus and the density of a material of the film 18c, and the size of the frame 14, and the like may be appropriately set.

Additionally, the arrangement position of the film 18c in the frame 14, and a method of fixing the film 18c to the frame 14 are the same as those of the sound insulation structure 10a.

Here, it is preferable that the size of the sound insulation structure 10c is a size equal to or less than the wavelength of the sound in the third shielding peak frequency. According to the present inventor's study, it can be seen that a transmission phase difference begins to be disturbed in a case where the size of sound insulation structure is set to a size equal to or more than the wavelength of the sound in the third shielding peak frequency.

As mentioned above, the sound insulation structure 10c exhibits a high sound shielding characteristic by controlling a phase difference between the sound waves transmitted through the second sound insulation cell 30 and the sound waves transmitted through the first sound insulation cell 32. For that reason, in a case where the phases are disturbed, the third shielding peak frequency cannot be controlled, and it becomes difficult to exhibit a desired sound shielding characteristic.

Hence, it is preferable that the size of the sound insulation structure 10c is a size equal to or less than the wavelength of the sound in the third shielding peak frequency.

Additionally, according to the present inventor's study, it can be seen that it is possible to more suitably shield the sound by setting the size of the sound insulation structure 10c to a size equal to or less than the wavelength of sound in the frequency to be shielded.

Additionally, in the sound insulation structure 10c, a sound absorbing member, a deodorizing material, or the like may be disposed inside the second frame hole part 36 of the second sound insulation cell 30.

By disposing the sound absorbing member, the sound shielding characteristic can be further improved due to a sound absorption effect resulting from the sound absorbing member.

The sound absorbing member is not particularly limited, and various well-known sound absorbing members, such as a urethane plate and a non-woven fabric, are available.

The deodorizing material is not particularly limited, and various well-known deodorizing materials, such as a deodorizing sheet (for example, a semi-deodorizing sheet made by Asahi Chemical Industry Co., Ltd) in which activated carbon is blended, and a deodorizing sheet (for example, DI-NOC Film made by 3M) using a catalyst, are available.

Additionally, in the sound insulation structure 10c, the second frame hole part 36 of the second sound insulation cell 30 may be covered with a member that allows sound to pass therethrough as sound waves.

In the sound shielding in the sound insulation structure 10c, it becomes important that both the frame hole part that allows sound to be transmitted therethrough as sound waves instead of vibrating and the film that allows sound to pass therethrough as film vibration are present. Therefore, in the frame hole part that allows sound to be transmitted therethrough, even in a state where sound is covered with a member that allows the sound to pass therethrough as sound waves, it is possible to obtain a sound shielding peak similarly to a case where the through-hole is opened.

Such a member is generally a member having air permeability. Members having air permeability mentioned in the sound insulation structure 10a can be used as examples of such a member having air permeability.

Next, an example of a method of manufacturing the sound insulation structure 10c will be described.

First, the frame body 16 having the plurality of (for example, 225) through-holes, and the sheet-like film body 20c that covers all the through-holes of the frame body 16 are prepared.

Next, the sheet-like film body 20c is fixed to all the frames (frame parts) of the frame body 16 with an adhesive, the films 18c that cover all the through-holes, respectively, are formed to constitute the plurality of first sound insulation cells 32 each having a structure including a frame 14 and a film 18c.

Next, the second sound insulation cell 30 is formed by removing the film 18c to expose the frame hole part by a processing method that absorbs energy, such as laser processing, or a machining method resulting from physical contact, such as a cutter, in a sound insulation cell at a predetermined position among the plurality of first sound insulation cells 32.

In this way, the sound insulation structure 10c can be manufactured.

Moreover, a still further example of the sound insulation structure will be described.

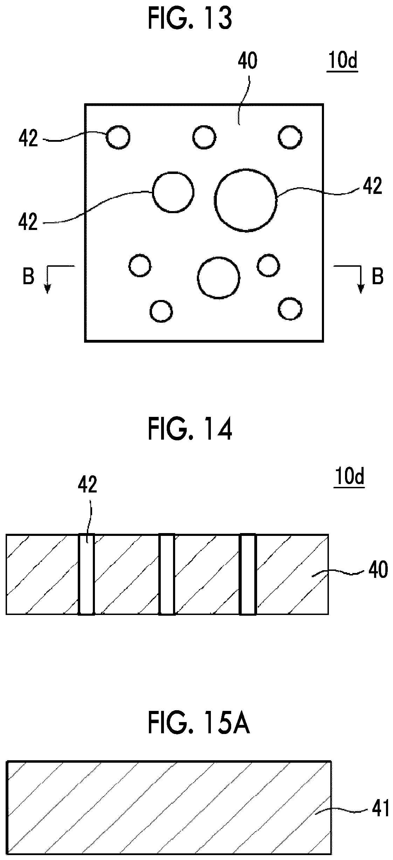

Fig. 13 is a front view schematically illustrating a still further example of the sound insulation structure, and Fig. 14 is a cross-sectional view taken along line B-B of Fig. 13.

A sound insulation structure 10d of Figs. 13 and 14 is a sound insulation structure which includes a plate-shaped member 40 having a plurality of through-holes 42 penetrating in a thickness direction thereof, in which the average opening diameter of the through-holes 42 is 0.1 $\mu$m or more and less than 100 $\mu$m, and in which an average opening ratio rho of the through-holes is within a range more than 0 and less than 1 and is within a range having rho_center - (0.052 x (phi/30)$^{-2}$) as a lower limit and having rho_center+ (0.795 x (phi/30)$^{-2}$) as an upper limit, with rho_center = (2 + 0.25 x t) x phi$^{-1.6}$ as a center in a case where the average opening diameter of the through-holes is phi ($\mu$m) and the thickness of the plate-shaped member is t ($\mu$m).

The average opening diameter of the plurality of through-holes 42 formed in the plate-shaped member 40 is 0.1 $\mu$m or more and less than 100 $\mu$m.

Additionally, the average opening ratio rho of the through-holes is within a range having rho_center - (0.052 x (phi/30)$^{-2}$) as a lower limit and having rho_center+ (0.795 x (phi/30)$^{-2}$) as an upper limit, with rho_center = (2 + 0.25 x t) x phi$^{-1.6}$ as a center in a case where the average opening diameter of the through-holes is phi ($\mu$m) and the thickness of the plate-shaped member is t ($\mu$m). In addition, the average opening ratio rho of the through-holes is within a range more than 0 and smaller than 1.

The present inventors have found out that the sound absorption effect is obtained by providing the sound insulation structure which includes the plate-shaped member having the plurality of through-holes penetrating in the thickness direction, in which the average opening diameter of the through-holes is 0.1 $\mu$m or more and less than 100 $\mu$m, and in which the average opening ratio is within the above range.

The present inventors have estimated that a mechanism of sound absorption of the sound insulation structure 10d is a change of the energy of sound to thermal energy by the friction with inner wall surfaces of the through-hole 42 and the air in a case where the sound passes through the fine through-holes 42. Since this mechanism is caused by the size of the through-holes 42 being fine, this mechanism is different from a mechanism resulting from resonance. Paths along which sound directly passes as sound in the air by the through-holes 42 have far smaller impedance compared to a path along which sound is first converted into the film vibration and is then radiated as sound again. Hence, sound is likely to pass along the paths of the through-holes 42 finer than the film vibration. In a case where sound passes through the portions of the through-holes 42, the sound collectively passes from a wide area of the entire plate-shaped member 40 to a narrow area of the through-holes 42. As the sound is collected in the through-holes 42, local speed is extremely large. Since the friction is correlated with speed, the friction becomes large within the fine through-holes 42, and the friction is converted into heat.

Since the ratio of circumferential length to opening area becomes large in a case where the average opening diameter of the through-holes 42 is small, it is believed that the friction caused at edge parts or inner wall surfaces of the through-holes 42 can be increased. By increasing the friction in a case where the sound passes through the through-holes 42, the energy of the sound can be converted into thermal energy and the sound can be absorbed.

Additionally, according to the present inventor's study, it was found that, in a case where an optimal percentage for the average opening ratio of the through-holes 42 is present and the average opening diameter is relatively as large as about 50 $\mu$m or more, the absorbance is higher as the average opening ratio is smaller. In a case where the average opening ratio is large, sound passes through each of a number of the through-holes 42, whereas in a case where the average opening ratio is small, the number of through-holes 42 decreases. Therefore, the sound that passes through one through-hole 42 increases. As a result, it is believed that the local speed of air in a case where the air passes through the through-holes 42 can be further increased and the friction caused at the edge parts or inner wall surfaces of the through-holes 42 can be further increased.

In this way, since the sound insulation structure 10d functions as a single plate-shaped member 40 having the fine through-holes 42, the size thereof can be made small.

Additionally, as described above, the sound insulation structure 10d absorbs sound with the friction in a case where the sound passes through the through-holes 42. Thus, the sound can be absorbed irrespective of the frequency range of the sound, and the sound can be absorbed in a broadband.

Additionally, since the through-holes 42 are provided, air permeability can be secured.

Here, from a viewpoint of sound absorbing performance or the like, the average opening diameter of the through-holes 42 is less than 100 $\mu$m, preferably 80 $\mu$m or less, more preferably 70 $\mu$m or less, and most preferably 50 $\mu$m or less. This is because, as the average opening diameter of the through-holes 42 becomes smaller, the ratio of the length of outer peripheral parts contributing to the friction in the through-holes 42 to the opening area of the through-holes 42 becomes larger, and the friction is likely to occur.

Additionally, as mentioned above, the average opening ratio rho of the through-holes is within a range that is more than 0 and less than 1 in a case where the average opening diameter of the through-holes is phi ($\mu$m) and the thickness of the plate-shaped member is t ($\mu$m), and the average opening ratio rho of the through-holes falls within a range having rho_center - (0.052 x (phi/30)$^{-2}$) as a lower limit and having rho_center+ (0.795 x (phi/30)$^{-2}$) as an upper limit, with rho_center = (2 + 0.25 x t) x phi$^{-1.6}$ as a center.

Additionally, the average opening ratio rho is preferably within a range of rho_center - 0.050 x (phi/30)$^{-2}$ or more and rho_center + 0.505 x (phi/30)$^{-2}$ , more preferably within a range of rho_center - 0.048 x (phi/30)$^{-2}$ or more and rho_center + 0.345 x (phi/30)$^{-2}$, still more preferably within a range of rho_center - 0.085 x (phi/20)$^{-2}$ or more and rho_center + 0.35 x (phi/20)$^{-2}$, particularly preferably within a range of (rho_center - 0.24 x (phi/10)$^{-2}$) or more and (rho_center + 0.57 x (phi/10)$^{-2}$), and most preferably within a range of (rho_center - 0.185 x (phi/10)$^{-2}$) or more and (rho_center + 0.34 x (phi/10)$^{-2}$). This point will be described in detail by the simulation to be described below.

In addition, the average opening diameter of the through-holes 42 is obtained by imaging the surface of the plate-shaped member 40 at a magnification of 200 times using a high-resolution scanning electron microscope (SEM) from one surface of the plate-shaped member, extracting twenty through-holes 42 of which the circumference are annularly connected in the obtained SEM photograph, reading the opening diameters thereof, and calculating an average value of the opening diameter as an average opening diameter. In a case where the number of through-holes is less than 20 within one SEM photograph, the SEM photograph is taken at another surrounding position, and counting is performed until a total number reaches 20.

In addition, the opening diameters were evaluated by measuring the areas of the portions of the through-holes 42, respectively, and using a diameter (a circle equivalent diameter) at the time of being substituted with a circle having the same area. That is, since shape of the opening part of each through-hole 42 is not limited to the substantially circular shape, in a case where the shape of then opening part is a non-circular shape, evaluation was performed using the diameter of a circle having the same area. Hence, for example, even in the case of a through-hole 42 of such a shape that two or more through-holes are integrated with each other, this through-hole is regarded as one through-hole 42, and the circle equivalent diameter of the through-hole 42 is used as the opening diameter.

From these tasks, all of the circle equivalent diameter, the opening ratio, and the like can be calculated by Analyze Particles, for example, using "Image J".

Additionally, the average opening ratio is obtained by imaging the surface of the plate-shaped member 40 at a magnification of 200 times using the high-resolution scanning electron microscope (SEM) from one surface of the plate-shaped member, binarizing visual fields (30 mm x 30 mm) (five points) of the obtained SEM photograph with image analysis software or the like to observe the portions of the through-holes 42 and a non-through-hole portion, calculating a ratio (opening area/geometric area) from the total of the opening area of the through-holes 42 and the area (geometric area) of the visual fields, and calculating an average value in the respective visual fields (five points as an average opening ratio).

Here, in the sound insulation structure 10d, the plurality of through-holes 42 may be regularly arranged or may be randomly arranged. From viewpoints of the productivity of the fine through-holes 42, the robustness of the sound absorption characteristic, suppressing the diffraction of sound, and the like, it is preferable that the through-holes are randomly arranged. Regarding the diffraction of sound, in a case where the through-holes 42 is periodically arranged, there is concern that the diffraction phenomenon occurs in accordance with the cycle of the through-holes 42, the sound is bent due to the diffraction and a direction in which the noise propagates is divided into a plurality of directions. The term "randomly" means a state where the through-holes are arranged without periodicity in which the through-holes are perfectly arranged, and an arrangement in which an absorption effect resulting from each through-hole 42 appears, while a diffraction phenomenon resulting from a minimum distance between the through-holes does not occur.

Additionally, although the through-holes 42 of the periodic arrangement can be formed by etching processing during roller-like continuous processing, it is easier to form a random pattern at a time, such as surface treatment, rather than a process of forming the periodic arrangement for mass production. Therefore, it is preferable that the through-holes are randomly arranged also from a viewpoint of productivity.

Additionally, the plurality of through-holes 42 may be constituted of through-holes of one type of opening diameter or may be constituted of the through-holes 42 of two or more types of opening diameters.

From viewpoints of productivity, durability, and the like, it is preferable to have the through-holes 42 of two or more types of opening diameters. Regarding the productivity, the productivity is further improved by allowing variations in hole diameter from a viewpoint of performing the etching processing in large quantities, similar to the above random arrangement. Additionally, from the viewpoint of the durability, dirt or dust have different sizes depending on environments. Therefore, in a case where the through-holes 42 of one types of opening diameter are formed, all the holes are influenced in a case where the size of main dust substantially coincides with that of the through-holes 42. By providing the through-holes 42 of a plurality of types of opening diameters, devices applicable to various environments are obtained.

Additionally, from a viewpoint of further increasing the friction in a case where sound passes through the inside of each through-hole 42, it is preferable that the inner wall surfaces of the through-holes 42 are roughened. Specifically, a surface roughness Ra of the inner wall surfaces of the through-holes 42 is preferably 0.1 $\mu$m or more, more preferably 0.1 $\mu$m to 10.0 $\mu$m, and more preferably 0.2 $\mu$m or more and 1.0 $\mu$m or less.

Here, the surface roughness Ra can be measured by measuring the inside of each through-hole 42 by an atomic force microscope (AFM). Since the roughness is on the order of several microns, it is easier to use AFM in terms of scale measurement than as other measurement methods.

Additionally, the average particle diameter of protrusions can be calculated from an SEM image within each through-hole 42 by regarding each of the protrusions of irregularities within the through-hole 42 as a particle.

Specifically, an SEM image (visual field (about 1 mm x 1 mm)) captured at a magnification of 2000 times is taken into Image J, and binarized into black and white such that the protrusions become white, the areas of each the protrusions is found by Analyze Particles. A circle equivalent diameter in which a circle having the same area as the area of each of the protrusions is supposed is found regarding each protrusion, and an average value of the circle equivalent diameters is calculated as an average particle diameter.

The average particle diameter of the protrusions is preferably 0.1 $\mu$m or more and 10.0 $\mu$m or less, and more preferably 0.2 $\mu$m or more and 5.0 $\mu$m or less.

Additionally, although the thickness of the plate-shaped member 40 is not limited, the friction energy received in a case where sound passes through the through-hole 42 increases as the thickness is larger. Therefore, it is considered that the sound absorbing performance is further improved. Additionally, in a case where the thickness is extremely small, handling is difficult and breaking is likely to occur, it is desirable that the thickness is larger to such a degree that holding can be made. Meanwhile, in terms of compactness and air permeability, it is preferable the thickness is smaller. Additionally, in a case where the etching or the like is used for a method of forming the through-holes 42, more time is taken for manufacturing as the thickness is larger. Therefore, from a viewpoint of productivity, it is desirable that the thickness is smaller.

From a viewpoint of sound absorbing performance, compactness, and air permeability, the thickness of the plate-shaped member 40 is preferably 5 $\mu$m to 500 $\mu$m, more preferably 7 $\mu$m to 300 $\mu$m, and particularly preferably 10 $\mu$m to 100 $\mu$m.

Materials of the plate-shaped member are not limited, various metals, such as aluminum, titanium, nickel, permalloys, 42 alloys, kovar, nichrome, copper, beryllium, phosphor bronze, brass, german silver, tin, zinc, iron, a tantalum, niobium, molybdenum, zirconium, gold, silver, platinum, palladium, steel, tungsten, lead, and iridium; resin materials, such as polyethylene terephthalate (PET), triacetylcellulose (TAC), polychlorinated vinylene, polyethylene, polychlorinated vinyl, polymethylpentene, cycloolefin polymers (COP), polycarbonate, zeonor, polyethylenenaphthalate (PEN), polypropylene, and polyimide; and like are available. Moreover, glass materials, such as thin film glass, and fiber-reinforced plastic materials, such as carbon fiber reinforced plastic (CFRP) or glass fiber reinforced plastic (GFRP) can also be used.

From viewpoints that the Young's modulus is high, the film vibration is unlikely to occur even in a case where the thickness is small, the sound absorption effect resulting from the friction in minute through-holes 42 is easily obtained, it is preferable to use the metallic materials. Among them, from viewpoints of being lightweight, being easy to form the minute through-holes 42 by etching or the like, availability, cost, and the like, it is preferable to use aluminum.

Additionally, in a case where the metallic materials are used, metal plating may be performed to a surface from a viewpoint of suppression of rusting, or the like.

Moreover, the average opening diameter of the through-holes 42 may be adjusted to a smaller range by performing metal plating on at least inner surfaces of the through-holes 42.

Meanwhile, the resin materials and the glass materials can also be used as the plate-shaped member 40. For example, a PET film among the resin materials has relatively high Young's modulus, is easily available, and also has high transparency, the sound insulation structure 10d that is suitable for forming the through-holes 42 can be obtained.

<Aluminum base material>

An aluminum base material used as the plate-shaped member is not particularly limited. For example, well-known aluminum base materials, such as alloy numbers 1085, 1N30, and 3003 described JIS Standard H4000 can be used. In addition, the aluminum base material is an alloy plate that has aluminum as a main component and includes small amounts of different elements.

Although the thickness of the aluminum base material is not particularly limited, the thickness is preferably 5 $\mu$m to 1000 $\mu$m, more preferably 5 $\mu$m to 200 $\mu$m, and particularly preferably 10 $\mu$m to 100 $\mu$m.

Next, a method of manufacturing the sound insulation structure 10d will be described taking a case where the aluminum base material is used as an example.

The method of manufacturing the sound insulation structure using the aluminum base material has a film forming step of forming a film having aluminum hydroxide as a main component on a surface of the aluminum base material; a through-hole forming step of performing through-hole forming processing to form through-holes after the film forming step; and a film removing step of removing the aluminum hydroxide film after the through-hole forming step. By having the film forming step, the through-hole forming step, and the film removing step, through-holes of which the average opening diameter is 0.1 $\mu$m or more and less than 100 $\mu$m can be suitably formed.

Next, the respective steps will be described in detail after the respective steps of the method of manufacturing the sound insulation structure 10d are described with reference to Figs. 15A to 15E.

Figs. 15A to 15E are schematic cross-sectional views illustrating an example of a preferred embodiment of the method of manufacturing the sound insulation structure 10d using the aluminum base material.

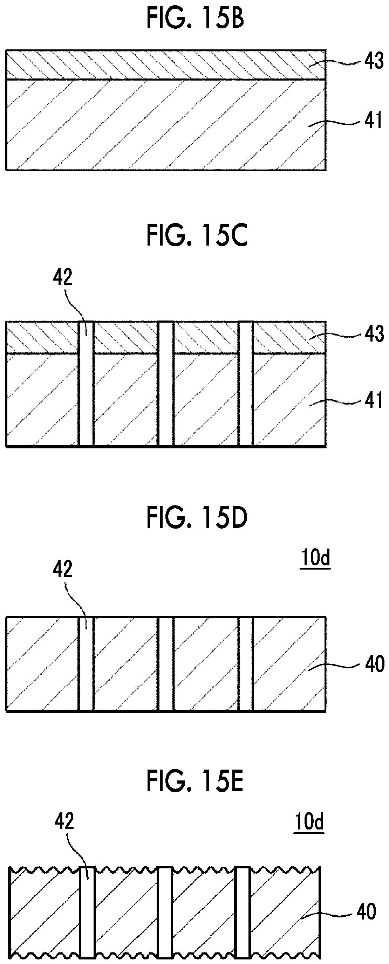

As illustrated in Figs. 15A to 15E, the method of manufacturing the sound insulation structure 10d is a manufacturing method having a film forming step of performing film forming processing on one principal surface of an aluminum base material 41 to form an aluminum hydroxide film 43 (Figs. 15A and 15B); a through-hole forming step of performing electrolysis dissolution processing to forms through-holes 42 after the film forming step, and forming through-holes in the aluminum base material 41 and the aluminum hydroxide film 43 (Figs. 15B and 15C); a film removing step of removing the aluminum hydroxide film 43 and making a sound insulation structure 10d including a plate-shaped member 40 having the through-holes 42, after the through-hole forming step (Figs. 15C and 15D).

Additionally, it is preferable that the method of manufacturing the sound insulation structure 10d has a roughening processing step of performing electrochemical roughening processing on the plate-shaped member 40 having the through-holes 42 and roughening the surface of the plate-shaped member 40, after the film removing step (Figs. 15D and 15E).

Since small holes are likely to be made in the aluminum hydroxide film, through-holes of which the average opening diameter is 0.1 $\mu$m or more and less than 100 $\mu$micrometer can be formed by performing the electrolysis dissolution processing in the through-hole forming step to forming the through-holes, after the film forming step of forming the aluminum hydroxide film.

[Film forming step]

The film forming step that the method of manufacturing the sound insulation structure 10d has is a step of performing the film forming processing on the surface of the aluminum base material and forming the aluminum hydroxide film.

<Film forming processing>

The above film forming processing is not particularly limited. For example, the same processing as aluminum hydroxide film forming processing that is well known in the related art.

As the film forming processing, for example, the conditions and the apparatus that are described in Paragraphs < 0013> to <0026> of JP2011-201123 can be appropriately adopted.

In the invention, since the conditions of the film forming processing change variously depending on electrolytic solutions to be used, the conditions cannot be determined as a rule. However, generally, it is appropriate that the electrolytic solution concentration is 1 to 80 % by mass, the liquid temperature is 5 to 70°C, the current density is 0.5 to 60 A/dm$^2$, the voltage is 1 to 100 V, the electrolysis time is 1 second to 20 minutes, and these are adjusted so as to obtain a desired film amount.

In the invention, it is preferable to perform electrochemical processing, using the nitric acid, hydrochloric acid, sulfuric acid, phosphoric acid, oxalic acid, or mixed acids of two or more of these acids as the electrolytic solution. In a case where the electrochemical processing is performed in an electrolytic solution including the nitric acid and hydrochloric acid, a direct current may be applied between the aluminum base material and a counter electrode, or an alternating current may be applied therebetween. In a case where the direct current is applied to the aluminum base material, the current density is preferably 1 to 60 A/dm$^2$ and more preferably 5 to 50 A/dm$^2$. In a case where the electrochemical processing is continuously performed, it is preferable to perform a liquid power supply method of supplying electric power to the aluminum base material via the electrolytic solution.

In the invention, the amount of the aluminum hydroxide film formed by the film forming processing is preferably 0.05 to 50 g/m$^2$ and more preferably 0.1 to 10 g/m$^2$.

[Through-hole forming step]

The through-hole forming step is a step of performing the electrolysis dissolution processing to form through-holes after the film forming step.

<Electrolysis dissolution processing>

The above electrolysis dissolution processing is not particularly limited, and can use an acidic solution can be used for the electrolytic solution, using the direct current or the alternating current. Among them, it is preferable to perform the electrochemical processing using at least one or more acids of the nitric acid and hydrochloric acid, and it is more preferable to perform the electrochemical processing using at least one or more mixed acids of sulfuric acid, phosphoric acid, and oxalic acid in addition to these acids.

In the invention, as the acidic solution that is the electrolytic solution, the electrolytic solutions written in the respective specifications of US Patent No. 4,671,859, US Patent No. 4,661,219, US Patent No. 4,618,405, US Patent No. 4,600,482, US Patent No. 4,566,960, US Patent No. 4,566,958, US Patent No. 4,566,959, US Patent No. 4,416,972, US Patent No. 4,374,710, US Patent No. 4,336,113, US Patent No. 4,184,932, and the like can also be used in addition to the above acids.

The concentration of the acidic solution is preferably 0.1 to 2.5 % by mass and particularly preferably 0.2 to 2.0 % by mass. Additionally, the liquid temperature of the acidic solution it is preferably 20 to 80°C and more preferably 30 to 60°C.

Additionally, an aqueous solution having the above acids as main constituents can be used by adding at least one of a nitric compound having nitrate ions, such as aluminum nitrate, sodium nitrate, and ammonium nitrate, a hydrochloric compound having hydrochloric ions, such as aluminum chloride, sodium chloride, and ammonium chloride, and a sulfuric compound having sulphate ions, such as aluminum sulfate, sodium sulfate, and ammonium sulfate, to an aqueous solution of acids having a concentration of 1 to 100 g/L within a range till saturation from 1 g/L.

Additionally, metals included in aluminum alloys, such as iron, copper, manganese, nickel, titanium, magnesium, and silica, may be dissolved in the aqueous solution having the above acids as main constituents. It is preferable to use a liquid obtained by adding aluminum chloride, aluminum nitrate, aluminum sulfate, and the like such that aluminum ions have 1 to 100 g/L in an aqueous solution of which the concentration of the acids is 0.1 to 2 % by mass.

Although the direct current is mainly used for electrochemical dissolution processing, in a case where the alternating current is used, alternating current power source waves are not particularly limited and sign waves, rectangular waves, trapezoidal waves, triangular waves, and the like are used. Among them, particularly rectangular waves or trapezoidal waves are preferable, and the trapezoidal waves are particularly preferable.

(Nitric acid electrolysis)

In the invention, through-holes of which the average opening diameter is 0.1 μm or more and less than 100 μm can be easily formed by electrochemical dissolution processing using an electrolytic solution having the nitric acid as a main constituent (hereinafter also abbreviated as "the nitric acid dissolution processing"). Here, it is preferable that the nitric acid dissolution processing is electrolysis processing performed under the conditions that the direct current is used, the average current density is 5 A/dm$^2$ or more and the electric quantity is 50 C/dm$^2$ or more, for the reason it is easy to control dissolution points for forming the through-holes. In addition, it is preferable that the average current density is 100 A/dm$^2$ or less and, it is preferable that the electric quantity is 10000 C/dm$^2$ or less. Additionally, the density and the temperature of the electrolytic solution in the nitric acid electrolysis are not limited. For example, electrolysis can be performed at 30 to 60°C, using a nitric acid electrolytic solution of a high concentration, for example, a nitric acid concentration of 15 to 35 % by mass, or electrolysis can be performed at a high temperature, for example, 80°C or more, using a nitric acid electrolytic solution of a nitric acid concentration of 0.7 to 2 % by mass. Additionally, electrolysis can be performed using an electrolytic solution in which at least one of sulfuric acid, oxalic acid, or phosphoric acid having a concentration 0.1 to 50 % by mass, is mixed with the above the nitric acid electrolytic solution.

(Hydrochloric acid electrolysis)

In the invention, through-holes of which the average opening diameter is 1 $\mu$m or more and less than 100 $\mu$m can be easily formed also by electrochemical dissolution processing using an electrolytic solution having hydrochloric acid as a main constituent (hereinafter also abbreviated as "hydrochloric acid dissolution processing").

Here, it is preferable that the hydrochloric acid dissolution processing is electrolysis processing performed under the conditions that the direct current is used, the average current density is 5 A/dm$^2$ or more and the electric quantity is 50 C/dm$^2$ or more, for the reason it is easy to control dissolution points for forming the through-holes. In addition, it is preferable that the average current density is 100 A/dm$^2$ or less and, it is preferable that the electric quantity is 10000 C/dm$^2$ or less.

Additionally, the density and the temperature of the electrolytic solution in the hydrochloric acid electrolysis are not limited. For example, electrolysis can be performed at 30 to 60°C, using a hydrochloric acid electrolytic solution of a high concentration, for example, a hydrochloric acid concentration of 10 to 35 % by mass, or electrolysis can be performed at a high temperature, for example, 80°C or more, using a hydrochloric acid electrolytic solution of a hydrochloric acid concentration of 0.7 to 2 % by mass.

Additionally, electrolysis can be performed using an electrolytic solution in which at least one of sulfuric acid, oxalic acid, or phosphoric acid having a concentration 0.1 to 50 % by mass, is mixed with the above hydrochloric acid electrolytic solution.

[Film removing step]

The film removing step is a step of performing chemical dissolution processing to remove the aluminum hydroxide film. In the above film removing step, the aluminum hydroxide film can be removed, for example, by performing acid etching processing or alkali etching processing to be described below.

<Acid etching processing>

The above dissolution processing is processing of dissolving the aluminum hydroxide film, using a solution (hereinafter an "aluminum hydroxide solution") in which the aluminum hydroxide is preferentially dissolved rather than aluminum.

Here, as the aluminum hydroxide solution, for example, an aqueous solution containing at least one kind selected from a group including the nitric acid, hydrochloric acid, sulfuric acid, phosphoric acid, oxalic acid, a chromium-based compound, a zirconium-based compound, a titanium-based compound, lithium salt, cerium salt, magnesium salt, sodium silicofluoride, zinc fluoride, a manganese compound, a molybdenum compound, a magnesium compound, a barium compound, and a halogen simple substance is preferable.

Specifically, the chromium-based compound includes, for example, chromium oxide ((III)), anhydrous chromium acid (VI), and the like.

The zirconium-based compound includes, for example, zircon ammonium fluoride, zirconium fluoride, and zirconium chloride.

The titanium compound includes, for example, titanium oxide and titanium sulfide.

The lithium salt includes, for example, lithium fluoride and lithium chloride.

The cerium salt includes, for example, cerium fluoride and cerium chloride. The magnesium salt includes, for example, magnesium sulfide.

The manganese compound includes, for example, sodium permagnanate and calcium permagnanate.

The molybdenum compound includes, for example, sodium molybdate.

The magnesium compound includes, for example, magnesium fluoride and pentahydrate.

The barium compound includes, for example, barium oxide, barium acetate, barium carbonate, barium chlorate, barium chloride, barium fluoride, barium iodide, barium lactate, barium oxalate, barium perchlorate, barium selenate, barium selenite, barium stearate, barium sulfate, barium titanate, barium hydroxide, barium nitrate, hydrates thereof, and the like.

Among the above barium compounds, barium oxide, barium acetate, and barium carbonate are preferable, and barium oxide is particularly preferable.

The halogen simple substance includes, for example, chlorine, fluorine, or bromine.

Among them, it is preferable that the above aluminum hydroxide solution is an aqueous solution containing an acid, and this acid may include, the nitric acid, hydrochloric acid, sulfuric acid, phosphoric acid, oxalic acid, and the like, or may be a mixture of two or more kinds of acids.

The acid concentration is preferably 0.01 mol/L or more, more preferably 0.05 mol/L or more, and it still more preferably 0.1 mol/L or more. Although there is particularly no upper limit, generally, it is preferable that the upper light is 10 mol/L or less and more preferably 5 mol/L or less.

The dissolution processing is performed by bringing the aluminum base material, on which the aluminum hydroxide film is formed, into contact with the above-described solution. The contacting method is not particularly limited, and includes, for example, a dipping method and a spray method. Among them, the dipping method is preferable.

The dipping processing is processing of dipping the aluminum base material, on which the aluminum hydroxide film is formed, in the above-described solution. In a case where agitation is performed during the dipping processing, it is preferable because uniform processing is performed.

The time of the dipping processing is preferably 10 minutes or more, more preferably 1 hour or more, and still more preferably 3 hours or more and 5 hours or more.

<Alkali etching processing>

Alkali etching processing is processing of dissolving a surface layer by bringing the above aluminum hydroxide film into contact with an alkaline solution.

Alkali used for the alkaline solution includes, for example, caustic alkali and alkali metal salt. Specifically, the caustic alkali includes, for example, sodium hydroxide (caustic soda) and caustic potash. Additionally, the alkali metal salt includes, for example, alkali metal silicate, such as sodium metasilicate, sodium silicate, potassium metasilicate, and potassium silicate; alkali metal carbonate, such as sodium carbonate and potassium carbonate; alkali metal aluminate, such as sodium aluminate and potassium aluminate; alkali metal aldonate, such as sodium gluconate and potassium gluconate, and alkali metal hydrogen phosphate, such as sodium secondary phosphate, potassium secondary phosphate, sodium tertiary phosphate, and potassium tertiary phosphate. Among them, from a viewpoint of high etching rate and low cost, a solution containing caustic alkali, and a solution containing both caustic alkali and alkali metal aluminate are preferable. Particularly, an aqueous solution of sodium hydroxide is preferable.

The concentration of the alkaline solution is preferably 0.1 to 50 % by mass and more preferably 0.2 to 10 % by mass. In a case where aluminum ions are dissolved in the alkaline solution, the concentration of the aluminum ions is preferably 0.01 to 10 % by mass and more preferably 0.1 to 3 % by mass. It is preferable that the temperature of the alkaline solution is 10 to 90°C. It is preferable that the processing time is 1 to 120 seconds.

The method of bringing the alkaline solution into contact with the aluminum hydroxide film includes, for example, a method of passing the aluminum base material, in which the aluminum hydroxide film is formed, through the inside of a tank into which the alkaline solution is put, a method of dipping the aluminum base material, in which the aluminum hydroxide film is formed, in the tank in which the alkaline solution is put, and a method of spraying the alkaline solution onto the surface (aluminum hydroxide film) of the aluminum base material on which the aluminum hydroxide film is formed.

[Roughening processing step]

In the invention, an arbitrary roughening processing step that the method of manufacturing the sound insulation structure 10d may have is a step of performing electrochemical roughening processing (also abbreviated as "electrolysis roughening processing") on the aluminum base material from which the aluminum hydroxide film is removed, and roughening the surface or back of the aluminum base material.

In addition, in the above embodiment, a configuration in which the roughening processing is performed after the through-holes are formed is adopted. The invention is not limited to this. A configuration in which the through-holes are formed after the roughening processing may be adopted.

In the invention, the surface can be easily roughened by electrochemical roughening processing using the electrolytic solution having the nitric acid as a main constituent (hereinafter also abbreviated as "nitric acid electrolysis"). Alternatively, the surface can be easily roughened by electrochemical roughening processing using the electrolytic solution having the hydrochloric acid as a main constituent (hereinafter also abbreviated as "hydrochloric acid electrolysis").

[Metal coating step]

It is preferable that the method of manufacturing the sound insulation structure 10d has a metal coating step of coating at least a portion or all of the surface of the aluminum base material including inner walls of the through-holes 42 with metals other than aluminum after the above-described film removing step, for the reason that the average opening diameter of the through-holes 42 formed by the above-described electrolysis dissolution processing can be adjusted to a small range of about 0.1 $\mu$m to 20 $\mu$m.

Here, the expression "coating at least a portion or all of the surface of the aluminum base material including inner walls of the through-holes 42 with metals other than aluminum" means that at least the inner walls of the through-holes 42 in the entire surface of the aluminum base material including the inner walls of the through-holes 42 are coated, and the surface other than the inner walls may not be coated, and portions or all thereof may be coated.

The metal coating step performs, for example, substitution processing and plating processing to be described below on the aluminum base material having the through-holes.

<Substitution processing>

The above substitution processing is processing of performing substitution plating of zinc or a zinc alloy on at least a portion or all of the surface of the aluminum base material including the inner walls of the through-holes.

A substitution plating solution includes, for example, a mixed solution of 120 g/L of sodium hydroxide, 20 g/L of zinc oxide, 2 g/L of crystalline ferric chloride, 50 g/L of rosell salt, and 1 g/L of sodium nitrate, and the like.

Additionally, commercially available a Zn or Zn alloy plating solutions may be used. For example, SubstarZn-1, Zn-2, Zn-3, Zn-8, Zn-10, Zn-111, Zn-222, Zn-291, and the like made by OKUNO CHEMICAL INDUSTRIES CO., LTD can be used.

It is preferable that the dipping time of the aluminum base material into such substitution plating solution is 15 seconds to 40 seconds, and it is preferable that the dipping temperature is 15 seconds to 40 seconds.

<Plating processing>

In a case where the substitution plating of the zinc or zinc alloy is performed on the surface of the aluminum base material by the above-described substitution processing to form a zinc film, for example, it is preferable to perform plating processing of precipitating various metals by electrolysis plating to be described below after the zinc film is substituted with nickel by electroless plating to be described below.

(Electroless plating processing)

As a nickel plating solution used for the electroless plating processing, commercially available products can be broadly used. For example, the nickel plating solution includes an aqueous solution including 30 g/L of nickel sulfate, 20 g/L of sodium hypophosphite, and 50 g/L of ammonium citrate.

Additionally, a nickel alloy plating solution includes a nickel-P alloy plating solution in which a phosphrus compound serves as a reducing agent, and a nickel-B plating solution in which a boron compound serves as a reducing agent. It is preferable that the dipping time into such a nickel plating solution or such a nickel alloy plating solution is 15 seconds to 10 minutes, and it is preferable that the dipping temperature is 30°C to 90°C.

(Electrolysis plating processing)

In the electrolysis plating processing, for example, the plating solution in a case where Cu is electroplated includes, for example, a plating solution in which 60 to 110 g/L of sulfuric acid Cu, 160 to 200 g/L of sulfuric acid, and 0.1 to 0.15 mL/L of hydrochloric acid are added to pure water, and 5 to 5.0 mL/L of Top Luccina SF Base WR 1z, 0.5 to 2.0 mL/L of Top Luccina SF-B, and 3.0 to 10 mL/L of Top Lucina SF Leverer, which are made by Okuno Pharmaceutical Co., Ltd., are added as additives.

The dipping time to such a copper plating solution is not particularly limited because the dipping time depends on the thickness of a Cu film. However, for example, in a case where a 2 $\mu$m Cu film is attached, it is preferable to perform the dipping for about 5 minutes with a current density of 2 A/dm, it is preferable that the dipping temperature is 20°C to 30°C.

[Washing processing]

In the method of manufacturing the sound insulation structure 10d, it is preferable to perform washing after the end of the above-described respective processing steps. Pure water, well water, tap water, or the like can be used for the washing. A nip device may be used in order to prevent carry-in of a processing liquid to the next step.

In such a method of manufacturing the sound insulation structure 10d, manufacturing may be performed using a cut sheet-like aluminum base material, or may be performed by Roll to Roll (hereinafter also referred to R to R).

As is well known, the R to R is a manufacturing method of pulling out a raw material from a roll formed by winding an elongated raw material, conveying the pulled-out raw material in a longitudinal direction, performing various kinds of processing, such as surface treatment, and winding the processed raw material again in a roll shape.

In the manufacturing method of forming the through-holes in the aluminum base material as described above, the through-holes of about 20 $\mu$m can be easily and efficiently formed by the R to R.

Additionally, the method of forming the through-holes are not limited to the above-dcscribed method, and may be performed by well-known methods in accordance with materials for forming the plate-shaped member, or the like.

For example, in a case where resin films, such as the PET film, are used as the plate-shaped member, the through-holes can be formed by a processing method that absorbs energy, such as laser processing, punching, a machining method resulting from physical contact, such as needle processing, or the like.

[Simulation]

Next, the results obtained by performing simulation regarding he configuration of the sound insulation structure 10d illustrated in Fig. 13 and finding a suitable relationship between the average opening diameter and the average opening ratio of the through-holes will be described. Since a system of the sound insulation structure 10d of Fig. 13 is an interaction system between the film vibration and the sound waves in the air, and the friction resulting from the through-holes is important, friction sound absorption resulting from heat sound in coupled analysis of sound and vibration was also analyzed.

Specifically, designing was performed using a sound module of COMSOLver5.1 that is analysis software of a finite element method. By using a heat sound model within the sound module, sound waves transmitted through a fluid (also including air) and sound absorption resulting from the wall friction can be calculated. Additionally, at this time, the film vibration of a thin film was also taken in for calculation by inputting physical property values of an actual material. By setting an edge part to have a periodic structure, a model in which through-holes are made in accordance with an average opening diameter and an average opening ratio in the thin film that is infinitely in a horizontal direction was constructed. The thin film that is movable freely was modeled by supporting the edge part by roller fixing and adopting restraint in which the film is movable freely in a perpendicular direction of the film.

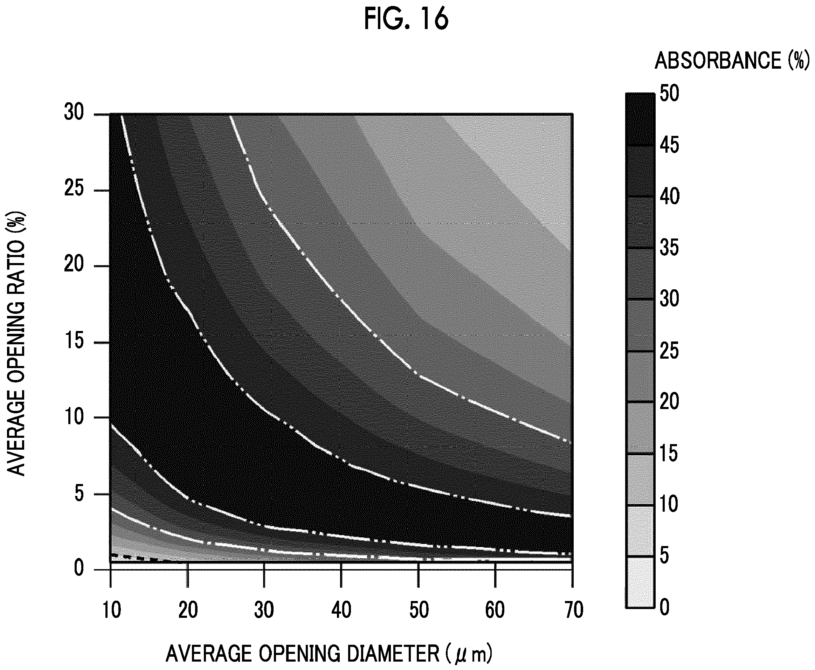

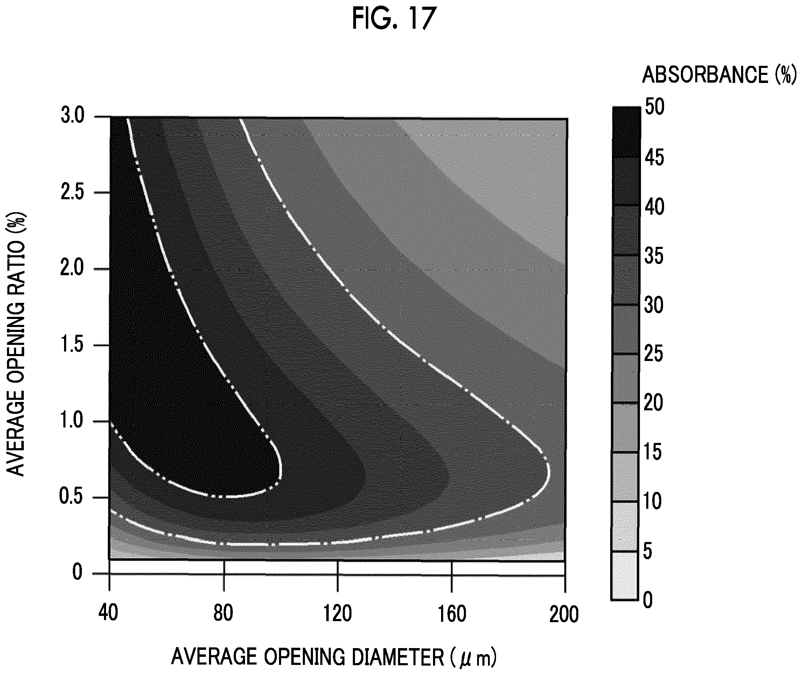

First, the thickness of the plate-shaped member was fixed to 20 $\mu$m, and only the absorbance resulting from the friction of the minute through-holes was found by neglecting the film vibration of the plate-shaped member. The average opening diameter and the average opening ratios were variously changed to find the absorbance at a frequency of 3000 Hz. The results are illustrated in Figs. 16 and 17. Since an optimal average opening ratio is in a region of 1% or less in a region where the average opening diameter is large, the range of calculation is divided into two types. Additionally, in Figs. 16 and 17, a boundary of which the absorbance is 45% is indicated by a two-dot chain line, a boundary of which the absorbance is 30% is indicated by a one-dot chain line, and a boundary of which the absorbance is 10% is indicated by a dashed line.

Regarding the condition the absorbance is maximum, the average opening diameter and the average opening ratio has an inversely proportional relationship.

Characteristically, in a case where the average opening diameter is larger than about 70 $\mu$m, a maximum value of the absorbance becomes smaller as compared to the case of a smaller average opening diameter at any average opening ratio. That is, it can be seen that it is more preferable to make the through-holes themselves as small as about 70 $\mu$m or less in order to sufficiently obtain the effect of absorption of the minute through-holes.

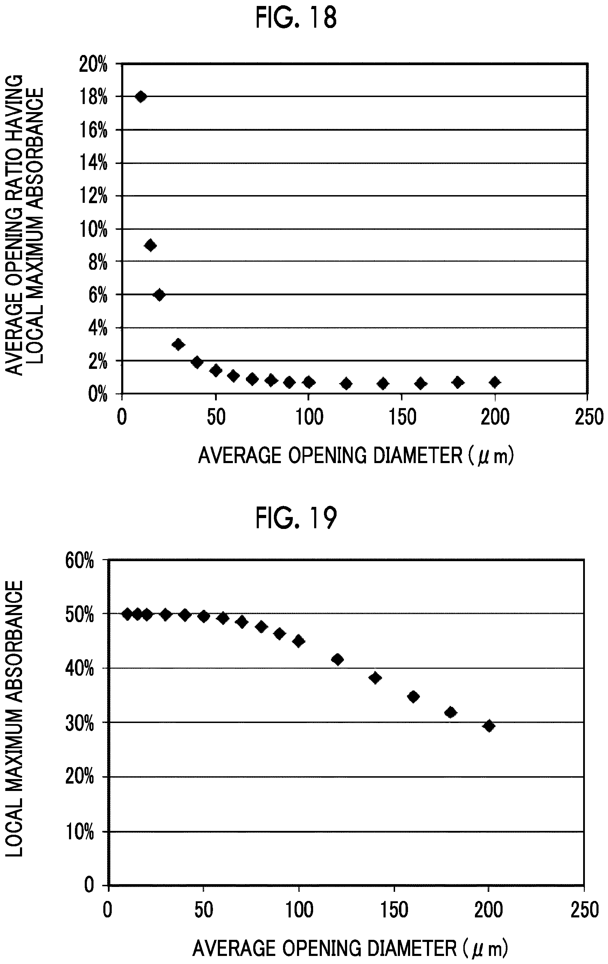

A graph showing a relationship between the average opening ratio at which the absorbance has a local maximum value and the average opening diameter is illustrated in Fig. 18, and a graph showing a relationship between the local maximum value of the absorbance and the average opening diameter is illustrated in Fig. 19. As illustrated in the drawings, in the average opening diameter of about 70 $\mu$m or more, it can be seen that the local maximum value of the absorbance becomes small substantially linearly with respect to the average opening diameter.

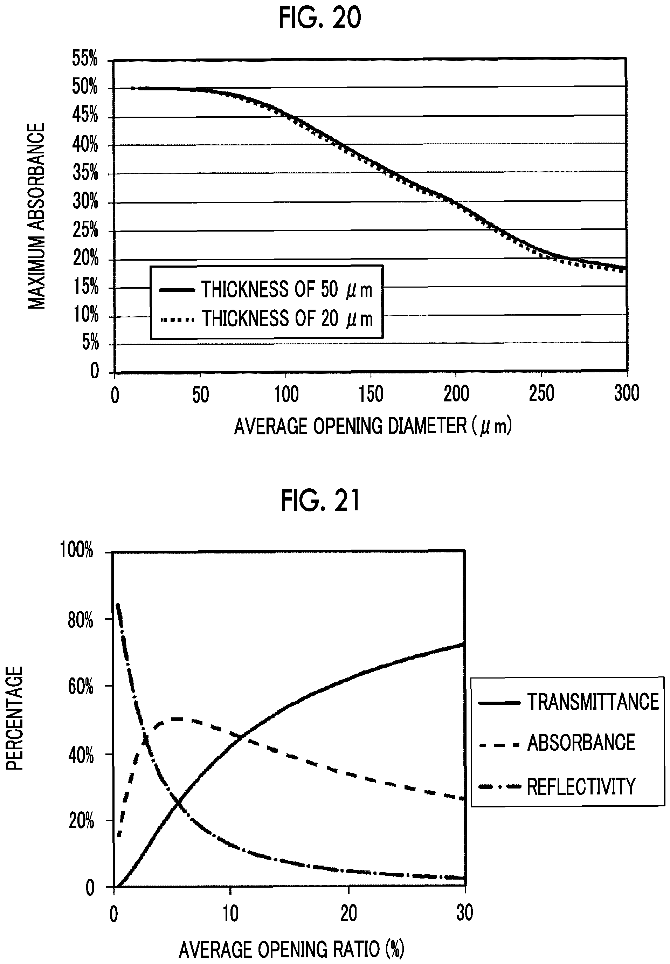

The same simulation as above was performed by changing the thickness of the plate-shaped member to 50 $\mu$m. A graph showing a relationship between the local maximum value of the absorbance and the average opening diameter is illustrated in Fig. 20.

Even in a case where the thickness of the plate-shaped member is 50 $\mu$m, similar to a case where the thickness is 20 $\mu$m, it can be seen that the local maximum value of the absorbance becomes small in an average opening diameter larger than 70 $\mu$m. It can be seen that the maximum absorbance is determined depending on the average opening diameter of the through-holes almost irrespective of the thickness of the plate-shaped member. It can be seen that the maximum absorbance is 50% in a case where the average opening diameter is as small as 50 $\mu$m or less, but the absorbance becomes small in a case where average opening diameter is larger than 50 $\mu$m. The absorbance becomes small up to 45% at an average opening diameter of 100 $\mu$m and becomes small up to 30% at an average opening diameter of 200 $\mu$m. Therefore, it became clear that it is desirable that the average opening diameter is smaller.

Additionally, from this result, it is believed that the local maximum value of the absorbance is a robust value that is not substantially dependent on the thickness of the plate-shaped member and is determined by the average opening diameter.

Simulation of transmittance, reflectivity, and absorbance was performed by fixing the thickness of the plate-shaped member to 20 $\mu$m, fixing the average opening diameter of the through-holes to 20 $\mu$m, and changing the average opening ratio. The results are illustrated in Fig. 21.

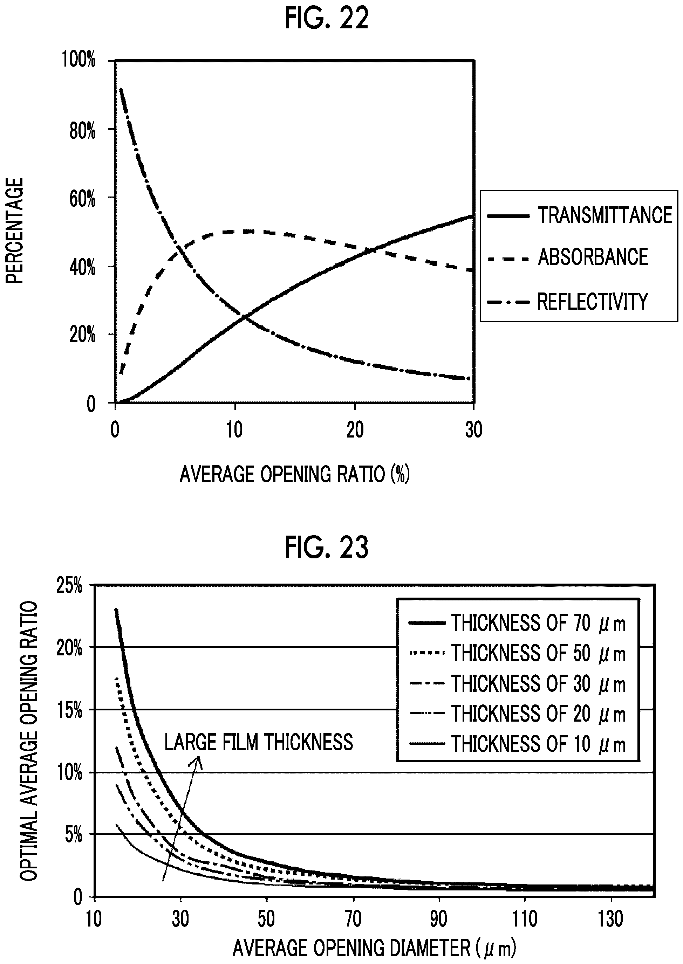

Additionally, the same simulation was performed by fixing the thickness to 50 $\mu$m, fixing the average opening diameter to 20 $\mu$m, and changing the average opening ratio. The results are illustrated in Fig. 22.

From Figs. 21 and 22, as the average opening ratio becomes larger, the reflection becomes smaller and the transmission becomes larger. It became clear that the absorption is maximized under the condition the transmission and the reflection are substantially equal to each other.

Therefore, it became clear that the transmittance and the reflectivity become equal to each other in a case where the absorption of the fine through-holes is locally maximized.

From the results hitherto, it is needless to say that the absorption becomes larger as the average opening ratio is smaller, and it became clear that the optimal average opening ratio is present depending on the thickness of the plate-shaped member and the average opening diameter of the through-holes.

Additionally, it can be seen that a region of large absorption spreads gently with the optimal average opening ratio as a center.

In order to determine the optimal average opening ratio, the thickness of the plate-shaped member was changed to 10 $\mu$m, 20 $\mu$m, 30 $\mu$m, 50 $\mu$m, and 70 $\mu$m, respectively, the average opening diameter of the through-holes was changed within a range of 20 $\mu$m to 140 $\mu$m, and the average opening ratio in which the absorbance in each condition is maximized, and the absorbance at that case was calculated and summarized. The results are illustrated in Fig. 23.

In a case where the average opening diameter of the through-holes is small, the optimal average opening ratio changes depending on the thickness of a plate-shaped member. However, in a case where the average opening diameter of the through-holes is about 100 $\mu$m or more, an extremely small average opening ratio of 0.5% to 1.0% has an optimal value.

That is, in order to obtain a large absorbance in the through-holes having an average opening diameter of 100 $\mu$m or more, it is necessary to make the average opening ratio low, and it is difficult to obtain a structure for obtaining a high opening ratio and a large absorbance.

Meanwhile, in the through-holes having an average opening diameter of less than 100 $\mu$m that the sound insulation structure of the invention has, the optimal average opening ratio has a function of the average opening diameter. Therefore, there is a condition that high opening ratio structure can be realized. Additionally, since the maximum absorbance also has a large value of 45% or more, a high opening ratio and a high sound absorption can be realized.

Moreover, from Fig. 20, the average opening diameter is desirably 80 $\mu$m or less at which the maximum absorbance is 48% or more, more desirably 70 $\mu$m or less at which the maximum absorbance is 49% or more, and still more desirably 50 $\mu$m or less at which the maximum absorbance reaches about 50% of the maximum value.

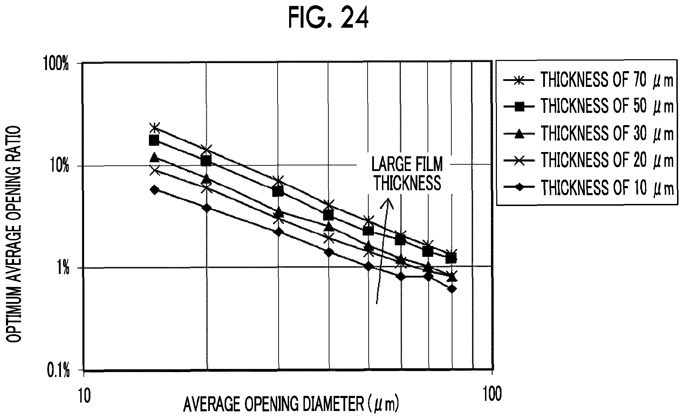

Calculation in a case where the average opening diameter is 100 $\mu$m or less with in the optimal average opening ratio with respect to the average opening diameter of the through-holes was performed in detail. The results showing optimal average opening ratios for the respective average opening diameters of the through-holes regarding respective thicknesses (10 $\mu$m, 20 $\mu$m, 30 $\mu$m, 50 $\mu$m, and 70 $\mu$m) are illustrated in a double logarithmic graph in Fig. 24. From the graph of Fig. 24, it was found that the average opening ratio changes approximately in a power of -1.6 with respect to the average opening diameter of the through-holes.

More specifically, it became clear that, in a case where the optimal average opening ratio is rho_center, the average opening diameter of the through-holes is phi ($\mu$m), and the thickness of the plate-shaped member is t ($\mu$m), the optimal average opening ratio rho_center is determined by rho_center = a x phi$^{-1.6}$ and a = 2 + 0.25 x t by approximating the double logarithmic graph of Fig. 24. It became clear that, particularly in a case where the average opening diameter of the through-holes is small in this way, the absorbance does not become larger as the average opening ratio is smaller, and the optimal average opening ratio is determined depending on the thickness of a plate-shaped member and the average opening diameter of the through-holes. The optimal average opening ratio becomes larger as the thickness of the plate-shaped member is larger, and becomes smaller as the average opening diameter is larger.

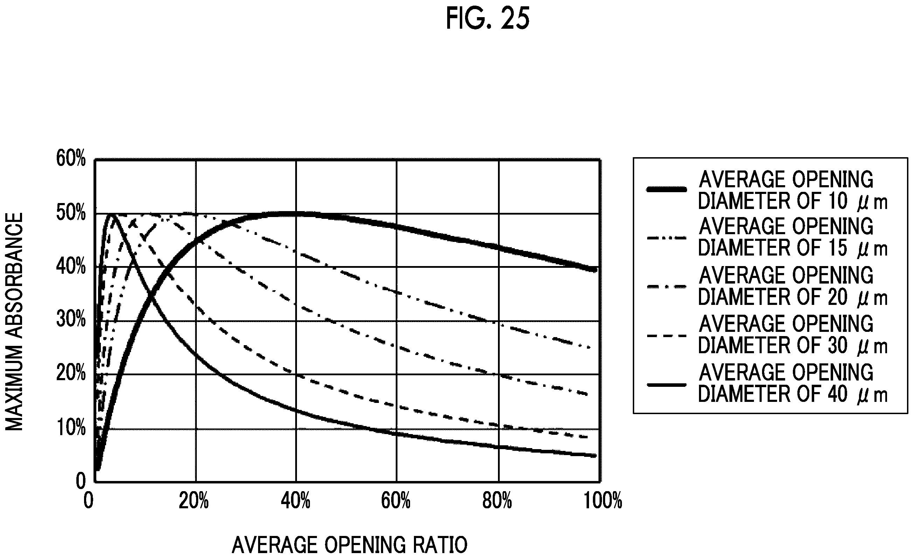

As described above, the range where the absorbance becomes large spreads gently with the optimal average opening ratio as a center. For this detailed analysis, the results obtained by changing the average opening ratio in the simulation of which the thickness of the plate-shaped member is 50 $\mu$m is illustrated in Fig. 25. The average opening diameter of the through-holes was 10 $\mu$m, 15 $\mu$m, 20 $\mu$m, 30 $\mu$m, and 40 $\mu$m, and the average opening ratio was changed from 0.5% to 99%.

In any average opening diameter, the range of the average opening ratio of which the absorbance becomes large spreads to the periphery of the optimal average opening ratio. As a feature, as the average opening diameter of the through-holes is smaller, the range of the average opening ratio where the absorbance becomes large ranges over a wider range. Additionally, as the average opening ratio is higher than the optimal average opening ratio, the range where the absorbance becomes larger.

Since the maximum value of the absorbance is about 50% in any average opening diameter within the range of the average opening diameter of 0.1 $\mu$m or more and less than 100 $\mu$m, the opening ratio of a lower limit and the opening ratio of an upper ratio at which the absorbance is 30%, 40%, and 45% are shown in Table 1, respectively. Additionally, the ranges of the respective absorbances from the optimal average opening ratio are shown in Table 2.

For example, in a case where the average opening diameter of the through-holes is 20 $\mu$m, the optimal average opening ratio is 11%, and the average opening ratio at which the absorbance is 40% or more has a lower limit of 4.5% and an upper limit of 28%. In this case, since the range of the average opening ratio that is 40% of absorbance the optimal average opening ratio as a reference is = (4.5% - 11.0%) - 6.5% - (28.0% - 11.0%) = 17.0%, -6.5% and -17.0% are shown in Table 2.

[Table 1]

| Average Opening Diameter | Optimal Average Opening Ratio | 30% Range | | 40% Range | | 45% Range | |
|---|---|---|---|---|---|---|---|
| | | Lower Limit | Upper Limit | Lower Limit | Upper Limit | Lower Limit | Upper Limit |
| 10$\mu$m | 39.0% | 9.0% | exceeding 90% | 15.0% | 96.0% | 20.5% | 73.0% |
| 15$\mu$m | 17.5% | 4.5% | 77.0% | 7.0% | 47.0% | 9.5% | 34.0% |
| 20$\mu$m | 11.0% | 2.5% | 46.0% | 4.5% | 28.0% | 6.0% | 20.5% |
| 30$\mu$m | 5.5% | 1.5% | 23.0% | 2.5% | 13.5% | 3.0% | 10.0% |
| 40$\mu$m | 3.0% | 1.0% | 14.0% | 1.5% | 8.0% | 2.0% | 6.0% |

[Table 2]

| Average Opening Diameter | Rang from Optimal Average Opening Ratio | | |
|---|---|---|---|
| | 45% Range | 40% Range | 30% Range |
| 10$\mu$m | -18.5%~34% | -24.0%~57.0% | -30.0%~ |
| 15$\mu$m | -8.0%~6.5% | -10.5%~29.5% | -13.0%~59.5% |
| 20$\mu$m | -5.0~9.5% | -6.5%~17.0% | -8.5%~35.0% |
| 30$\mu$m | -2.5%~4.5% | -3.0%~8.0% | -4.0%~17.5% |
| 40$\mu$m | -1.0%~3.0% | -1.5%~5.0% | -2.0%~11.0% |

From Table 2, in a case where the widths of the absorbances for the respective average opening diameters of the through-holes are compared with each other, the widths of the absorbances vary substantially in a ratio of 100 x phi$^{-2}$ where the average opening diameters of the through-holes are phi ($\mu$m). Therefore, regarding the absorbances of 30%, 40%, and 45%, suitable ranges for the respective average opening diameters can be determined.

That is, the range of the absorbance of 30% needs to fall within a range where rho_center - 0.085 x (phi/20)$^{-2}$ is an average opening ratio of a lower limit and rho_center + 0.35 x (phi/20)$^{-2}$ is an average opening ratio of an upper limit, using a range where the average opening diameter of the through-holes is 20 $\mu$m with the above-described optimal average opening ratio rho_center as a reference. However, the average opening ratio is limited to a range that is larger 0 and smaller than 1 (100%).

It is desirable that the absorbance is within an absorbance of 40%, and a range where rho_center - 0.24 x (phi/10)$^{-2}$ is an average opening ratio of a lower limit and rho_center + 0.57 x (phi/10)$^{-2}$ is an average opening ratio of an upper limit. Here, in order to make an error as small as possible, the reference of the average opening diameter was 10 $\mu$m.

It is more desirable that the absorbance is within a range of 45% and a range where rho_center - 0.185 x (phi/10)$^{-2}$ is an average opening ratio of a lower limit and rho_center + 0.34 x (phi/10)$^{-2}$ is an average opening ratio of an upper limit.

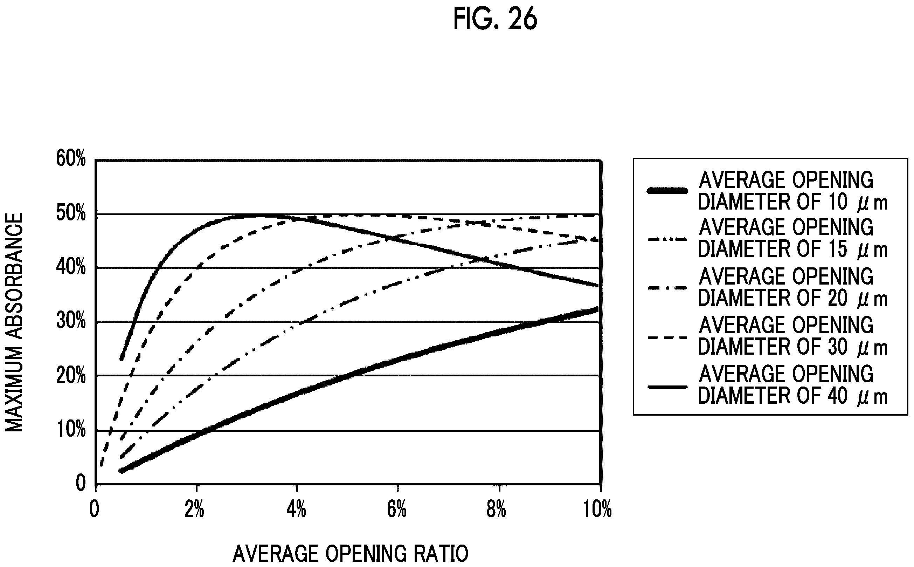

Moreover, in order to determine the range of the optimal average opening ratio in the case of a smaller absorbance, calculation was finely made in a range where the average opening ratio is small. As a representative example, the results in a case where the thickness of the plate-shaped member is 50 μm and the average opening diameter of the through-holes is 30 μm are illustrated in Fig. 26.

Regarding the absorbances of 10%, 15%, and 20%, respectively, the ranges of the average opening ratios having the absorbances, and approximate equations are illustrated in Table 3 and 4, respectively. In addition, "rho_center" is written as "rc" in Table 4.

[Table 3]

| Average Opening Diameter | Optimal Average Opening Ratio | 10% Range | | 15% Range | | 20% Range | |
|---|---|---|---|---|---|---|---|
| | | Lower Limit | Upper Limit | Lower Limit | Upper Limit | Lower Limit | Upper Limit |
| 30μm | 5.5% | 0.3% | 85.0% | 0.5% | 56.0% | 0.7% | 40.0% |

[Table 4]

| | Lower Limit | Upper Limit |
|---|---|---|
| 10% Range | $rc - 0.052 \times (phi/30)^{-2}$ | $rc + 0.795 \times (phi/30)^{-2}$ |
| 15% Range | $rc - 0.050 \times (phi/30)^{-2}$ | $rc + 0.505 \times (phi/30)^{-2}$ |
| 20% Range | $rc - 0.048 \times (phi/30)^{-2}$ | $rc + 0.345 \times (phi/30)^{-2}$ |

From Table 3 and 4, the range of the absorbance of 10% needs to fall within a range where rho_center - 0.052 x (phi/30)$^{-2}$ is an average opening ratio of a lower limit and rho_center + 0.795 x (phi/30)$^{-2}$ is an average opening ratio of an upper limit, using a range where the average opening diameter of the through-holes is 30 μm with the above-described optimal average opening ratio rho_center as a reference. However, the average opening ratio is limited to a range that is larger 0 and smaller than 1 (100%).

Desirably, the absorbance is 15% or more and a range where rho_center - 0.050 x (phi/30)$^{-2}$ is an average opening ratio of a lower limit and rho_center + 0.505 x (phi/30)$^{-2}$ is an average opening ratio of an upper limit.

More desirably, the absorbance is 20% or more and a range where rho_center - 0.048 x (phi/30)$^{-2}$ is an average opening ratio of a lower limit and rho_center + 0.345 x (phi/30)$^{-2}$ is an average opening ratio of an upper limit.

Still more desirably, the average opening ratio falls within a range where the above-described absorbance is 30% or more, 40% or more, or 45% or more, and the absorbance can be made larger.

As described above, the feature of the sound absorption phenomenon resulting from the friction within the through-holes was clarified using the simulation. Additionally, the size of the absorbance was determined depending on the thickness of the plate-shaped member and the average opening diameter and the average opening ratio of the through-holes, and the optimal value range thereof were determined.

Moreover, a still further example of the sound insulation structure will be described.

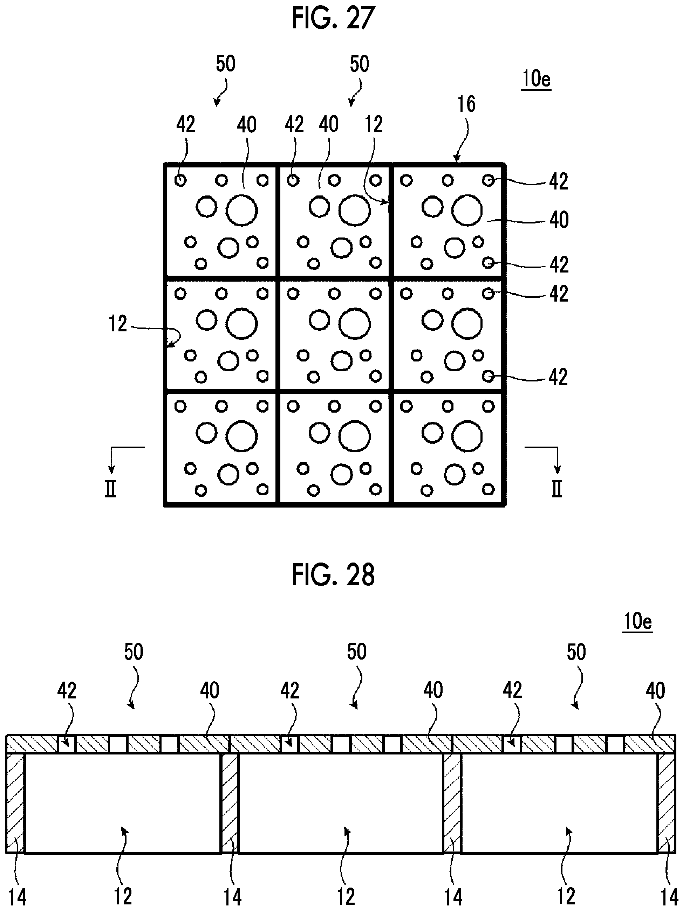

Fig. 27 is a front view schematically illustrating a still further example of the sound insulation structure, and Fig. 28 is a cross-sectional view taken along line II-II of Fig. 27.

In addition, a sound insulation structure 10e illustrated in Figs. 27 and 28 includes a plate-shaped member having a plurality of through-holes penetrating in a thickness direction, and a frame having a frame hole part, the plate-shaped member performs film vibration by fixing the plate-shaped member to a circumferential edge of the frame hole part of the frame, the average opening diameter of the through-holes is 0.1 μm or more and 250 μm or less, and the first natural vibration frequency of the film vibration of the plate-shaped member is present between 10 Hz to 100000 Hz.

In addition, since the sound insulation structure 10e illustrated in Fig. 27 has the same configuration as the sound insulation structure 10a illustrated in Figs. 6 and 7 except that the average opening diameters of the through-holes 42 are different from each other and the same plate-shaped member 40 as the plate-shaped member 40 in illustrated in Fig. 13 is provided instead of the film 18a, the same parts will be designated by the same reference signs, and the following description will mainly be performed regarding different parts.

The sound insulation structure 10e illustrated in Figs. 27 and 28 is a sound insulation structure which includes a plate-shaped member 40 having a plurality of through-holes 42 penetrating in a thickness direction, and a frame 14 that has a penetrating frame hole part 12 and fixes and supports a circumferential edge part of the plate-shaped member 40, and in which the average opening diameter of the through-holes 42 is 0.1 μm or more and 250 μm or less and the first natural vibration frequency of the film vibration of the plate-shaped member is present between 10 Hz to 100000 Hz.

Here, the plate-shaped member 40 is the same as that of the plate-shaped member 40 having the through-holes 42 illustrated in Figs. 13 and 14 except that the ranges of the average opening diameters are different from each other.

Here, the example illustrated in Figs. 27 and 28 has a frame body 16 that forms a plurality of (nine in the illustrated example) frames 14 which have frame hole parts 12, respectively, and are two-dimensionally disposed; a plurality of (nine in the illustrated example) plate-shaped members 40 that are fixed to the respective frames 14 so as to cover the frame hole parts 12 of the respective frames 14; and a plurality of through-holes 42 pierced so as to penetrate the plate-shaped members 40 within the respective frames 14.

That is, the sound insulation structure 10e is configured to have a plurality of (nine in the illustrated example) sound insulation cells 50 each having one frame 14, a plate-shaped member 40 fixed to the frame 14, and a plurality of through-holes 42 provided in the plate-shaped member 40.

The plate-shaped member 40 has the plurality of through-holes 42, is fixed so as to cover an opening part inside the frame 14 and be suppressed by the frame 14, and absorbs or reflects and insulates the energy of sound waves by sound passing through the through-holes in correspondence with the sound waves from the outside and by performing the film vibration.

Additionally, as mentioned above, the average opening diameter of the plurality of through-holes 42 formed in the plate-shaped member 40 is 0.1 $\mu$m or more and 250 $\mu$m or less.

Additionally, the average opening ratio of the plurality of through-holes 42 is preferably 2% or more.

By providing the sound insulation structure which includes the plate-shaped member 40 having the plurality of through-holes 42 of which the average opening diameter is 0.1 $\mu$m or more and 250 $\mu$m or less and the frame 14 that have the opening part and fixes and supports the circumferential edge part of the plate-shaped member 40, and of which the first natural vibration frequency of the film vibration of the plate-shaped member is present between 10 Hz to 100000 Hz, the present inventors have found that the average absorbance on a lower frequency side equal to or lower than the first natural vibration frequency of the film vibration of the plate-shaped member 40 is larger than the absorbance in the first natural vibration frequency and the sound absorption effect is obtained on the lower frequency side equal to or lower than the first natural vibration frequency of the film vibration.

According to the present inventor's study, it is believed that, since the plate-shaped member 40 and the through-holes 42 are present in the configuration of the sound insulation structure 10d, sound passes through and is transmitted through any one of these two types. A path along which sound is transmitted through the plate-shaped member 40 is a path along which solid vibration converted once into the film vibration of the plate-shaped member 40 is reradiated to as sound waves, and a path along which sound is transmitted the through-holes 42 is a path along which sound directly passes through the through-holes 42 as a gas propagation sound. It is considered that a path along which sound passes through the through-holes 42 is dominant as a current absorption mechanism.

Here, it was estimated that a mechanism of sound absorption in the path along which sound is transmitted through the through-holes 42 is a change of the energy of sound to thermal energy by the friction with inner wall surfaces of the through-hole 42 and the air in a case where the sound passes through the fine through-holes 42. Since this mechanism is caused by the size of the through-holes 42 being fine, this mechanism is different from a mechanism resulting from resonance. Paths along which sound directly passes as sound in the air by the through-holes 42 have far smaller impedance compared to a path along which sound is first converted into the film vibration and is then radiated as sound again. Hence, sound is likely to pass along the paths of the through-holes 42 finer than the film vibration. In a case where sound passes through the portions of the through-holes 42, the sound collectively passes from a wide area of the entire plate-shaped member 40 to a narrow area of the through-holes 42. As the sound is collected in the through-holes 42, local speed is extremely large. Since the friction is correlated with speed, the friction becomes large within the fine through-holes 42, and the friction is converted into heat.

Since the ratio of circumferential length to opening area becomes large in a case where the average opening diameter of the through-holes 42 is small, it is believed that the friction caused at edge parts or inner wall surfaces of the through-holes 42 can be increased. By increasing the friction in a case where the sound passes through the through-holes 42, the energy of the sound can be converted into thermal energy and the sound can be absorbed.

Additionally, sound can be absorbed with the friction in a case where the sound passes through the through-holes 42. Thus, the sound can be absorbed irrespective of the frequency range of the sound, and the sound can be absorbed in a broadband.

Here, a region where the amount of sound shielding is determined depending on the stiffness of the plate is present on the lower frequency side than the first natural vibration frequency of the film vibration, and this is referred to a rigid rule.

The present inventors have found this time that, in this rigid rule, a significant absorption effect is obtained depending on the effect of the through-holes irrespective of being on the lower frequency side than the first natural vibration frequency.

In the rigid rule, a motion dominated by a spring equation in which that a film (plate-shaped member) moved by attaching the film to a frame member is pulled from an end part is larger than a motion dominated by a motion equation in which sound waves push the film. In this rigid rule, the effect that a tension is increased by pulling the film from the frame member is shown, and there is the effect that the apparent firmness of the film becomes extremely large compared to the Young's modulus of an actual film.

Generally, in a low frequency region, a force that vibrates the film is large and the film vibration increased. However, in the configuration of the sound insulation structure 10e, the first natural vibration frequency of the film vibration of the plate-shaped member is set between 10 Hz to 100000 Hz and the apparent firmness of the film is increased by making a rigid rule region on the lower frequency side than the first natural vibration frequency so as not to excessively increase the vibration of the film even in the low frequency region. In this case, since the film seldom vibrates even in the low frequency region, the sound waves often pass through the fine through-holes 42. Frictional heat is generated due to the effect of the fine through-holes 42, and sound can be widely absorbed on the lower frequency side.

On the other hand, in a high frequency region, the film vibration is not so large from the beginning, and the sound waves often pass through through-holes 42. Therefore, even in the high frequency region, the sound absorption resulting from the friction with the fine through-holes 42 becomes dominant.

In this way, the sound insulation structure 10e is a structure that shows the sound absorption effect resulting from the friction with the fine through-holes 42 even in the low frequency region while the sound absorption effect resulting from the friction within the fine through-holes in the high frequency region is left by attaching the frame to make the rigid rule region, in addition to the absorption feature of the high frequency region that is an original function of the fine through-holes 42.

In addition, the first natural vibration frequency in the structure including the frame 14 and the plate-shaped member 40, that is, the first natural vibration frequency of the plate-shaped member 40 fixed so as to be suppressed by the frame 14 is the frequency of a natural frequency mode in which sound waves are greatly transmitted at the frequency thereof in a location where the sound waves vibrate with the film vibration most due to a resonance phenomenon. In the sound insulation structure 10e, since the first natural vibration frequency is determined depending on a structure including the frame 14 and the plate-shaped member 40, the present inventors have found out that the first natural vibration frequency has substantially the same value irrespective of the presence or absence of the through-hole 42 pierced in the plate-shaped member 40.

Additionally, since the film vibration becomes large at a frequency near the first natural vibration frequency, the sound absorption effect resulting from the friction with the fine through-holes 42 becomes small. Hence, in the sound insulation structure 10e, the absorbance becomes minimum at a first natural vibration frequency of $\pm 100$ Hz.

Additionally, from viewpoints of the sound absorbing performance in the low frequency region, the sensitivity of human ears, and the like, the first natural vibration frequency of the film vibration of the plate-shaped member is preferably 20 Hz to 20000 Hz and more preferably 50 Hz to 15000 Hz.

In addition, the first natural vibration frequency of the film vibration of the sound insulation structure 10e can be appropriately set by adjusting the material and the size of the frame, and the material, thickness and the like of a plate-shaped member.

Here, the size of the sound insulation structure 10e can be made small.

Additionally, since a closed space is not provided on a back surface, air permeability can be secured.

In addition, the suitable ranges of the average opening ratio and the average opening diameter of the through-holes 42, the suitable range of the thickness of the plate-shaped member 40, the material and the manufacturing method of the plate-shaped member, and the roughening of the inner wall surfaces of the through-holes 42 are the same as those of the sound insulation structure 10d illustrated in Fig. 13.

Here, from the results of the previous simulation, it is preferable that the relationship between the average opening diameter and the average opening ratio of the through-holes in the sound insulation structure 10e also satisfies the following ranges.

That is, in a case where the average opening diameter of the through-hole is 0.1 $\mu$m or more and less than 100 $\mu$m and in a case where the average opening diameter is phi ($\mu$m) and the thickness of the plate-shaped member is t ($\mu$m), the average opening ratio rho of the through-holes preferably falls within a range having rho_center - (0.085 x (phi/20)$^{-2}$) as a lower limit and having rho_center+ (0.35 x (phi/20)$^{-2}$) as an upper limit, with rho_center = (2 + 0.25 x t) x phi$^{-1.6}$ as a center, more preferably a range of (rho_center - 0.24 x (phi/10)$^{-2}$) or more and (rho_center + 0.57 x (phi/10)$^{-2}$) or less, and still more preferably a range of (rho_center + (rho_center - 0.185 x (phi/10)$^{-2}$) and 0.34 x (phi/10)$^{-2}$) or less.

Additionally, in a case where the average opening diameter of the through-holes is 100 $\mu$m or more and 250 $\mu$m or less, it is preferable that the average opening ratio rho of the through-holes is 0.5 to 1.0%.

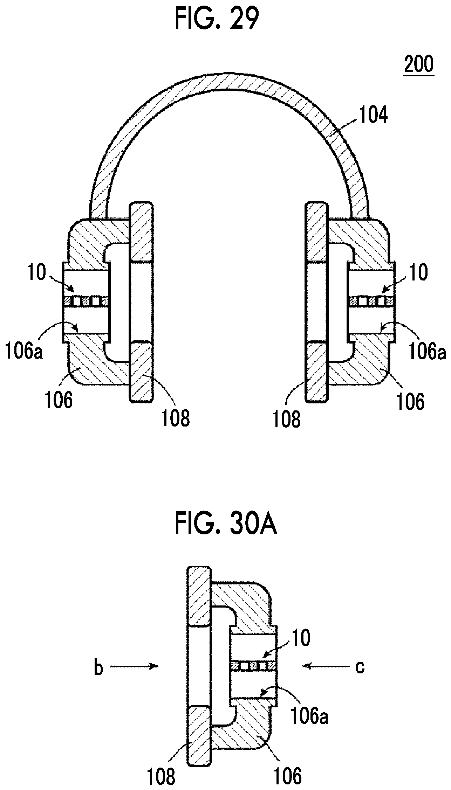

Next, the configuration of another embodiment of the earmuff of the invention will be described with reference to Figs. 29 and 30A to 30C.

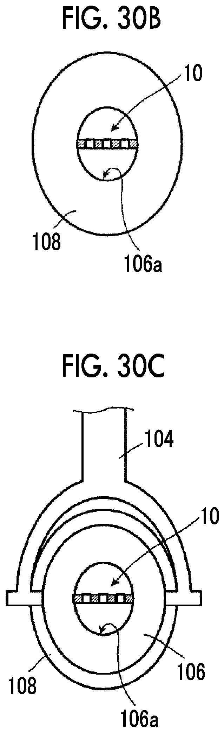

Fig. 29 is a cross-sectional view schematically illustrating another example of the earmuff of the invention, Fig. 30A is a cross-sectional view of an ear cup of the earmuff illustrated in Fig. 29, Fig. 30B is a side view of Fig. 30A as seen from direction b, and Fig. 30C is a side view of Fig. 30A as seen from direction c. An earmuff 200 of the invention of Figs. 29 and 30A to 30C includes a supporting member, and two ear cups each having a housing attached to the supporting member and an ear pad locked to the housing, the housing has a housing opening part, the ear cups each have a sound insulation structure that is disposed in the housing opening part to insulate sound of a specific frequency range, and the housing opening part having the sound insulation structure disposed therein has a ventilation hole, the sound insulation structure has one or more sound insulation cells, the one or more sound insulation cells each include a frame having a penetrating frame hole part, and a film that covers the frame hole part and is fixed to the frame, and the sound insulation structure is disposed in the housing opening part in a state where a film surface of the frame is inclined with respect of an opening cross-section of the housing opening part and the housing opening part is provided with a region serving as the ventilation hole through which gas passes.

In addition, since the earmuff 200 illustrated in Fig. 29 has the same configuration as the sound insulation structure 100 as illustrated in Fig. 1 except that the arrangement within the housing opening part 106a of the sound insulation structure 10 is different, the same parts will be denoted by the same reference signs, and the following description will mainly be performed regarding different parts.

As illustrated in Figs. 29 and 30A to 30C, in the earmuff 200, the sound insulation structure 10 is disposed in the housing opening part 106a in a state where the film surface of the sound insulation structure 10 is inclined with respect to the opening cross-section of the housing opening part 106a and the housing opening part 106a is provided with the region serving as the ventilation hole through which gas passes. That is, the sound insulation structure 10 is disposed such that a direction perpendicular to the film surface intersects to a direction perpendicular to the opening cross-section of the housing 106.

Since sound travels through the housing opening part 106a of the housing 106 in the direction substantially perpendicular to the opening cross-section, it can be said that the sound insulation structure 10 is disposed in a state where the film surface of the film is inclined with respect to the traveling direction of the sound. That is, in the earmuff 200, the sound insulation structure 10 absorbs sound that hits the film surface in an oblique direction or parallel thereto, without sound hitting the film surface perpendicularly thereto.

In addition, in the example illustrated in Fig. 18, the sound insulation structure 10 is disposed such that the direction perpendicular to the film surface of the film 12 is orthogonal to the direction perpendicular to the opening cross-section of the housing opening part 106a. However, the invention is not limited to this. The sound insulation structure 10 may be disposed such that the direction perpendicular to the film surface intersects the direction perpendicular to the opening cross-section of the housing opening part 106a.

From viewpoints of the sound absorbing performance, the air permeability, or the like, the angle of the direction perpendicular to the film surface of the sound insulation structure 10 with respect to the direction perpendicular to the opening cross-section of the housing opening part 106a is preferably 20° or more, more preferably 45° or more, and still more preferably 80° or more.

As illustrated in Fig. 18, the sound insulation structure 10 is disposed parallel to a plane perpendicular to an upward-downward direction of a paper plane in the drawing. However, the invention is not limited this. The sound insulation structure 10 may be disposed such that the direction perpendicular to the film surface of the film 12 is orthogonal to the direction perpendicular to the opening cross-section of the housing opening part 106a. For example, the sound insulation structure 10 may be disposed parallel to the paper plane in the drawing.

Additionally, a gap made between the sound insulation structure 10 and the wall of the housing opening part 106a as seen from the direction perpendicular to the opening cross-section of the housing opening part 106a serves as the ventilation hole that allows the gas formed in the housing opening part 106a to pass therethrough. The opening ratio (that is, the area of the ventilation hole to the opening area of the housing opening part 106a) of the ventilation hole is preferably 10% or more, more preferably 25% or more, and still more preferably 50% or more.

The reason why it is preferable that the opening ratio of the ventilation hole is 10% or more is because high sound insulating performance can be exhibited even in an opening ratio of two or more digits, in the case of a configuration in which the sound insulation structure 10 is used.

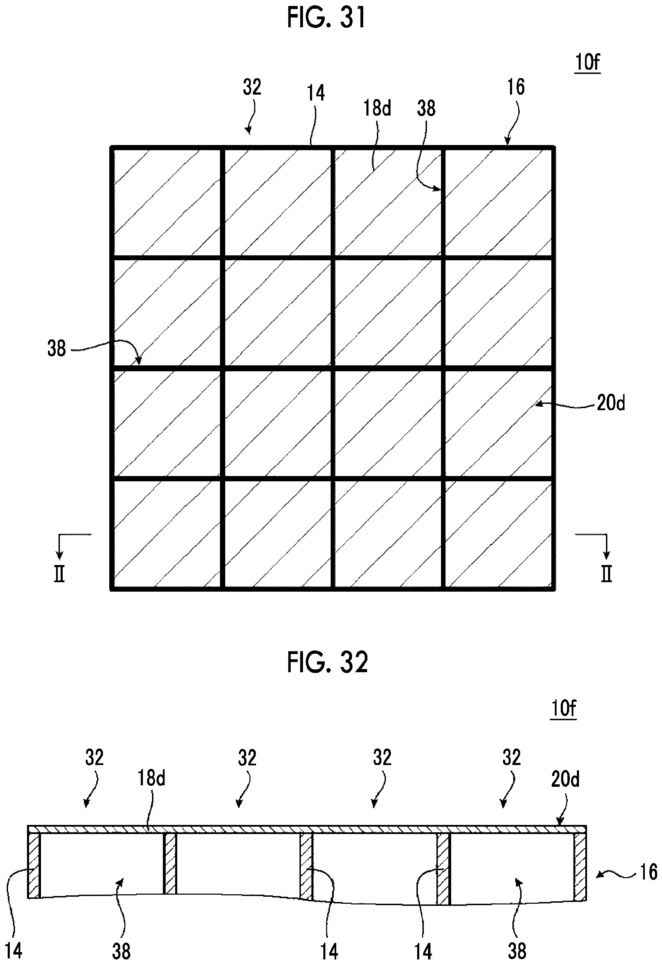

In the earmuff 200, the above-described sound insulation structures 10a to 10e can be used as the sound insulation structure 10 to be disposed in the housing opening part 106a. Additionally, the sound insulation structure 10f illustrated in Figs. 31 and 32 can also be used.

Fig. 31 is a front view schematically illustrating a still further example of the sound insulation structure, and Fig. 32 is a cross-sectional view taken along line II-II of Fig. 31.

A sound insulation structure 10f illustrated in Figs. 31 and 32 has the same configuration as the sound insulation structure 10a illustrated in Figs. 6 and 7 except not having the through-holes 22. Here, the sound insulation structure 10f has a frame body 16 that forms a plurality of (sixteen in the illustrated example) frames 14 which have frame hole parts 12, respectively, and are two-dimensionally disposed; a sheet-like film body 20d having a plurality of (sixteen in the illustrated example) films 18d that are fixed to the respective frames 14 so as to cover the frame hole parts 12 of the respective frames 14.

In the sound insulation structure 10f, one frame 14 and a film 18d fixed to the frame 14 constitute one sound insulation cell 32. That is, it can be said that the sound insulation cell 32 of the sound insulation structure 10f is a sound insulation cell of the same configuration as the first sound insulation cell 32 of the sound insulation structure 10c illustrated in Figs. 11 and 12. The sound insulation structure 10f is constituted of a plurality of (sixteen in the illustrated example) the sound insulation cells 32.

By inclining the sound insulation structure 10f having the sound insulation cell 32 including the frame 14 and the film 18d in the housing opening part 106a in a state where the film surface of the sound insulation structure 10f is inclined with respect to the opening cross-section of the housing opening part 106a and the housing opening part 106a is provided with the region serving as the ventilation hole through which gas passes, the energy of the sound waves of the frequency range near the natural vibration frequency can be absorbed and insulated by the film vibration of the film 18d. Additionally, since ventilation hole is provided, air permeability can be made high.

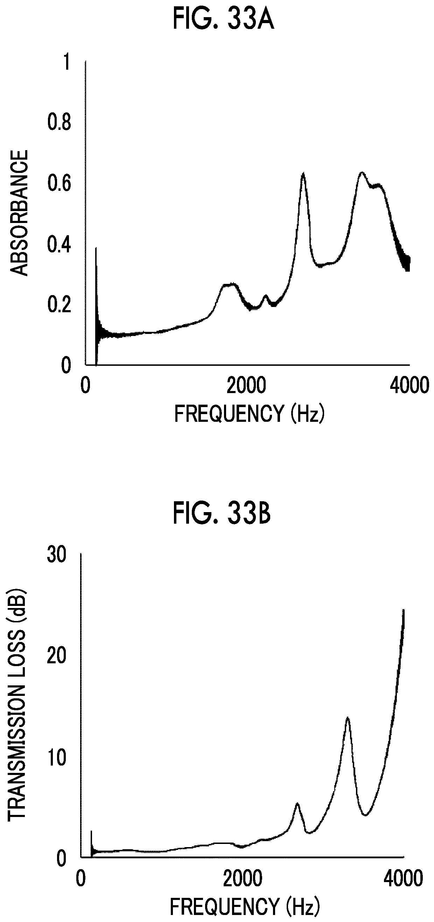

Meanwhile, in a configuration in which the sound insulation structure 10 is disposed so as to be inclined with respect to the opening cross-section of the housing opening part 106a as in the earmuff 200, as illustrated in Fig. 33A, peaks of absorption of the sound waves in which three absorbances have a peak (local maximum) appear from the lower frequency side, and as illustrated in Fig. 33B, shielding peaks of the sound waves in which three transmission losses have a peak (local maximum) from the lower frequency side.

Hence, in the earmuff 200 of the present embodiment, the sound absorption (absorbance) has a peak (local maximum) at three absorption peak frequencies. Therefore, sound of a certain frequency range centered on each absorption peak frequency can be selectively insulated, and the shielding (transmission loss) has a peak (local maximum) in three shielding peak frequencies. Therefore, sound of a certain frequency range centered on each shielding peak frequency can be selectively insulated.

In addition, in the measurement of the acoustic characteristics illustrated in Figs. 33A and 33B, an acoustic tube was simulated in the housing opening part 106a, a sound insulation structure was disposed within the acoustic tube, and the absorbance and the transmission loss (dB) were measured as follows.

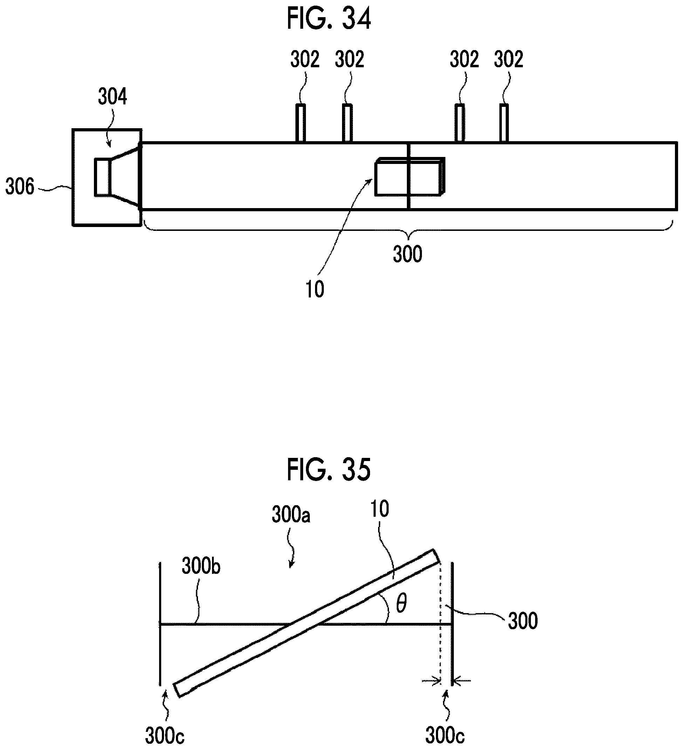

Regarding the acoustic characteristics, as illustrated in Fig. 34, measurement was by a transfer function method using four microphones 302 for an acoustic tube made of aluminum (tube body 300). This technique complies with "ASTM E2611-09: Standard Test Method for Measurement of Normal Incidence Sound Transmission of Acoustical Materials Based on the Transfer Matrix Method". As the acoustic tube, for example, the tube body 300 made of aluminum was used as one based on the same measurement principle as WinZac made by Nittoo Acoustic Engineering Co. Ltd. A cylindrical box body 306 that houses a loudspeaker 304 therein was disposed below the tube body 300, and the tube body 300 was placed on an upper surface of the box body 306. Sound of a predetermined sound pressure was output from the loudspeaker 304 and was measured by the four microphones 302. The sound transmission loss can be measured in a wide spectrum band by this method. Acrostic absorbance and transmission loss measurement was performed within a range of 100 Hz to 4000 Hz by reproducing a configuration in which the sound insulation structure 10 is disposed such that the film surface of the film 18 was inclined in a predetermined measurement region of the tube body 300 serving as the acoustic tube and the sound insulation structure 10 was disposed in the housing opening part 106a so as to be inclined. The results are illustrated in Figs. 33A and 33B.

The sound absorption characteristic expressed by the absorbance to the frequency and the sound shielding characteristic expressed by the transmission loss to the frequency are illustrated in Figs. 33A and 33B.

A tube body made of aluminum having a diameter of 4 cm is used for the tube body 300 used for the acoustic measurement, and the sound insulation structure 10e is disposed such that the film surface of the film 18d is inclined with respect to the opening cross-section of the tube body 300 (refer to Fig. 35). In the sound insulation structure 10, a 250-μm PET film serving as the film 18d is fixed to one surface of the frame hole part 12 of an acrylic frame 14, which has a thickness of 12 mm and is provided with six penetrating frame hole parts 12 of 20 mm square, with a double faced adhesive tape. Additionally, the sound insulation structure 10 has a configuration in which six sound insulation cells are connected in series. The height of the sound insulation structure 10 and the height of the frame 14 are 35 mm.

In the sound insulation structure 10 in which the measurement is performed, as illustrated in Fig. 33A, it can be seen that there are absorption peaks in 1776 Hz, 2688 Hz, and 3524 Hz. Additionally, as illustrated in Fig. 33B, it can be seen that the shielding peaks are present in 2669 Hz, 3298 Hz, and 4000 Hz.

Even in a state having a high opening ratio in this way, the film 18d made of the PET film vibrates with respect to the sound waves, and it is possible to provide high absorptivity and shielding performance with respect to a specific frequency.

In addition, in the sound insulation structure 10, the opening ratio defined by the following Equation (1) is about 67%, and high air permeability or ventilation performance can be obtained.

$$\text{Opening ratio (\%)} = \{1 - (\text{Cross-sectional area of sound insulation cell in opening cross-section/Cross-sectional area})\} \times 100 \qquad (1).$$

In the sound insulation structure 10 of the example illustrated in Fig. 34, as illustrated in Fig. 35, the sound insulation structure 10 is disposed within the tube body 300 simulated in the housing opening part 106a such that the film surface of the film 18 is inclined at a predetermined inclination angle θ with respect to an opening cross-section 300b of the tube body 300. In addition, a gap 300c made between the inclined film surface of the film 18 and the tube wall of the tube body 300 illustrated in Fig. 35 serves as the ventilation hole that allows the gas formed in an opening 300a of the tube body 300.

In the invention, the opening ratio of the ventilation hole is preferably 10% or more, more preferably 25% or more, and still more preferably 50% or more.

Here, the reason why it is preferable that the opening ratio of the ventilation hole is 10% or more is because the sound insulation structure of the invention can exhibit high sound insulating performance even in an opening ratio of two or more digits.

Additionally, in the invention, from a viewpoint of air permeability, the inclination angle θ is preferably 20° or more, more preferably 45° or more, and still more preferably 80° or more.

Here, the reason why it is preferable that the inclination angle θ is 20° or more is that, by including the inclination angle θ at 20° or more in a case where a device cross-section (the film surface of the film 18) of the sound insulation structure 10 is equal to the opening cross-section 300b, a preferable opening ratio of 10% or more can be obtained, and wind speed of 10% or more with respect to the wind speed in a case where the inclination angle ° is inclined at 90° can be obtained.

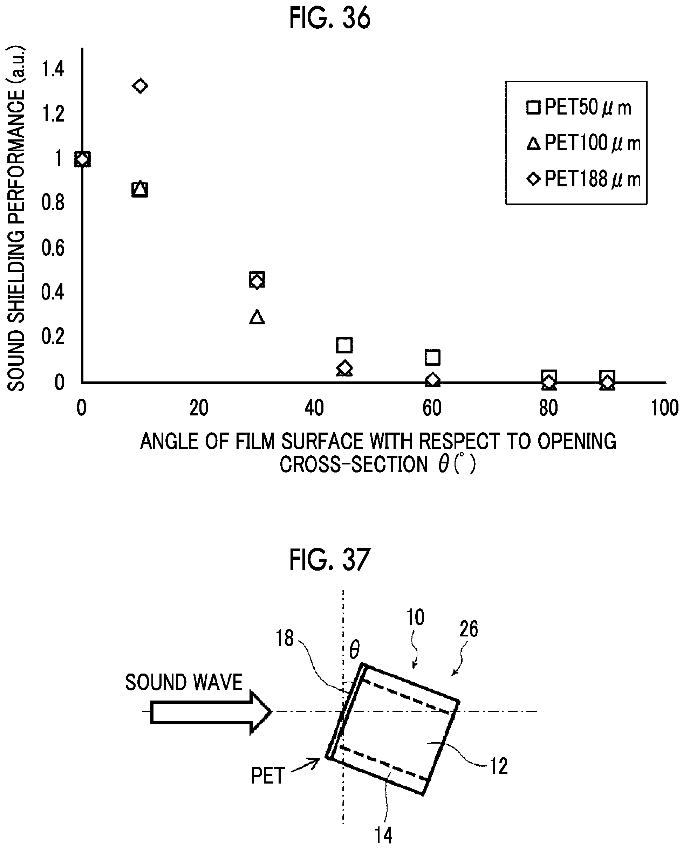

Additionally, this is because the sound shielding peak of a first vibration mode of a low frequency is present at an inclination angle θ of 20° to 45°, and as illustrated in Fig. 36, it is possible to maintain sound shielding performance of 10% or more with respect to the maximum sound shielding (θ = 0°), which is preferable.

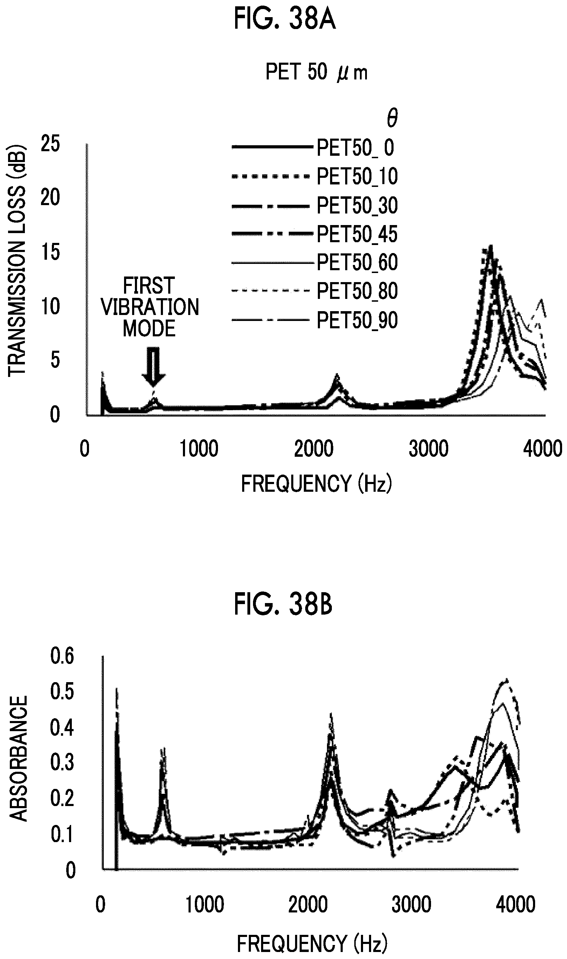

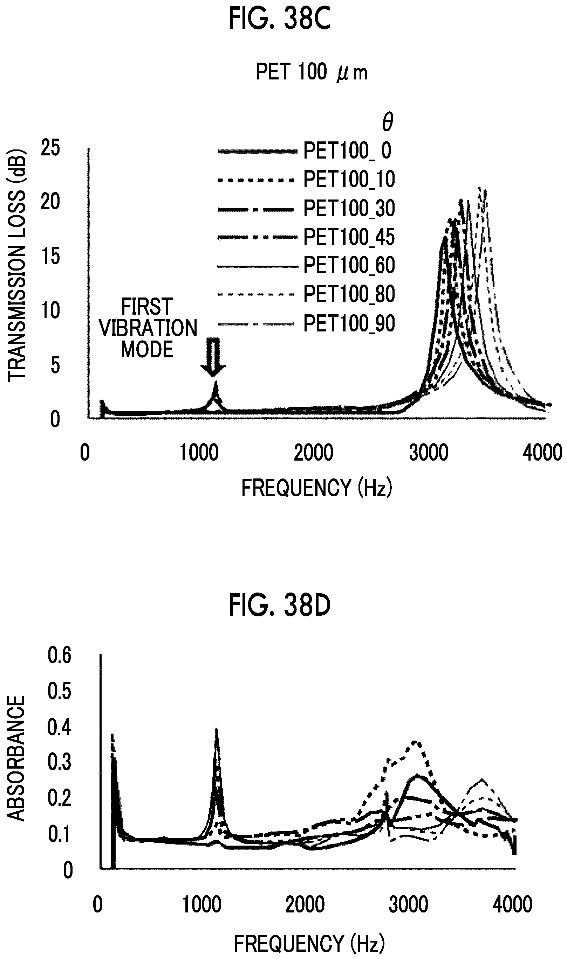

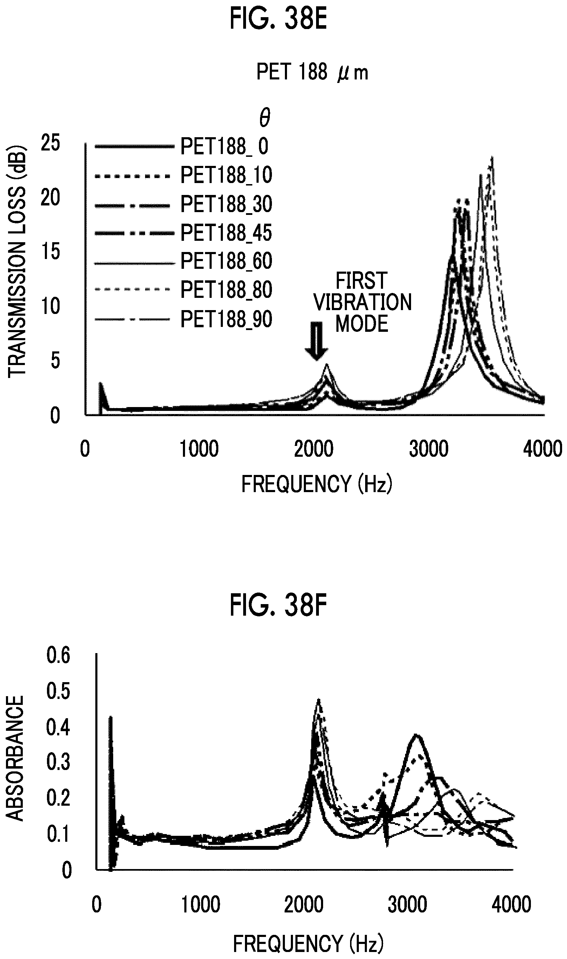

Here, as illustrated in Fig. 37, the inclination angle dependability of the film surface of the sound shielding performance of the sound insulation structure illustrated in Fig. 36 can be obtained by changing the inclination angle θ of the film surface of the film 18, which is fixed to one surface of the frame hole part 12 of the frame 14 of the sound insulation cell 26 of the sound insulation structure 10, with respect to the traveling direction of the sound waves, to measure the transmission loss. In such a method, regarding the sound insulation structure 10 using PET films having three types of different thicknesses of 50 μm, 100 μm, and 188 μm as the film 18, the results obtained by measuring the transmission loss while changing the inclination angle θ within a range of 0° to 90°, respectively are illustrated in Figs. 38A, 38C, and 38E, and the results obtained by measuring the absorbance are illustrated in Figs. 38B, 38D, and 38F.

A graph of the angular dependability of the first vibration mode sound shielding performance illustrated in Fig. 36 can be obtained from the results obtained by measuring the transmission loss illustrated in Figs. 38A, 38C, and 38E. The sound shielding performance of a vertical axis of Fig. 36 is standardized by the transmission loss at 0°.

As illustrated in Fig. 36, it can be seen that, as long as the inclination angle θ is 45° or less, the sound shielding performance of the first vibration mode advantageous to low frequency sound shielding can be maintained at 10% or more with respect to the maximum sound shielding (θ = 0°).

Next, the transmission loss was measured while inclining a film surface of another embodiment of the sound insulation structure at a predetermined inclination angle with respect to the traveling direction of the sound waves, to find the dependability of the sound shielding characteristic (transmission loss) on a sound wave incidence angle.

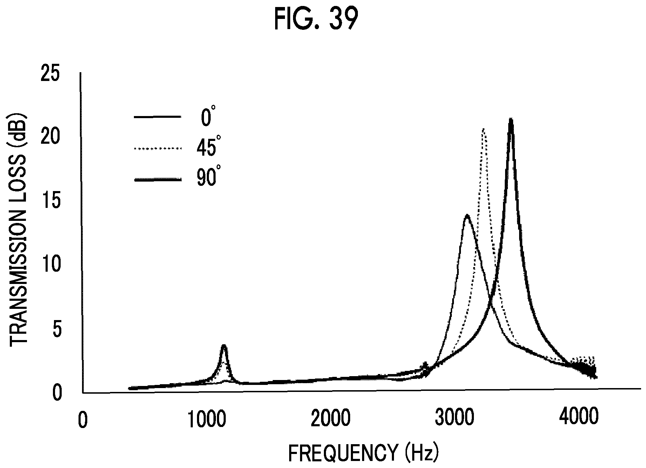

The dependability of the sound shielding characteristic (transmission loss) of a sound insulation structure of another embodiment of the obtained sound insulation structure on sound wave incidence angle is illustrated in Fig. 39.

In the sound insulation structure 10 in which the measurement is performed, a PET film having a thickness of 100 μm as the film 18 is fixed to one surface of the frame 14, in which a 16x16-mm penetrating frame hole part 12 is formed in a 20-mm cube block (rod-shaped sound insulation structure 15) made of vinyl chloride, with a double faced adhesive tape. This sound insulation structure is disposed within the tube body 300 that is an acoustic tube such that the film surface of the film 18 is inclined with respect to the opening cross-section 300b of the tube body 300, and the sound insulating performance (transmission loss) was measured while changing the sound wave incidence angle. It can be seen that, in a case where the incidence angle of the sound waves with respect to the film surface of the film 18 of the sound insulation structure 10 is changed to 90°, 45°, and 0°, the shielding peak frequency on the high frequency side is changed to low frequencies of 3465 Hz, 3243 Hz, and 3100 Hz.

It can be seen that the shielding peak frequency can be adjusted by inclining the film surface of the film 18 with respect to the opening cross-section 300b in this way.

In addition, the suitable range of the thickness, the range of the Young's modulus, the range of the density, the material in the film 18d, the fixing method of the film, and the like are the same as those of the film 18a of the sound insulation structure 10a illustrated in Figs. 6 and 7.

Additionally, in a case where a configuration in which that the sound insulation structure 10 is disposed in an inclined manner within the housing opening part 106a is adopted, a configuration in which the sound insulation structure 10 is disposed at a position protruding from an end surface of the housing opening part 106a may be adopted. Specifically, it is preferable to dispose the sound insulation structure 10 within an opening end correction distance from an opening end of the housing opening part 106a. Since the antinode of the stationary waves of a sound field protrudes to the outside of the housing opening part 106a by the opening end correction distance, the sound insulating performance can be obtained even outside the housing opening part 106a. In addition, in a case where the housing opening part 106a has a cylindrical shape, the opening end correction distance is given by about 0.61 x Tube radius.

Additionally, the sound insulation structure 10f may have a configuration in which a weight is disposed on the film 18d. By changing the weight of the weight, the natural vibration frequency of the film vibration can be adjusted, and the frequency of the sound shielding peak and the sound shielding performance can be controlled.

In addition, the suitable size, weight, and material of the weight are the same as those of the weight 25 of the sound insulation structure 10b illustrated in Figs. 10A and 10B.

As an example using such sound insulation structure, measurement of the absorbance and the transmission loss was performed in the same way as before, regarding the sound insulation structure 10 in which the 100-$\mu$m PET film serving as the film 18 is fixed to a film surface of the frame hole part 12 of the acrylic frame 14, which has a thickness of 12 mm and is provided with the six penetrating frame hole parts 12 of 20 mm square, with a double-stick tape, and a 55-mg weight made of stainless steel is fixed to the film 18 with a double faced adhesive tape.

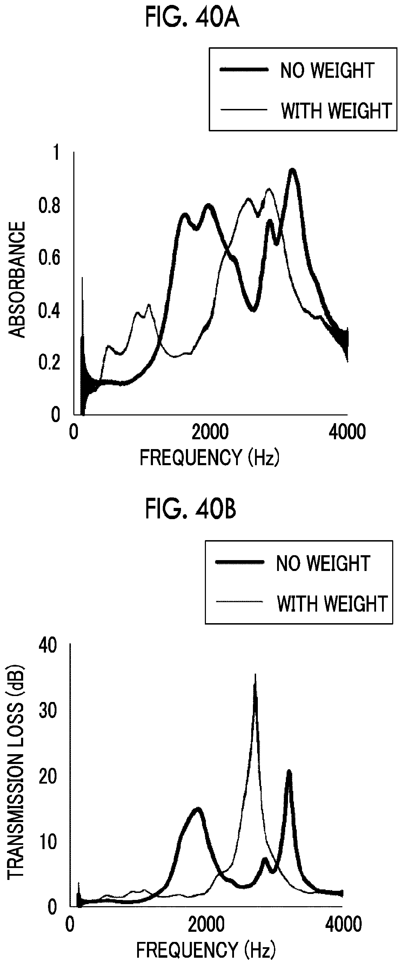

The measurement results are illustrated in Figs. 40A and 40B.

In the absorbance illustrated in Fig. 40A, it can be seen that two absorption peaks 1772 Hz and 3170 Hz in a case where there is no weight shift to the low frequency (993 Hz and 2672 Hz) side by fixedly disposing the weight on the film. Hence, in order to perform low frequency sound absorption, it is preferable to dispose the weight on the film. Additionally, regarding the sound shielding illustrated in Fig. 40B, a sound shielding peak of no less than 35 dB can be obtained by disposing the weight on the film.

Additionally, in the earmuff 200 illustrated in Fig. 29, as the sound insulation structure 10 disposed in the housing opening part 106a, in a case where the sound insulation structure 10a having the through-holes 22 is used for the film 18a as illustrated in Figs. 6 and 7, the frequency of the sound shielding peak and the sound shielding performance can be controlled by changing the diameter of the through-holes 22.

In addition, in the earmuff 200 illustrated in Fig. 29, in a case where the sound insulation structure 10a in which the through-holes 22 are provided in the film 18 as illustrated in Fig. 6 is used as the sound insulation structure 10 to be disposed, the opening ratio of the through-holes 22 (opening part 24) is not particularly limited, and may be set in accordance with the sound shielding frequency range of the sound be selectively insulate. The opening ratio is preferably 0.000001% to 50%, more preferably 0.00001% to 20%, and most preferably 0.0001% to 10%. By setting the opening ratio of the through-holes 42 to the above ranges, the sound shielding peak frequency that is located at the center of the sound shielding frequency range of the noise to be selectively shielded, and the transmission loss of the sound shielding peak can be determined.

Additionally, the size of the through-holes 22 that constitutes the opening part 24 may be any size as long as the through-holes can be appropriately pierced by the above-described processing methods, and is not particularly limited.

However, it is necessary that the size of the through-holes 22 is smaller than the size of the film 18 that is size of the frame hole part 12. However, the size of the through-hole 22 is preferably 100 $\mu$m or more on a lower limit side thereof from a viewpoint of the processing accuracy of laser processing, such as the accuracy of a diaphragm of a laser, the processing accuracy of punching processing, needle processing, or the like, and manufacturability, such as the easiness of processing.

In addition, since the upper limit value of the size of the through-hole 22 needs to be less than the size of the frame 14, normally, the size of the frame 14 is on the order of mm. In a case where the size of the through-hole 22 is set to the order of several hundreds of $\mu$m, the upper limit value of the size of the through-hole 22 does not exceed the size of the frame 14. However, in a case where the upper limit value exceeds the size of the frame 14, the upper limit value of the size of the through-hole 22 may be set to a value equal to or less than the size of the frame 14.

In addition, in a case where different sizes are included in a plurality of the films 18, it is preferable that the size of the through-holes 22 is expressed by the average size.

As an example using such sound insulation structure 10a, measurement of the absorbance and the transmission loss was performed in the same way as before, regarding the sound insulation structure 10a in which 100-$\mu$m PET films serving as films 18a are fixed to both surfaces of the frame hole part 12 of the acrylic frame 14, which has a thickness of 12 mm and is provided with the six penetrating frame hole parts 12 of 20 mm square, with a double faced adhesive tape, and the through-holes 22 having a diameter of 2 mm are formed in the center of one film 18a. This sound insulation structure 10a has a configuration in which six sound insulation cells are connected in series.

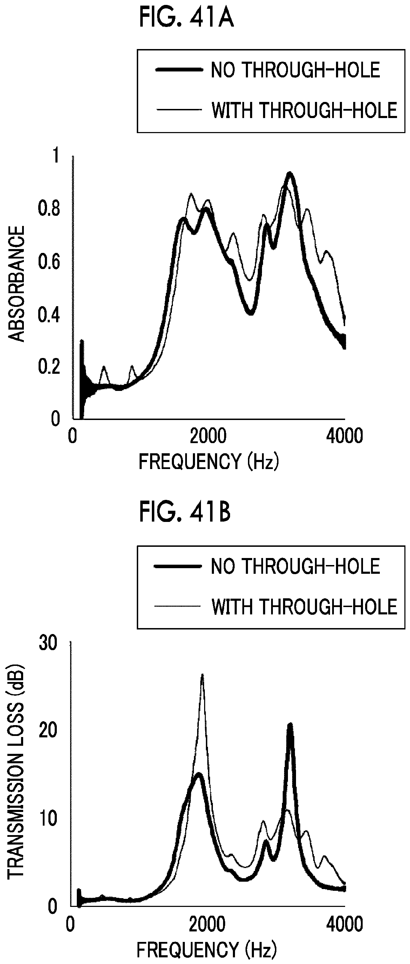

The measurement results are illustrated in Figs. 41A and 41B.

Regarding the absorbance illustrated in Fig. 41A, it can be seen that, as compared to a case where there are no through-holes, the absorption of valleys (2625 Hz) between absorption peaks is larger and the absorption on the high frequency side (3000Hz to 4000Hz) increases. For that reason, in a broadband sound absorption, the sound insulation structure 10a in which the through-holes 22 are formed in the film 18a is preferable.

Additionally, in the transmission loss illustrated in Fig. 41B, the sound shielding peak on the low frequency side of 1915 Hz increases. For that reason, even in a low frequency sound shielding, the sound insulation structure 10a in which the through-holes 22 are formed in the film 18a is preferable.

Similarly, in the earmuff 200 illustrated in Fig. 29, as the sound insulation structure 10 disposed in the housing opening part 106a, in a case where the sound insulation structure 10b in which the through-holes 22 are formed in the film 18b as illustrated in Figs. 10A and 10B and the weight 25 is provided, the natural vibration frequency of the film vibration can be adjusted and the frequency of the sound shielding peak and the sound shielding performance can be controlled, by changing the diameter of the through-holes 22 and the weight of the weight 25.

Additionally, in the earmuff 200 illustrated in Fig. 29, as the sound insulation structure 10 disposed in the housing opening part 106a, the sound insulation structure 10e which has the plate-shaped member 40 having the through-holes 42 of which the average opening diameter as illustrated in Figs. 27 and 28 is 0.1 μm or more and 250 μm or less, and the frame hole part 12 and in which the plate-shaped member 40 is fixed to the periphery the frame hole part 12 and the first natural vibration frequency of the film vibration of the plate-shaped member 40 is present between 10 Hz to 100000 Hz may be used.

In this way, in a case where the sound insulation structure 10e having the frame 14 that fixes the circumferential edge part of the plate-shaped member 42 that has the fine through-holes 42 is used, sound can be absorbed by the friction in a case where the sound passes through the through-holes 42, similar to a case where the sound insulation structure 10e is disposed so as to close the housing opening part 106a.

Although the various embodiments regarding the earmuff of the invention have been described above in detail, it is natural that the invention is not limited to these embodiments, and various improvements and modifications may be made without departing from the scope of the invention.

Explanation of References

10, 10a to 10f: sound insulation structure
12: frame hole part
14: frame
16: frame body
18a to 18d: film
20a to 20d: film body
22: through-hole
24: opening part
25: weight
26: sound insulation cell
30: second sound insulation cell
32: first sound insulation cell
34: second frame
36: second frame hole part
38: first frame hole part
40: plate-shaped member
41: aluminum base material
42: through-hole
43: aluminum hydroxide film
50: sound insulation cell
100, 110, 120: earmuff
102: ear cup
104: headband
106: housing
106a: housing opening part
108: ear pad
122: cassette member
124: case
124a: case opening part
132: sound insulating material

**Claims**

1. An carmuff comprising:

   a supporting member; and
   two ear cups each having a housing attached to the supporting member and an ear pad locked to the housing, wherein the housing has a housing opening part, and

   wherein a sound insulation structure is disposed in the housing opening part and insulates sound of a specific frequency range, and
   wherein the housing opening part having the sound insulation structure disposed therein has a ventilation hole.

2. The earmuff according to claim 1,

   wherein the sound insulation structure has one or more sound insulation cells,
   wherein the one or more sound insulation cells each include a frame having a frame hole part penetrating thereof, a film fixed to the frame, an opening part including one or more through-holes pierced in the film, and
   wherein both end parts of the frame hole parts of the frame are not blocked, and the sound insulation structure is disposed to close the housing opening part.

3. The earmuff according to claim 2, further comprising:

   a weight disposed on the film.

4. The earmuff according to claim 1,

   wherein the sound insulation structure has two or more sound insulation cells that are two-dimensionally arranged,
   wherein at least one of the sound insulation cells is a first sound insulation cell including a first frame having a first frame hole part penetrating thereof, and a film fixed to the first frame,
   wherein at least other one of the sound insulation cells is a second sound insulation cell including a second frame having a second frame hole part penetrating thereof, and
   wherein the sound insulation structure is disposed so as to close the housing opening part.

5. The earmuff according to claim 1,

   wherein the sound insulation structure includes a plate-shaped member having a plurality of through-holes penetrating in a thickness direction thereof,
   wherein an average opening diameter of the through-holes is 0.1 $\mu$m or more and less than 100 $\mu$m,
   wherein an average opening ratio rho of the through-holes is within a range more than 0 and less than 1 and is within a range having rho_center - (0.052 x (phi/30)$^{-2}$) as a lower limit and having rho_center+ (0.795 x (phi/30)$^{-2}$) as an upper limit, with rho_center = (2 + 0.25 x t) x phi$^{-1.6}$ as a center in a case where the average opening diameter of the through-holes is phi ($\mu$m) and a thickness of the plate-shaped member is t ($\mu$m), and
   wherein the sound insulation structure is disposed so as to close the housing opening part.

6. The earmuff according to claim 1,

   wherein the sound insulation structure includes a plate-shaped member having a plurality of through-holes penetrating in a thickness direction, and a frame having a frame hole part, and the plate-shaped member performs film vibration by fixing the plate-shaped member to a circumferential edge of the frame hole part of the frame,
   wherein an average opening diameter of the through-holes is 0.1 $\mu$m or more and 250 $\mu$m or less, and
   wherein a first natural vibration frequency of the film vibration of the plate-shaped member is present between 10 Hz to 100000 Hz.

7. The earmuff according to claim 1,

   wherein the sound insulation structure has one or more sound insulation cells,

wherein the one or more sound insulation cells each include a frame having a frame hole part penetrating thereof, and a film that covers the frame hole part and is fixed to the frame, and

wherein the sound insulation structure is disposed in the housing opening part in a state where a film surface of the film is inclined with respect to an opening cross-section of the housing opening part and the housing opening part is provided with a region serving as a ventilation hole through which gas passes.

8. The earmuff according to claim 7,

   wherein the film has a plurality of through-holes penetrating in a thickness direction thereof, and
   wherein an average opening diameter of the through-holes is 0.1 $\mu$m or more and 250 $\mu$m or less.

9. The earmuff according to any one of claims 1 to 8, further comprising:

   a sound insulating material disposed within the housing.

10. The earmuff according to any one of claims 1 to 9,

    wherein two or more sound insulation structures in which frequency ranges of sound to be insulated are different from each other are disposed in the housing opening part.

11. The earmuff according to any one of claims 1 to 10,

    wherein the sound insulation structure is attachably and detachably disposed in the housing opening part.

12. The earmuff according to any one of claims 1 to 11, further comprising:

    a case having a case opening part penetrating thereof,
    wherein the case opening part includes a cassette member in which the sound insulation structure is disposed, and
    wherein the cassette member is attachably and detachably disposed in the housing.

## FIG. 1

<u>100</u>

102 →                                    ← 102

10                                        10

106a                                      106a

106                                       106

108                  108

## FIG. 2A

102

b →          ← c

10

106a

108          106

## FIG. 2B

## FIG. 2C

## FIG. 3

## FIG. 4

## FIG. 5A

## FIG. 5B

## FIG. 6

## FIG. 7

FIG. 8

FIG. 9

## FIG. 10A

## FIG. 10B

# FIG. 11

# FIG. 12

FIG. 13

40    10d

42

42

42

42

B    B

FIG. 14

42    10d

40

FIG. 15A

41

## FIG. 15B

43

41

## FIG. 15C

42

43

41

## FIG. 15D

10d

42

40

## FIG. 15E

42

10d

40

# FIG. 16

EP 3 437 595 A1

## FIG. 17

EP 3 437 595 A1

## FIG. 18

## FIG. 19

## FIG. 20

## FIG. 21

## FIG. 22

Graph: PERCENTAGE vs AVERAGE OPENING RATIO (%)
Legend: TRANSMITTANCE, ABSORBANCE, REFLECTIVITY

## FIG. 23

Graph: OPTIMAL AVERAGE OPENING RATIO vs AVERAGE OPENING DIAMETER ($\mu$m)
Legend: THICKNESS OF 70 $\mu$m, THICKNESS OF 50 $\mu$m, THICKNESS OF 30 $\mu$m, THICKNESS OF 20 $\mu$m, THICKNESS OF 10 $\mu$m
LARGE FILM THICKNESS

# FIG. 24

FIG. 25

FIG. 26

EP 3 437 595 A1

## FIG. 27

## FIG. 28

## FIG. 29

200

104

10

106a

106

108

10

106a

106

108

## FIG. 30A

10

b →

← c

106a

108

106

# FIG. 30B

10

108

106a

# FIG. 30C

104

10

108

106

106a

## FIG. 31

10f

32    14    18d   38     16

38

20d

II     II

## FIG. 32

10f

32     32     32     32   20d

18d

16

14    38     14   14     38

## FIG. 33A

## FIG. 33B

## FIG. 34

## FIG. 35

## FIG. 36

## FIG. 37

# FIG. 38A

PET 50 $\mu$m

# FIG. 38B

## FIG. 38C

PET 100 $\mu$ m

## FIG. 38D

## FIG. 38E

PET 188 $\mu$m

## FIG. 38F

## FIG. 39

## FIG. 40A

## FIG. 40B

## FIG. 41A

| | |
|---|---|
| ▬▬ | NO THROUGH-HOLE |
| ── | WITH THROUGH-HOLE |

## FIG. 41B

| | |
|---|---|
| ▬▬ | NO THROUGH-HOLE |
| ── | WITH THROUGH-HOLE |

# EP 3 437 595 A1

## INTERNATIONAL SEARCH REPORT

International application No.

PCT/JP2017/004352

### A. CLASSIFICATION OF SUBJECT MATTER
*A61F11/14*(2006.01)i, *G10K11/16*(2006.01)i

According to International Patent Classification (IPC) or to both national classification and IPC

### B. FIELDS SEARCHED

Minimum documentation searched (classification system followed by classification symbols)
A61F11/14, G10K11/16

Documentation searched other than minimum documentation to the extent that such documents are included in the fields searched
Jitsuyo Shinan Koho        1922-1996    Jitsuyo Shinan Toroku Koho    1996-2017
Kokai Jitsuyo Shinan Koho  1971-2017    Toroku Jitsuyo Shinan Koho    1994-2017

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>Y | DE 3623315 A1 (WOLFGANG Brede),<br>21 January 1988 (21.01.1988),<br>column 5, line 43 to column 8, line 3; fig. 1<br>to 9<br>(Family: none) | 1,9,11-12<br>2-3,6,10 |
| X<br>Y | US 4924502 A (ALLEN, Clayton H. and BERGER,<br>Elliott H.),<br>08 May 1990 (08.05.1990),<br>column 3, line 40 to column 5, line 57; fig. 1<br>to 6<br>(Family: none) | 1,11-12<br>2-3,6,10 |
| X<br>Y | JP 349285 Z2 (Emori ADACHI),<br>20 December 1947 (20.12.1947),<br>page 1; fig. 1 to 5<br>(Family: none) | 1,11-12<br>2-3,6,10 |

[×] Further documents are listed in the continuation of Box C.    ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 19 April 2017 (19.04.17) | 09 May 2017 (09.05.17) |

| Name and mailing address of the ISA/<br>    Japan Patent Office<br>    3-4-3,Kasumigaseki,Chiyoda-ku,<br>    Tokyo 100-8915,Japan | Authorized officer<br><br><br>Telephone No. |
|---|---|

Form PCT/ISA/210 (second sheet) (January 2015)

**INTERNATIONAL SEARCH REPORT**

| International application No. |
|---|
| PCT/JP2017/004352 |

C (Continuation).   DOCUMENTS CONSIDERED TO BE RELEVANT

| Category* | Citation of document, with indication, where appropriate, of the relevant passages | Relevant to claim No. |
|---|---|---|
| Y | US 2011/0240402 A1  (CHOU Jung-Tsung et.al.), 06 October 2011 (06.10.2011), paragraphs [0028], [0031]; fig. 4 to 6, 10 to 11 & TW 201133468 A | 2-3,6 |
| Y | GB 2075849 A  (RACAL ACOUSTICS LTD.), 25 November 1981 (25.11.1981), page 3, left column, lines 16 to 36; fig. 6 to 6A & DE 3119260 A        & NO 811555 A & SE 8102931 A        & CA 1168589 A & DK 215581 A | 10 |
| A | FR 497801 A  (EMMANUEL Laime), 18 December 1919 (18.12.1919), entire text; all drawings (Family: none) | 1-12 |
| A | US 2013/0319788 A1  (LARS Peter Franzen), 05 December 2013 (05.12.2013), entire text; all drawings & EP 2671547 A1 | 1-12 |
| A | DE 2910315 A1  (BATTELLE INSTITUT E V), 18 September 1980 (18.09.1980), entire text; all drawings (Family: none) | 1-12 |

Form PCT/ISA/210 (continuation of second sheet) (January 2015)

**EP 3 437 595 A1**

**Patent documents cited in the description**

- JP 2011015338 A
- JP 2011201123 A
- US 4671859 A
- US 4661219 A
- US 4618405 A
- US 4600482 A
- US 4566960 A
- US 4566958 A
- US 4566959 A
- US 4416972 A
- US 4374710 A
- US 4336113 A
- US 4184932 A

**Non-patent literature cited in the description**

- Sanseido Daijirin. Acoustical Materials Association of Japan